(19) Europäisches Patentamt
European Patent Office
Office européen des brevets

(11) Veröffentlichungsnummer: **0 121 082**
**B1**

(12) # EUROPÄISCHE PATENTSCHRIFT

(45) Veröffentlichungstag der Patentschrift: **08.11.89**

(21) Anmeldenummer: **84101910.2**

(22) Anmeldetag: **23.02.84**

(51) Int. Cl.⁴: **C 07 D 239/42,** C 07 D 239/46, C 07 D 213/75, C 07 D 251/18, C 07 D 251/46, C 07 D 409/12, C 07 D 405/12, A 01 N 47/44

(54) **Guanidin-Derivate.**

(30) Priorität: **04.03.83 DE 3307679**
**23.09.83 DE 3334455**

(43) Veröffentlichungstag der Anmeldung:
**10.10.84 Patentblatt 84/41**

(45) Bekanntmachung des Hinweises auf die Patenterteilung:
**08.11.89 Patentblatt 89/45**

(84) Benannte Vertragsstaaten:
**AT BE CH DE FR GB IT LI NL SE**

(56) Entgegenhaltungen:
**EP-A- 0 030 092**
**EP-A- 0 059 597**
**EP-A- 0 061 318**
**EP-A- 0 117 014**
**CH-A- 408 933**
**FR-A- 922 733**
**GB-A- 1 267 433**

**AGRIC. & BIOLOGICAL CHEMISTRY, Band 42, Nr. 4, April 1978, Seiten 803-807; S. TANAKA et al.: "Fungicidal activities of 1,1-diisopropyl-2-(3-pyridyl)-3-p-ethoxyphenylguanidine and its analogs"**

**Die Akte enthält technische Angaben, die nach dem Eingang der Anmeldung eingereicht wurden und die nicht in dieser Patentschrift enthalten sind.**

(73) Patentinhaber: **BAYER AG,**
**D-5090 Leverkusen 1 Bayerwerk (DE)**

(72) Erfinder: **Moriya, Koichi, Namiki-cho 39-15, Hachioji-shi Tokyo (JP)**
Erfinder: **Pfister, Theodor, Dr., Lichtenbergerstrasse 30, D-4019 Monheim (DE)**
Erfinder: **Riebel, Jochem, Dr., In der Beek 92, D-5600 Wuppertal 1 (DE)**
Erfinder: **Eue, Ludwig, Dr., Paul-Klee-Strasse 36, D-5090 Leverkusen 1 (DE)**
Erfinder: **Schmidt, Robert R., Dr., Im Waldwinkel 110, D-5060 Bergisch-Gladbach 2 (DE)**
Erfinder: Lürssen, Klaus, Dr., August-Kierspel-Strasse 151, D-5060 Bergisch-Gladbach 2 (DE)

**EP 0 121 082 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Guanidin-Derivate, mehrere Verfahren zu ihrer Herstellung sowie ihre Verwendung als Herbizide und Pflanzenwuchsregulatoren.

Verschiedene Guanidine sind aus Patentschriften (vgl. z.B. DE-AS 1 089 210, DD-PS 71 016 und 84 530) als potentielle Herbizide bekannt geworden, haben jedoch bisher als Mittel zur Unkrautbekämpfung und/oder Regulierung des Pflanzenwachstums keine größere Bedeutung erlangt.

Weiterhin ist bekannt, daß bestimmte N'-Phenyl-N''-pyridyl-guanidine fungizide Eigenschaften besitzen (vgl. Agric. Biol. Chem. 42(4), S. 803–807 (1978), und daß bestimmte N'-Phenyl-N''-pyrimidinyl-guanidine pharmakologische Wirkungen aufweisen (vgl. CH-A-408 933).

Ferner ist eine Reihe von N'-monosubstituierten und N',N''-disubstituierten Guanidin-Derivaten bekannt, welche als Pharmazeutika verwendet werden können (vgl. EP-A-30 092, EP-A-59 597 und EP-A-61 318).

Es wurden neue Guanidin-Derivate der allgemeinen Formel (I) gefunden,

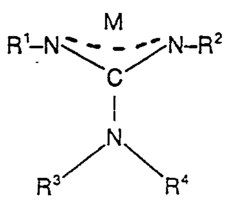 (I)

in welcher

$R^1$ für Wasserstoff oder für den Rest $-S(O)_m-R^5$ steht, worin

m für die Zahlen Null, 1 oder 2 steht und

$R^5$ für den Rest

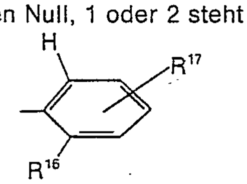

steht, worin

$R^{16}$ und $R^{17}$ gleich oder verschieden sind und für Wasserstoff, Halogen [wie insbesondere Fluor, Chlor und/oder Brom], Cyano, Nitro, $C_1$–$C_6$-Alkyl [welches gegebenenfalls durch Fluor, Chlor, Cyano, $C_1$–$C_4$-Alkoxy-carbonyl, $C_1$–$C_4$-Alkoxy, $C_1$–$C_4$-Alkylthio, $C_1$–$C_4$-Alkylsulfinyl, $C_1$–$C_4$-Alkylsulfonyl oder Phenyl substituiert ist], für $C_2$–$C_6$-Alkenyl [welches gegebenenfalls durch Fluor, Chlor, Cyano oder $C_1$–$C_4$-Alkoxy-carbonyl substituiert ist], für $C_1$–$C_4$-Alkoxy [welches gegebenenfalls durch Fluor, Chlor, Cyano, $C_1$–$C_4$-Alkoxy-carbonyl, $C_1$–$C_4$-Alkylthio, $C_1$–$C_4$-Alkylsulfinyl oder $C_1$–$C_4$-Alkylsulfonyl substituiert ist], für den Rest $-S(O)_p-R^{18}$, wobei p für die Zahlen Null, 1 oder 2 steht und $R^{18}$ für $C_1$–$C_4$-Alkyl [welches gegebenenfalls durch Fluor oder Chlor substituiert ist], für Phenyl oder für den Rest $-CO-R^{19}$ stehen, wobei $R^{19}$ für $C_1$–$C_6$-Alkyl, $C_1$–$C_6$-Alkoxy, $C_3$–$C_6$-Alkenoxy, $C_1$–$C_4$-Alkylthio, $C_1$–$C_4$-Alkylamino oder Di-($C_1$–$C_4$-alkyl)-amino steht [welche gegebenenfalls durch Fluor und/oder Chlor substituiert sind], worin weiter

$R^5$ für den Rest

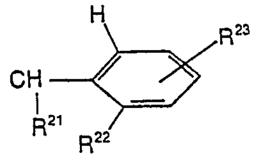

steht, worin

$R^{21}$ für Wasserstoff oder $C_1$–$C_3$-Alkyl steht und

$R^{22}$ und $R^{23}$ gleich oder verschieden sind und für Wasserstoff, Fluor, Chlor, Nitro, Cyano, $C_1$–$C_4$-Alkoxy [welches gegebenenfalls durch Fluor und/oder Chlor substituiert ist], $C_1$–$C_4$-Alkoxy-carbonyl, $C_1$–$C_4$-Alkylsulfonyl oder Di-($C_1$–$C_4$-alkyl)-aminosulfonyl stehen; worin weiter

$R^5$ für den Rest

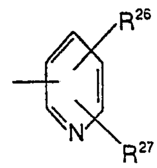

steht, worin

$R^{26}$ und $R^{27}$ gleich oder verschieden sind und für Wasserstoff, Fluor, Chlor, Nitro, Cyano, $C_1$–$C_4$-Alkyl [welches gegebenenfalls durch Fluor und/oder Chlor substituiert ist], $C_1$–$C_4$-Alkoxy [welches gegebenenfalls durch Fluor und/oder Chlor substituiert ist], für $C_1$–$C_4$-Alkylthio, $C_1$–$C_4$-Alkylsulfinyl oder $C_1$–$C_4$-Alkylsulfonyl [welche gegebenenfalls durch Fluor und/oder Chlor substituiert sind], sowie für Di-($C_1$–$C_4$-alkyl)-aminosulfonyl oder $C_1$–$C_4$-Alkoxy-carbonyl stehen, worin weiter

$R^5$ für den Rest

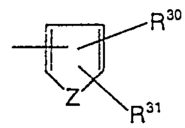

steht, worin

$R^{30}$ und $R^{31}$ gleich oder verschieden sind und für Wasserstoff, Fluor, Chlor, Brom, Cyano, Nitro, $C_1$–$C_4$-Alkyl [welches gegebenenfalls durch Fluor und/oder Chlor substituiert ist], $C_1$–$C_4$-Alkoxy [welches gegebenenfalls durch Fluor und/oder Chlor substituiert ist], $C_1$–$C_4$-Alkylthio, $C_1$–$C_4$-Alkylsulfinyl oder $C_1$–$C_4$-Alkylsulfonyl [welches gegebenenfalls durch Fluor und/oder Chlor substituiert ist], Di-($C_1$–$C_4$-alkyl)-amino-sulfonyl oder $C_1$–$C_4$-Alkoxy-carbonyl stehen, und

Z für Schwefel steht, in welcher weiter

$R^2$ für den Rest

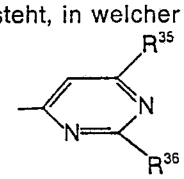

steht, worin

$R^{35}$ und $R^{36}$ gleich oder verschieden sind und für Wasserstoff, Fluor, Chlor, Brom, $C_1$–$C_4$-Alkyl [welches gegebenenfalls durch Fluor und/oder Chlor substituiert ist], $C_1$–$C_4$-Alkoxy [welches gegebenenfalls durch Fluor und/oder Chlor substituiert ist], $C_1$–$C_4$-Alkylamino oder Di-($C_1$–$C_4$-alkyl)-amino stehen mit der Maßgabe, daß wenigstens einer der Reste $R^{35}$ und $R^{36}$ von Wasserstoff verschieden ist; in welcher weiter

$R^2$ für den Rest

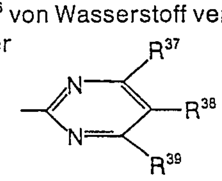

steht, worin

$R^{37}$ für Wasserstoff, Fluor, Chlor, Brom, $C_1$–$C_4$-Alkyl [welches gegebenenfalls durch Fluor und/oder Chlor substituiert ist] oder $C_1$–$C_4$-Alkoxy [welches gegebenenfalls durch Fluor und/oder Chlor substituiert ist], steht,

$R^{38}$ für Wasserstoff, Fluor, Chlor, Brom, $C_1$–$C_4$-Alkyl [welches gegebenenfalls durch Fluor und/oder Chlor substituiert ist], Cyano, Formyl, $C_1$–$C_4$-Alkyl-carbonyl oder $C_1$–$C_4$-Alkoxy-carbonyl steht und

$R^{39}$ für $C_1$–$C_4$-Alkyl [welches gegebenenfalls durch Fluor und/oder Chlor substituiert ist], $C_1$–$C_4$-Alkoxy [welches gegebenenfalls durch Fluor und/oder Chlor substituiert ist], Amino, $C_1$–$C_4$-Alkylamino oder Di-($C_1$–$C_4$-alkyl)-amino steht, oder

$R^{38}$ und $R^{39}$ gemeinsam für $C_3$–$C_4$-Alkandiyl stehen, in welcher weiter

$R^2$ für den Rest

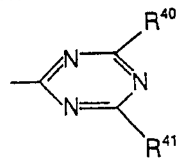

steht, worin

$R^{40}$ und $R^{41}$ gleich oder verschieden sind und für $C_1$–$C_4$-Alkyl [welches gegebenenfalls durch Fluor und/oder Chlor substituiert ist] oder $C_1$–$C_4$-Alkoxy [welches gegebenenfalls durch Fluor und/oder Chlor substituiert ist] stehen; in welcher weiter

$R^3$ für Wasserstoff, $C_1$–$C_4$-Alkyl [welches gegebenenfalls durch Fluor, Chlor, Brom, Cyano, Hydroxy oder $C_1$–$C_2$-Alkoxy substituiert ist], $C_3$–$C_6$-Cycloalkyl, $C_3$–$C_6$-Alkenyl, $C_3$–$C_6$-Alkinyl, Benzyl [welches gegebenenfalls durch Fluor, Chlor oder Methyl substituiert ist] oder für den Rest $-S(O)_n-R^6$ steht, worin

n für die Zahlen Null, 1 oder 2 steht und

$R^6$ die oben für $R^5$ angegebene Bedeutung hat, jedoch nicht in jedem Einzelfall mit $R^5$ identisch ist; in welcher weiter

$R^3$ und $R^4$ gemeinsam für $C_4$–$C_6$-Alkandiyl stehen, welches gegebenenfalls durch eine Sauerstoff-Brücke unterbrochen ist, in welcher weiter

$R^4$ für den Rest $-X-R^8$ steht, worin

X für Sauerstoff steht und

$R^8$ für $C_1$–$C_6$-Alkyl [welches gegebenenfalls durch Fluor, Chlor, $C_1$–$C_4$-Alkoxy, $C_1$–$C_4$-Alkylthio, $C_1$–$C_4$-Alkylsulfinyl oder $C_1$–$C_4$-Alkylsulfonyl substituiert ist], $C_3$–$C_6$-Alkenyl, $C_3$–$C_6$-Alkinyl, $C_3$–$C_6$-Cycloalkyl, Benzyl [welches gegebenenfalls durch Fluor, Chlor oder Methyl substituiert ist] oder Phenyl [welches gegebenenfalls durch Fluor, Chlor, Brom, Nitro, Cyano, $C_1$–$C_4$-Alkyl, Trifluormethyl, $C_1$–$C_4$-Alkoxy, Trifluormethoxy, $C_1$–$C_4$-Alkylthio oder Trifluormethylthio substituiert ist] steht; in welcher weiter

$R^4$ für den Rest 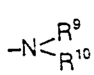

steht, worin

$R^9$ für Wasserstoff oder $C_1$–$C_4$-Alkyl steht und

$R^{10}$ für $C_1$–$C_4$-Alkyl, $C_3$–$C_6$-Alkenyl, $C_3$–$C_6$-Alkinyl, $C_3$–$C_6$-Cycloalkyl [welches gegebenenfalls durch eine $-SO_2$-Brücke unterbrochen ist], Benzyl oder Phenylethyl, Phenyl [welches gegebenenfalls durch Fluor, Chlor, Brom, Nitro, Cyano, $C_1$–$C_4$-Alkyl, Trifluormethyl, $C_1$–$C_4$-Alkoxy, Trifluormethoxy, $C_1$–$C_4$-Alkylthio oder Trifluormethylthio substituiert ist], Pyrimidyl, $C_1$–$C_4$-Alkyl-carbonyl, Benzoyl, $C_1$–$C_4$-Alkoxy-carbonyl, $C_1$–$C_4$-Alkylsulfonyl oder Phenylsulfonyl [welches gegebenenfalls durch Fluor, Chlor, Brom oder Methyl substituiert ist], steht, oder

$R^9$ und $R^{10}$ gemeinsam für $C_4$–$C_6$-Alkandiyl [welches gegebenenfalls durch eine Sauerstoffbrücke unterbrochen ist], stehen; in welcher weiter

$R^4$ für den Rest

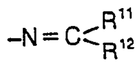

steht, worin

$R^{11}$ für Wasserstoff oder $C_1$–$C_4$-Alkyl steht und

$R^{12}$ für $C_1$–$C_4$-Alkyl, Benzyl oder Phenylethyl oder Phenyl [welches gegebenenfalls durch Fluor, Chlor, Brom, $C_1$–$C_4$-Alkyl, Trifluormethyl, Cyano, Nitro, $C_1$–$C_4$-Alkoxy oder Trifluormethoxy substituiert ist], steht, oder

$R^{11}$ und $R^{12}$ gemeinsam für $C_4$–$C_6$-Alkandiyl stehen; in welcher weiter

$R^4$ – für den Fall, daß $R^3$ von Wasserstoff verschieden ist – für Wasserstoff oder Hydroxy oder für $C_1$–$C_6$-Alkyl [welches gegebenenfalls durch Fluor, Chlor, Cyano, $C_1$–$C_4$-Alkoxy-carbonyl, Hydroxy oder $C_1$–$C_4$-Alkoxy substituiert ist], $C_3$–$C_6$-Cycloalkyl [welches gegebenenfalls durch eine $-SO_2$-Brücke unterbrochen ist], $C_3$–$C_6$-Alkenyl, $C_3$–$C_6$-Alkinyl, Benzyl [welches gegebenenfalls durch Fluor, Chlor und/oder Methyl substituiert ist] oder Phenyl [welches gegebenenfalls durch Fluor, Chlor, Cyano, Nitro, Amino, $C_1$–$C_4$-Alkyl, Trifluormethyl, $C_1$–$C_4$-Alkoxy, Trifluormethoxy, $C_1$–$C_4$-Alkylthio, Trifluormethylthio, Aminosulfonyl oder $C_1$–$C_4$-Alkoxy-carbonyl substituiert ist] steht und in welcher ferner

M für Wasserstoff, ein Natrium-, Kalium-, Magnesium-, Calcium- oder Eisen-äquivalent oder einen gegebenenfalls durch $C_1$–$C_6$-Alkyl, $C_3$–$C_6$-Alkenyl und/oder Benzyl substituierten Ammoniumrest steht.

Gegenstand der Erfindung sind ferner 1 : 1-Addukte von Verbindungen der Formel (I) – wie vorausgehend definiert – mit Halogenwasserstoffsäuren (wie Hydrogen-fluorid, -chlorid, -bromid und -iodid), mit Schwefelsäure, Phosphorsäure, mit gegebenenfalls durch Fluor oder Chlor substituierten Alkansulfonsäuren mit bis zu 4 Kohlenstoffatomen oder auch Benzol- oder Naphthalinsulfonsäuren, welche gegebenenfalls durch Fluor, Chlor oder Methyl substituiert sind.

Die durch die allgemeine Formel (I) skizzierten neuen Guanidin-Derivate liegen für den Fall, daß M für Wasserstoff steht, als Gemische von Tautomeren der Formeln (IA) und (IB) vor:

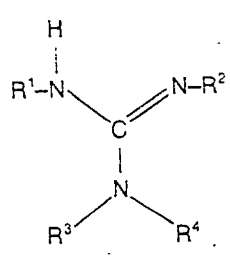

(IA)

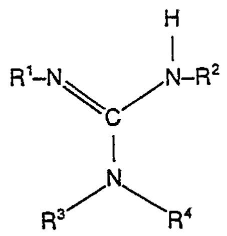

(IB)

Das Mischungsverhältnis hängt von aggregationsbestimmenden Faktoren wie z. B. Temperatur, Lösungsmittel und Konzentration ab.

Für den Fall, daß neben M auch $R^3$ oder $R^4$ für Wasserstoff stehen, steht die allgemeine Formel (I) auch für weitere mögliche Tautomere, wie sie in den Formeln (IC) und (ID) skizziert sind:

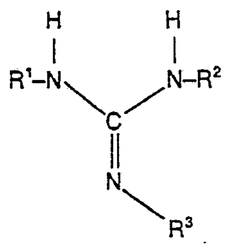

(IC)

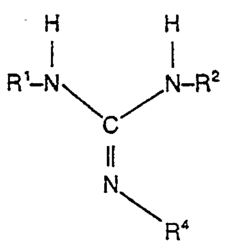

(ID)

Man erhält die neuen Guanidin-Derivate der Formel (I)

(a) für den Fall, daß $R^1$ für Wasserstoff steht, $R^3$ für die oben genannten Reste – jedoch ausgenommen den Rest $-S(O)_n-R^5$ – steht, M für Wasserstoff steht und die Reste $R^2$ und $R^4$ die oben angegebenen Bedeutungen haben, wenn man Cyanoverbindungen der Formel (II)

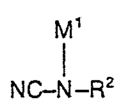

(II)

in welcher

$M^1$ für Wasserstoff steht und

$R^2$ die oben angegebene Bedeutung hat, mit Aminoverbindungen der Formel (III)

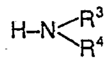

(III)

in welcher

$R^3$ für die oben genannten Reste – jedoch ausgenommen den Rest $-S(O)_n-R^6$ – steht und

$R^4$ die oben angegeben Bedeutung hat, bzw. mit Hydrochloriden von Aminoverbindungen der Formel (III), gegebenenfalls in Gegenwart von Verdünnungsmitteln umsetzt und gegebenenfalls die Umsetzungsprodukte mit Säureakzeptoren behandelt; oder

(b) für den Fall, daß $R^1$ für den Rest $-S(O)_m-R^5$ steht, worin

m und $R^5$ die oben angegebenen Bedeutungen haben, und

M die bei (a) angegebene Bedeutung hat, wobei ferner die Reste $R^2$, $R^3$ und $R^4$ die oben angegebenen Bedeutungen haben, oder für den Fall, daß $R^3$ für den Rest $-S(O)_n-R^6$ steht, worin

n und $R^6$ die oben angegebenen Bedeutungen haben, und

M die bei (a) angegebene Bedeutung hat, wobei ferner die Reste $R^1$, $R^2$ und $R^4$ die oben angegebenen Bedeutungen haben, wenn man die nach dem oben unter (a) beschriebenen Herstellungsverfahren erhältlichen Guanidin-Derivate der Formel (I), in welcher $R^1$ für Wasserstoff steht, $R^3$ für die oben genannten Reste – jedoch ausgenommen den Rest $-S(O)_n-R^6$ – steht, M für Wasserstoff steht und die Reste $R^2$ und $R^4$ die oben angegebenen Bedeutungen haben, mit Halogen-Schwefel-Verbindungen der Formel (IV)

$$R^5-S(O)_m-X^1 \qquad (IV)$$

in welcher

$X^1$ für Fluor, Chlor oder Brom steht und

m und $R^5$ die oben angegebenen Bedeutungen haben, und/oder mit Halogen-Schwefel-Verbindungen der Formel (V)

$$R^6-S(O)_n-X^2 \qquad (V)$$

in welcher

$X^2$ für Fluor, Chlor oder Brom steht und

n und $R^6$ die oben angegebenen Bedeutungen haben, gegebenenfalls in Gegenwart von Säureakzeptoren und gegebenenfalls in Gegenwart von Verdünnungsmitteln umsetzt; oder

(c) für den Fall, daß $R^3$ für die oben genannten Reste – jedoch ausgenommen den Rest $-S(O)_n-R^6$ – steht und M, $R^1$, $R^2$ und $R^4$ die oben angegebenen Bedeutungen haben, wenn man Isothioharnstoffe der Formel (VI)

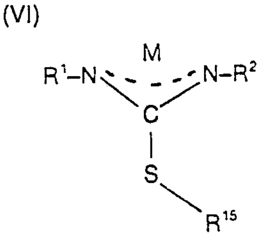

(VI)

in welcher

$R^{15}$ für $C_1$–$C_4$-Alkyl oder Benzyl steht und

M und die Reste $R^1$ und $R^2$ die oben angegebenen Bedeutungen haben, mit Aminoverbindungen der Formel (III)

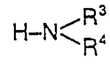

(III)

in welcher

$R^3$ für die oben genannten Reste – jedoch ausgenommen den Rest $-S(O)_n-R^6$ – steht und

$R^4$ die oben angegebene Bedeutung hat, bzw. mit Hydrochloriden von Aminoverbindungen der Formel (III), gegebenenfalls in Gegenwart von Säureakzeptoren und gegebenenfalls in Gegenwart von Verdünnungsmitteln umsetzt und gegebenenfalls die Umsetzungsprodukte mit Säuren behandelt; oder

(d) für den Fall, daß $R^3$ für die oben genannten Reste – jedoch ausgenommen den Rest $-S(O)_n-R^6$

– steht und M, $R^1$, $R^2$ und $R^4$ die oben angegebenen Bedeutungen haben, wenn man Guanidin-Derivate der Formel (I), in welcher

$R^3$ für den Rest $-S(O)_n-R^6$ steht, worin n und $R^6$ die oben angegebenen Bedeutungen haben sowie

M und die Reste $R^1$, $R^2$ und $R^4$ die oben angegebenen Bedeutungen haben, mit Aminoverbindungen der Formel (III)

$$H-N{<}^{R^3}_{R^4} \qquad (III)$$

in welcher

$R^3$ für die oben genannten Reste – jedoch ausgenommen den Rest $-S(O)_n-R^6$ – steht und

$R^4$ die oben angegebene Bedeutung hat, bzw. mit Hydrochloriden von Aminoverbindungen der Formel (III), gegebenenfalls in Gegenwart von Säureakzeptoren und gegebenenfalls in Gegenwart von Verdünnungsmitteln umsetzt; oder

(e) für den Fall, daß M für ein Metalläquivalent oder für einen gegebenenfalls substituierten Ammoniumrest – jeweils wie vorausgehend definiert – steht und die Reste $R^1$, $R^2$, $R^3$ und $R^4$ die oben angegebenen Bedeutungen haben, wenn man Guanidin-Derivate der Formel (I), in welcher M für Wasserstoff steht und die Reste $R^1$, $R^2$, $R^3$ und $R^4$ die oben angegebenen Bedeutungen haben, mit Metall-hydroxiden, -hydriden oder -alkanolaten oder mit metallorganischen Verbindungen bzw. mit Ammoniak oder entsprechenden Aminen gegebenenfalls in Gegenwart von Verdünnungsmitteln umsetzt; oder

(f) für den Fall, daß 1 : 1-Addukte von Guanidin-Derivaten der Formel (I) mit starken Säuren herzustellen sind, wenn man Guanidin-Derivate der Formel (I), in welcher M und die Reste $R^1$, $R^2$, $R^3$ und $R^4$ die oben angegebenen Bedeutungen haben, mit starken Säuren gegebenenfalls unter Verwendung von inerten Verdünnungsmitteln umsetzt.

Die neuen Guanidin-Derivate der Formel (I) und ihre 1 : 1-Addukte mit starken Säuren zeichnen sich durch starke herbizide Wirksamkeit aus und/oder sind zur Regulierung des Wachstums bestimmter Pflanzen geeignet.

Überraschenderweise zeigen die neuen Verbindungen der Formel (I) wesentlich bessere herbizide und pflanzenwuchsregulierende Wirkung als vorbekannte Guanidine gleicher Wirkungsrichtung bei guter Selektivität in Baumwolle und in verschiedenen Getreidearten.

Gegenstand der Erfindung sind vorzugsweise Verbindungen der Formel (I), in welcher

(A) $R^1$ für den Rest $-S(O)_m-R^5$ steht, worin

m für die Zahl 2 steht und

$R^5$ für den Rest

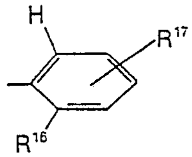

steht, worin

$R^{16}$ für Fluor, Chlor, Brom, Methyl, Trifluormethyl, $C_1$–$C_2$-Alkoxy, Difluormethoxy, Trifluormethoxy, Phenyl oder $C_1$–$C_2$-Alkoxycarbonyl steht und

$R^{17}$ für Wasserstoff steht; in welcher weiter

$R^2$ für den Rest

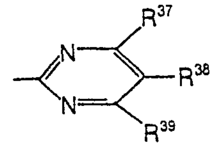

steht, worin

$R^{37}$ für Wasserstoff, Methyl oder Methoxy steht,

$R^{38}$ für Wasserstoff, Chlor, Methyl, Acetyl, oder Methoxycarbonyl steht und

$R^{39}$ für $C_1$–$C_4$-Alkyl oder $C_1$–$C_4$-Alkoxy oder zusammen mit $R^{38}$ für $C_3$–$C_4$-Alkandiyl steht; in welcher weiter

$R^3$ für Wasserstoff, Methyl oder den Rest $-S(O)_n-R^6$ steht, worin n für die Zahl 2 steht und $R^6$ die oben für $R^5$ als bevorzugt angegebene Bedeutung hat; in welcher weiter

$R^4$ für $C_1$–$C_4$-Alkoxy, $C_3$–$C_4$-Alkenoxy, $C_3$–$C_4$-Alkinoxy, Benzyloxy oder für den Rest

$$-N{<}^{R^9}_{R^{10}}$$

steht, worin

$R^9$ für Wasserstoff oder Methyl steht und

$R^{10}$ für $C_1$–$C_3$-Alkyl, Phenyl, Acetyl, Methoxycarbonyl, Phenylsulfonyl oder p-Toluolsulfonyl steht; in welcher weiter

M für Wasserstoff, Natrium, Kalium, ein Magnesium- oder Calcium-äquivalent steht; sowie – für den Fall, daß M für Wasserstoff steht – die 1 : 1-Addukte der vorausgehend definierten Verbindungen mit Salzsäure, Schwefelsäure, Benzolsulfonsäure und p-Toluolsulfonsäure; oder in welcher

(B) $R^1$ für Wasserstoff oder den Rest $-S(O)_m-R^5$ steht, worin

m für die Zahlen Null, 1 oder 2 steht und

$R^5$ für den Rest

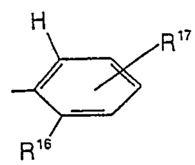

steht, worin

$R^{16}$ und $R^{17}$ für Wasserstoff stehen, oder

$R^{16}$ für Chlor, Nitro, Methyl, Trifluormethyl oder für Methoxy und

$R^{17}$ für Fluor, Chlor, Brom, Methyl, Trifluormethyl, Cyano, Nitro oder Methoxy steht; in welcher weiter

$R^2$ für den Rest

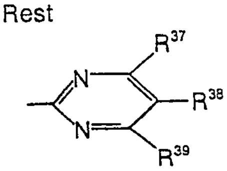

steht, worin

$R^{37}$ für Wasserstoff, Methyl oder Methoxy steht,

$R^{38}$ für Wasserstoff, Chlor, Methyl, Acetyl oder Methoxycarbonyl steht und

$R^{39}$ für $C_1$–$C_4$-Alkyl oder $C_1$–$C_4$-Alkoxy oder

$R^{38}$ und $R^{39}$ zusammen für $C_3$–$C_4$-Alkandiyl stehen, in welcher weiter

$R^3$ für Wasserstoff, Methyl oder den Rest $-S(O)_n-R^6$ steht, worin

n für die Zahlen Null, 1 oder 2 steht und

$R^6$ die oben für $R^5$ als bevorzugt angegebene Bedeutung hat; in welcher weiter

$R^4$ für $C_1$–$C_4$-Alkoxy, $C_3$–$C_4$-Alkenoxy, $C_3$–$C_4$-Alkinoxy, Benzoyloxy oder für den Rest

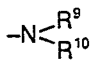

steht, worin

$R^9$ für Wasserstoff oder Methyl steht und

$R^{10}$ für $C_1$–$C_3$-Alkyl, Phenyl, Acetyl, Methoxycarbonyl, Phenylsulfonyl oder p-Toluolsulfonyl steht; in welcher weiter

$R^4$ für Hydroxy steht mit der Maßgabe, daß dann $R^3$ von Wasserstoff verschieden ist; in welcher weiter

M für Wasserstoff, Natrium, Kalium, ein Magnesium- oder Calcium-äquivalent steht, sowie – für den Fall, daß M für Wasserstoff steht – die 1 : 1-Addukte der vorausgehend definierten Verbindungen mit Salzsäure, Schwefelsäure, Benzolsulfonsäure und p-Toluolsulfonsäure; oder in welcher

(C) $R^1$ für den Rest $-S(O)_m-R^5$ steht, worin

m für die Zahl 2 steht und

$R^5$ für den Rest

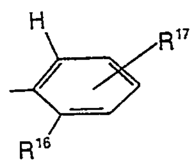

steht, worin

$R^{16}$ für Fluor, Chlor, Brom, Methyl, Trifluormethyl, $C_1$–$C_2$-Alkoxy, Difluormethoxy, Trifluormethoxy oder $C_1$–$C_2$-Alkoxy-carbonyl steht und

$R^{17}$ für Wasserstoff steht; in welcher weiter

$R^2$ für den Rest

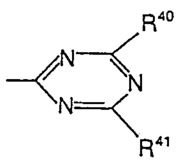

steht, worin

$R^{40}$ für Methyl, Methoxy oder Ethoxy steht und

$R^{41}$ für Methyl, Methoxy oder Ethoxy steht; in welcher weiter

$R^3$ für Wasserstoff, Methyl oder den Rest $-S(O)_n-R^6$ steht, worin

n für die Zahl 2 steht und

$R^6$ die oben für $R^5$ angegebene Bedeutung hat; in welcher weiter

$R^4$ für $C_1$–$C_4$-Alkoxy, $C_3$–$C_4$-Alkenoxy, $C_3$–$C_4$-Alkinoxy, Benzyloxy oder für den Rest

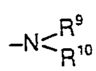

steht, worin

$R^9$ für Wasserstoff oder Methyl steht und

$R^{10}$ für $C_1$–$C_3$-Alkyl, Phenyl, Acetyl, Methoxycarbonyl, Phenylsulfonyl oder p-Toluolsulfonyl steht; in welcher weiter

M für Wasserstoff, Natrium, Kalium, ein Magnesium- oder Calcium-äquivalent steht; sowie – für den Fall, daß M für Wasserstoff steht – die 1 : 1-Addukte der vorausgehend definierten Verbindungen mit Salzsäure, Schwefelsäure, Benzolsulfonsäure und p-Toluolsulfonsäure; oder in welcher

(D) $R^1$ für Wasserstoff oder den Rest $-S(O)_m-R^5$ steht, worin

m für die Zahlen Null, 1 oder 2 steht und

$R^5$ für den Rest

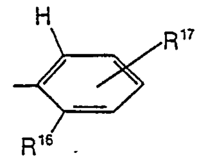

steht, worin

$R^{16}$ und $R^{17}$ beide für Wasserstoff stehen, oder

$R^{16}$ für Chlor, Nitro, Methyl, Trifluormethyl oder Methoxy und

$R^{17}$ für Fluor, Chlor, Brom, Methyl, Trifluormethyl, Cyano, Nitro oder Methoxy steht; in welcher weiter

$R^2$ für den Rest

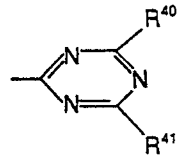

steht, worin

$R^{40}$ für Methyl, Methoxy oder Ethoxy steht und

$R^{41}$ für Methyl, Methoxy oder Ethoxy steht; in welcher weiter

$R^3$ für Wasserstoff, Methyl oder den Rest $-S(O)_n-R^6$ steht, worin

n für die Zahlen Null, 1 oder 2 steht und

$R^6$ die oben für $R^5$ als bevorzugt angegebene Bedeutung hat; in welcher weiter

$R^4$ für $C_1$–$C_4$-Alkoxy, $C_3$–$C_4$-Alkenoxy, $C_3$–$C_4$-Alkinoxy, Benzyloxy oder für den Rest

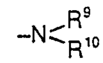

steht, worin

$R^9$ für Wasserstoff oder Methyl steht und

$R^{10}$ für $C_1$–$C_3$-Alkyl, Phenyl, Acetyl, Methoxycarbonyl, Phenylsulfonyl oder p-Toluolsulfonyl steht; in welcher weiter

$R^4$ für Hydroxy steht mit der Maßgabe, daß dann $R^3$ von Wasserstoff verschieden ist; in welcher weiter

M für Wasserstoff, Natrium, Kalium, ein Magnesium- oder Calcium-äquivalent steht, sowie – für den Fall, daß M für Wasserstoff steht – die 1 : 1-Addukte der vorausgehend definierten Verbindungen mit Salzsäure, Schwefelsäure, Benzolsulfonsäure und p-Toluolsulfonsäure.

Verwendet man beispielsweise für die Verfahrensvariante (a) O-Isopropyl-hydroxylamin-Hydrochlorid und 2-Cyanamino-4-methoxy-6-methyl-pyrimidin als Ausgangsstoffe, so kann der Reaktionsablauf durch folgendes Formelschema skizziert werden:

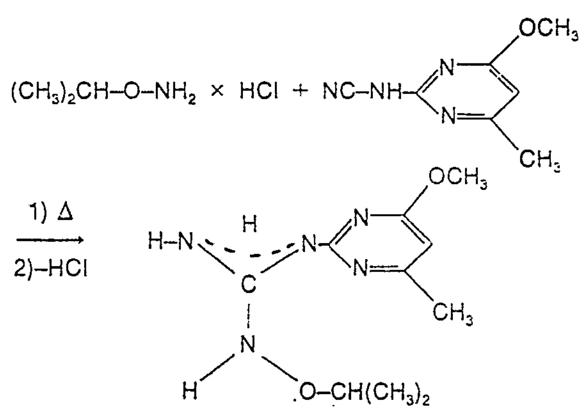

Verwendet man beispielsweise für die Verfahrensvariante (b) 2-Difluormethoxy-benzolsulfonsäurechlorid und N'-(4-Methoxy-6-methyl-s-triazin-2-yl)-N''-dimethyl-amino-guanidin als Ausgangsstoffe, so kann der Reaktionsablauf durch das folgende Formelschema skizziert werden:

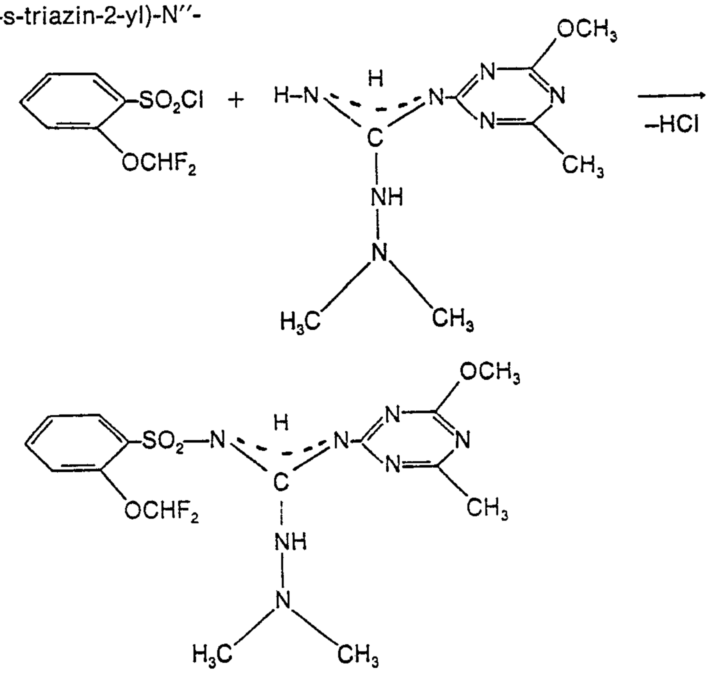


Verwendet man beispielsweise für die Verfahrensvariante (c) N'-(4-Methoxy-6-methyl-pyrimidin-2-yl)-N''-(2-fluor-benzolsulfonyl)-S-methyl-isothioharnstoff und Diethylamin als Ausgangsstoffe und behandelt das zunächst anfallende Ammoniumsalz mit Salzsäure, so kann der Reaktionsablauf durch folgendes Formelschema skizziert werden:

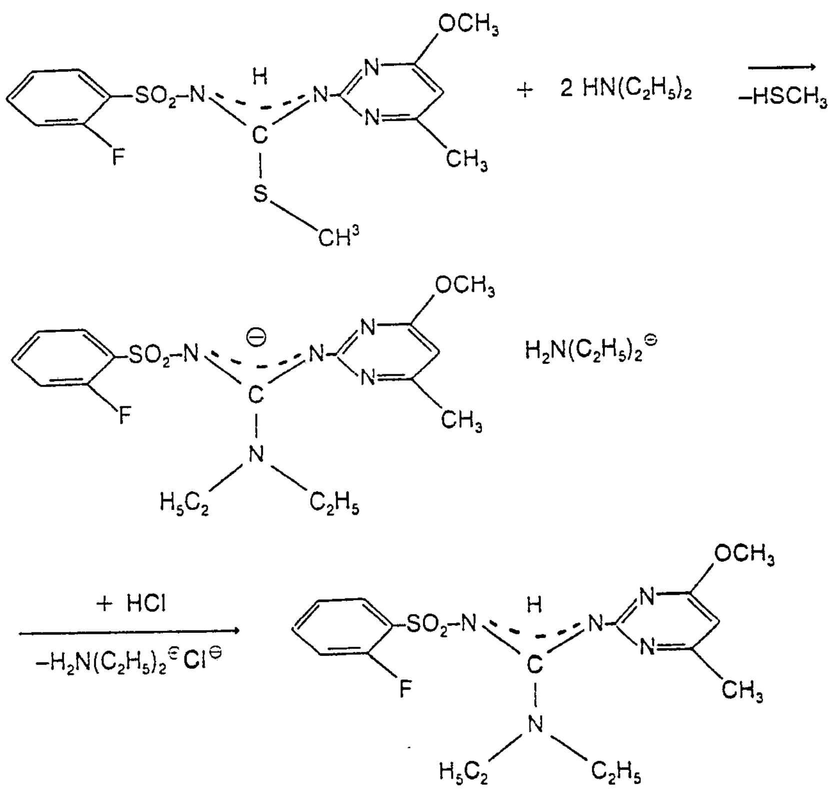


Verwendet man beispielsweise für die Verfahrensvariante (d) N'-(4,6-Dimethyl-pyrimidin-2-yl)-N''-methoxy-N'', N'''-bis-(2-chlor-benzolsulfonyl)-guanidin und N,N-Dimethyl-hydrazin als Ausgangsstoffe, so kann der Reaktionsablauf durch folgendes Formelschema skizziert werden:

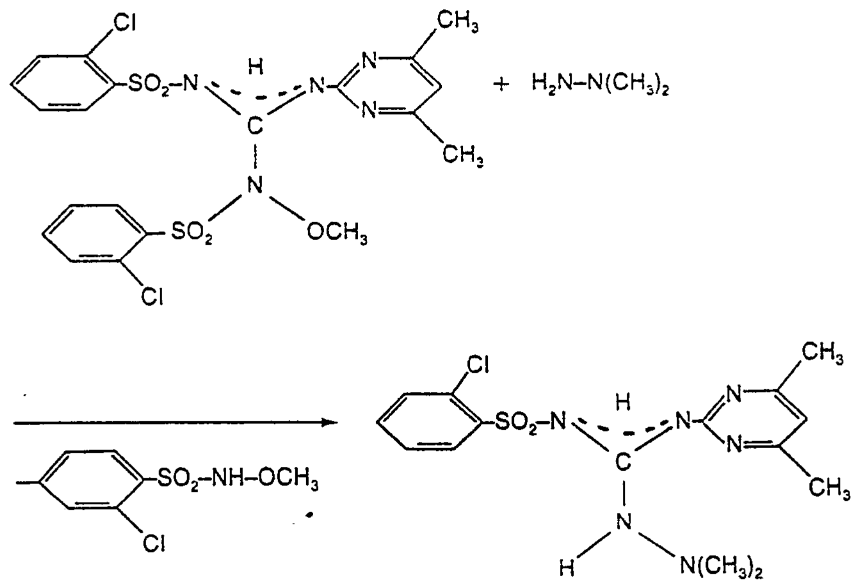


Verwendet man beispielsweise für die Verfahrensvariante (e) N′-(4,6-Dimethoxy-pyrimidin-2-yl)-N″-methoxy-N‴-(2-trifluor-methoxy-benzolsulfonyl)-guanidin und Kaliumethanolat als Ausgangsstoffe, so kann der Reaktionsablauf durch das folgende Formelschema skizziert werden:

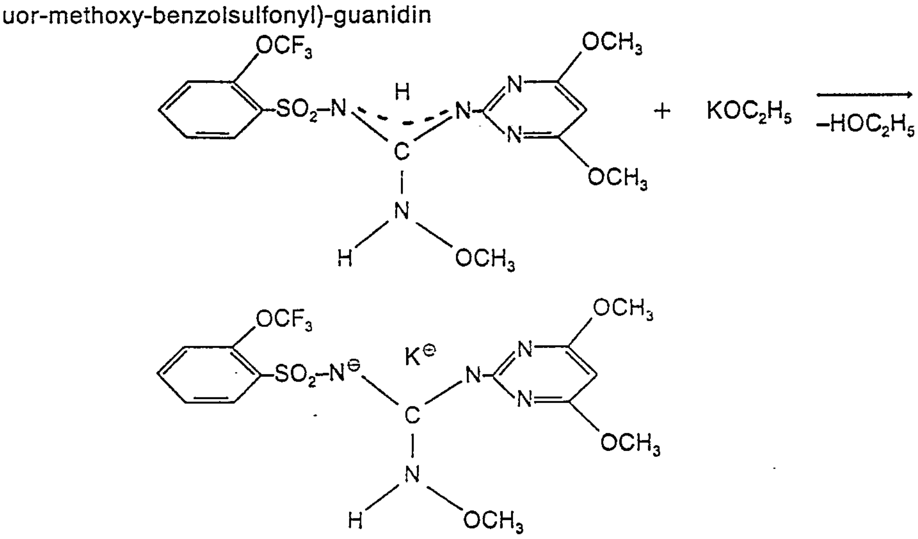


Verwendet man beispielsweise für die Verfahrenvariante (f) N′-(4,6-Dimethyl-pyrimidin-2-yl)-N″-methoxy-N‴-(2-methoxycarbonyl-benzolsulfonyl)-guanidin und Trifluormethan-sulfonsäure als Ausgangsstoffe, so kann der Reaktionsablauf durch folgendes Formelschema skizziert werden:

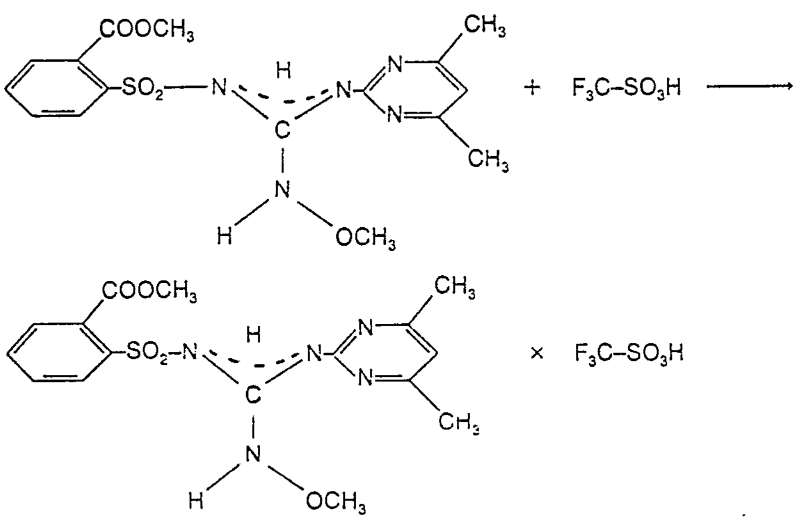

Die als Ausgangsstoffe für die Verfahrensvariante (a) zu verwendenden Cyanoverbindungen sind durch die Formel (II) allgemein definiert. In dieser Formel steht $M^1$ für Wasserstoff, und $R^2$ hat vorzugsweise die gleiche Bedeutung, wie sie oben im Rahmen der Substituentendefinition der Formel (I) vorzugsweise angegeben ist.

Als Ausgangsstoffe der Formel (II) seien beispielsweise genannt:
2-Cyanamino-4,6-dimethyl-pyrimidin,
2-Cyanamino-4-methoxy-6-methyl-pyrimidin,
2-Cyanamino-4,6-dimethoxy-pyrimidin,
2-Cyanamino-4-ethoxy-6-methyl-pyrimidin,
2-Cyanamino-4-methyl-6-propoxy-pyrimidin,
2-Cyanamino-4-methyl-6-isopropoxy-pyrimidin,
2-Cyanamino-4-methyl-6-butoxy-pyrimidin,
2-Cyanamino-4-methyl-6-isobutoxy-pyrimidin,
2-Cyanamino-4,6-dimethyl-s-triazin,
2-Cyanamino-4-methoxy-6-methyl-s-triazin,
2-Cyanamino-4,6-dimethoxy-s-triazin,
2-Cyanamino-4-ethoxy-6-methyl-s-triazin,
2-Cyanamino-5-chlor-4,6-dimethyl-pyrimidin und
2-Cyanamino-4,5,6-trimethyl-pyrimidin.

Von den Cyanoverbindungen der Formel (II) ist unseres Wissens nur eine Verbindung vorbekannt, nämlich
2-Cyanamino-4,6-dimethyl-pyrimidin
(vgl. J. Chem. Soc. 1953, S. 1725–1730). Man erhält die Verbindungen der Formel (II) im wesentlichen nach folgenden zwei Synthesewegen:

($a^1$) allgemein durch Umsetzung von Alkalimetall- oder Erdalkalimetall-Salzen von Cyanamid – wie z.B. Natriumcyanamid oder Calciumcyanamid – mit Halogenverbindungen der Formel (VII)

$$Hal^1–R^2 \qquad (VII)$$

in welcher
$R^2$ die oben angegebene Bedeutung hat und
$Hal^1$ für Fluor, Chlor, Brom oder Iod, insbesondere für Chlor, steht, gegebenenfalls in Gegenwart von inerten Verdünnungsmitteln, wie z.B. Aceton, Acetonitril oder Dimethylformamid, bei Temperaturen zwischen 0 °C und 150 °C, vorzugsweise zwischen 10 °C und 100 °C; nach Abdestillieren der flüchtigen Komponente und Auflösen des Rückstandes in Wasser können die Cyanoverbindungen der Formel (II) durch Ansäuern, z.B. mit Salzsäure, ausgefällt und durch Absaugen isoliert werden; oder

($a^2$) für den Fall, daß $R^2$ für einen substituierten Pyrimidinylrest steht, durch Umsetzung von Cyanoguanidin (‹Dicyandiamid›) mit β-Dicarbonylverbindungen, wie z.B. Acetylaceton (vgl. J. Chem. Soc. 1953, 1725–1730), Acetessigsäureestern (vgl. J. Prakt. Chem. 77, (1908), 542 und J. Chem. Soc. 1948, 586) oder Malonsäureestern (vgl. DE-PS 158 591).

Die aus Acetessigsäureestern bzw. Malonsäureestern erhaltenen 2-Cyanamino-4-hydroxy-6-methyl- bzw. -4,6-dihydro-pyrimidine können auf bekannte Weise durch Umsetzung mit Alkylierungsmitteln, wie z.B. Dimethyl- oder Diethylsulfat, gegebenenfalls in Gegenwart von Verdünnungsmitteln, wie z.B. Wasser, Methanol, Ethanol, n- und iso-Propanol, Aceton, Dioxan oder Dimethylformamid, und in Gegenwart von Säurebindemitteln, wie z.B. Natrium- oder Kalium-hydroxid, Natrium- oder Kalium-carbonat, in entsprechende 2-Cyanamino-4-alkoxy-6-methyl- bzw. -4,6-dialkoxy-pyrimidine umgewandelt werden. Zur Vermeidung einer N-Alkylierung wird gegebenenfalls mit einem Acylierungsmittel, wie z.B. Acetanhydrid oder Acetylchlorid acyliert und nach der Alkylierung mit wäßrigen Säuren oder Basen wieder entacyliert.

Die Halogenverbindungen der Formel (VII) sind bekannt (vgl. J. Chem. Soc. (C) 1966, 2031; Chem. Pharm. Bull. 11 (1963), 1382–1388; Arch. Pharm. 295 (1962), 649–657).

Die weiter als Ausgangsstoffe für die Verfahrensvariante (a) zu verwendenden Aminoverbindungen der Formel (III) sind weitgehend bekannt oder können nach an sich bekannten Verfahren hergestellt werden (vgl. Chem. Pharm. Bull. 15 (1967), 345–349; Bull. Soc. Chem. France 1958, 664; Synthesis 1976, 682).

In Formel (III) hat $R^4$ vorzugsweise die gleiche Bedeutung, wie sie oben im Rahmen der Substituentendefinition der Formel (I) vorzugsweise angegeben ist und $R^3$ steht vorzugsweise für Wasserstoff, $C_1$–$C_4$-Alkyl [welches gegebenenfalls durch Fluor, Chlor, Brom, Cyano, Hydroxy oder $C_1$–$C_4$-Alkoxy substituiert ist], $C_3$–$C_6$-Cycloalkyl, $C_3$–$C_6$-Alkenyl, $C_3$–$C_6$-Alkinyl oder Benzyl [welches gegebenenfalls durch Fluor, Chlor oder Methyl substituiert ist].

Insbesondere bevorzugt sind Ausgangsstoffe der Formel (III), in welcher $R^4$ die gleiche Bedeutung hat, wie sie oben im Rahmen der Substituentendefinition der Formel (I) als bevorzugt angegeben ist und $R^3$ für Wasserstoff, gegebenenfalls durch Hydroxy substituiertes $C_1$–$C_4$-Alkyl, $C_5$–$C_6$-Cycloalkyl, $C_3$–$C_4$-Alkenyl, $C_3$–$C_4$-Alkinyl oder Benzyl steht.

Als Ausgangsstoffe der Formel (III) seien beispielsweise genannt:

Dimethylamin, Diethylamin, Dipropylamin, Diisopropylamin, Dibutylamin, Dicyclopentylamin, Dicyclohexylamin, Diallylamin, Dipropargylamin, Dibenzylamin, N-Methyl-anilin, O-Methyl-hydroxylamin, O-Ethyl-hydroxylamin, O-Propyl-hydroxylamin, O-Isopropyl-hydroxylamin, O-Butylhydroxylamin, O-Isobutyl-hydroxylamin, O-Allylhydroxylamin, O-Propargyl-hydroxylamin, O-Benzyl-hydroxylamin, N,O-Dimethyl-hydroxylamin, Methylhydrazin, N,N-Dimethylhydrazin, N,N'-Dimethylhydrazin, Ethylhydrazin, n- und iso-Propylhydrazin sowie die Hydrochloride dieser Verbindungen; Phenylhydrazin, Acethydrazin, Hydrazinoameisensäure-methylester, Benzolsulfonsäurehydrazid und p-Toluolsulfonsäurehydrazid.

Die bei Verfahren (b) als Ausgangsstoffe zu verwendenden Guanidin-Derivate sind durch die Formel (I) und den oben unter (b) genannten Bedingungen allgemein definiert. In dieser Formel – soweit sie die als Ausgangsstoffe für Verfahren (b) zu verwendenden Guanidine betrifft – stehen vorzugsweise

$R^1$ für Wasserstoff,

$R^3$ für Wasserstoff, $C_1$–$C_4$-Alkyl [welches gegebenenfalls durch Fluor, Chlor, Brom, Cyano, Hydroxy oder $C_1$–$C_2$-Alkoxy substituiert ist], $C_3$–$C_6$-Cycloalkyl, $C_3$–$C_6$-Alkenyl, $C_3$–$C_6$-Alkinyl oder Benzyl [welches gegebenenfalls durch Fluor, Chlor oder Methyl substituiert ist],

M für Wasserstoff, und

$R^2$ und $R^4$ haben vorzugsweise die gleichen Bedeutungen, wie sie oben im Rahmen der Substituentendefinition der Formel (I) vorzugsweise angegeben sind.

Als Ausgangsstoffe für die Verfahrensvariante (b) werden insbesondere die Guanidin-Derivate der Formel (I) bevorzugt, in welcher

$R^1$ für Wasserstoff steht,

$R^3$ für Wasserstoff, gegebenenfalls durch Hydroxy substituiertes Alkyl, $C_5$–$C_6$-Cycloalkyl, $C_3$–$C_4$-Alkenyl, $C_3$–$C_4$-Alkinyl oder Benzyl steht,

M für Wasserstoff steht und

$R^2$ und $R^4$ die gleichen Bedeutungen haben, wie sie oben im Rahmen der Substituentendefinition der Formel (I) als bevorzugt angegeben sind.

Als Beispiele für Guanidin-Derivate der Formel (I), welche beim Herstellungsverfahren (b) als Ausgangsstoffe einzusetzen sind, seien genannt:

N'-(4,6-Dimethyl-pyrimidin-2-yl)-,
N'-(4-Methoxy-6-methyl-pyrimidin-2-yl)-,
N'-(4-Ethoxy-6-methyl-pyrimidin-2-yl)-,
N'-(4-Propoxy-6-methyl-pyrimidin-2-yl)-,
N'-(4-Isopropoxy-6-methyl-pyrimidin-2-yl)-,
N'-(4-Butoxy-6-methyl-pyrimidin-2-yl)-,
N'-(4-Isobutoxy-6-methyl-pyrimidin-2-yl)-,
N'-(4,6-Dimethoxy-pyrimidin-2-yl)-,
N'-(4,6-Dimethyl-s-triazin-2-yl)-,
N'-(4-Methoxy-6-methyl-s-triazin-2-yl)-,
N'-(4-Ethoxy-6-methyl-s-triazin-2-yl)-,
N'-(4,6-Dimethoxy-s-triazin-2-yl)-,
N'-(4,5,6-Trimethyl-pyrimidin-2-yl) und
N'-(5-Chlor-4,6-dimethyl-pyrimidin-2-yl)-guanidin,
-N''-methyl-guanidin,
-N''-ethyl-guanidin,
-N''-propyl-guanidin,
-N''-isopropyl-guanidin,
-N''-butyl-guanidin,
-N''-isobutyl-guanidin,
-N''-sec.-butyl-guanidin,
-N''-tert.-butyl-guanidin,
-N''-cyclopentyl-guanidin,
-N''-cyclohexyl-guanidin,
-N''-allyl-guanidin,
-N''-propargyl-guanidin,
-N''-benzyl-guanidin,
-N''-phenyl-guanidin,
-N''-(2-fluor-phenyl)-,
-(3-fluor-phenyl)- und
-(4-fluor-phenyl)-guanidin,
-N''-(2-chlor-phenyl)-,
-(3-chlor-phenyl)- und
-(4-chlor-phenyl)-guanidin,
-N''-(2-brom-phenyl)-,
-(3-brom-phenyl)- und
-(4-brom-phenyl)-guanidin,
-N''-(2-nitro-phenyl)-,
-(3-nitro-phenyl)- und
-(4-nitro-phenyl)-guanidin,
-N''-(2-aminophenyl)-,
-(3-aminophenyl)- und
-(4-aminophenyl)-guanidin,
-N''-(2-cyano-phenyl),
-(3-cyano-phenyl)- und
-(4-cyano-phenyl)-guanidin,
-N''-(4-amino-sulfonyl-phenyl)-guanidin,
-N''-(2-hydroxy-phenyl)-,
-(3-hydroxy-phenyl)- und
-(4-hydroxy-phenyl)-guanidin,
-N''-(2-methyl-phenyl)-,
-(3-methyl-phenyl)- und
-(4-methyl-phenyl)-guanidin,
-N''-(2-trifluormethyl-phenyl)-,
-(3-trifluormethyl-phenyl)- und
-(4-trifluormethyl-phenyl)-guanidin,
-N''-(2-methoxy-phenyl)-,
-(3-methoxy-phenyl)- und
-(4-methoxy-phenyl)-guanidin,
-N''-(2-trifluormethoxy-phenyl)- und
-(4-trifluormethoxy-phenyl)-guanidin,
-N''-(4-trifluormethylthio-phenyl)-guanidin,
-N''-(2-methoxycarbonyl-phenyl)- und
-(4-methoxycarbonyl-phenyl)-guanidin,
-N'',N''-dimethyl-guanidin,
-N'',N''-diethyl-guanidin,
-N'',N''-dipropyl-guanidin,
-N'',N''-diisopropyl-guanidin,
-N'',N''-dibutyl-guanidin,
-N'',N''-diisobutyl-guanidin,
-N'',N''-dicyclopentyl-guanidin,
-N'',N''-dicyclohexyl-guanidin,
-N'',N''-diallyl-guanidin,
-N'',N''-dipropargyl-guanidin,
-N'',N''-dibenzyl-guanidin,
-N''-methyl-N''-phenyl-guanidin,
-N''-methoxy-guanidin,
-N''-ethoxy-guanidin,
-N''-propoxy-guanidin,
-N''-isopropoxy-guanidin,
-N''-butoxy-guanidin,
-N''-isobutoxy-guanidin,
-N''-allyloxy-guanidin,
-N''-propargyloxy-guanidin,
-N''-benzyloxy-guanidin,
-N''-methyl-N''-methoxy-guanidin,
-N''-methylamino-guanidin,
-N''-dimethylamino-guanidin,
-N''-methyl-N''-methylamino-guanidin,
-N''-ethylamino-guanidin,
-N''-propylamino-guanidin,
-N''-isopropylamino-guanidin,
-N''-morpholino-guanidin,
-N''-acetamino-guanidin,
-N''-methoxycarbonylamino-guanidin,
-N''-benzolsulfonylamino-guanidin und
-N''-p-toluolsulfonamino-guanidin.

Die als Ausgangsstoffe für das Verfahren (b) zu verwendenden Guanidin-Derivate der Formel (I) sind weitgehend noch nicht in der Literatur beschrieben und können nach dem erfindungsgemäßen Verfahren (a) hergestellt werden.

Die bei Verfahren (b) weiter als Ausgangsstoffe zu verwendenden Halogen-Schwefel-Verbindungen sind durch die Formeln (IV) und (V) allgemein definiert. In diesen Formeln haben m, n, $R^5$ und $R^6$ vorzugsweise die gleichen Bedeutungen, wie sie oben im Rahmen der Substituentendefinition der Formel (I) vorzugsweise angegeben sind und $X^1$ und $X^2$ stehen vorzugsweise für Chlor.

Als Ausgangsstoffe der Formeln (IV) und (V) seien genannt:

2-Chlor-, 2-Fluor-, 2-Brom-, 2-Nitro-, 2-Methyl-, 2-Methoxycarbonyl-, 2-Ethoxycarbonyl-, 2-Methoxy-, 2-Ethoxy-, 2-Phenyl-, 2-Trifluormethyl-, 2-Difluormethoxy-, 2-Trifluormethoxy-, 2-Methyl-5-chlor-, 2,5-Dichlor- und 2-Chlor-5-trifluormethyl-benzolsulfonsäurechlorid sowie die entsprechenden Sulfensäure- und Sulfinsäure-chloride.

Die Halogen-Schwefel-Verbindungen der Formeln (IV) und (V) sind zum Teil bekannt (vgl. Chemistry Lett. 1978, 951; EP-PA 23 422, 35 893, 42 731, 44 808, 44 809, 51 466, 64 804 und 70 041; US-PS 2 929 820, 4 282 242 und 4 372 778; J. Org. Chem. 33 (1968), 2104).

Man erhält die Verbindungen der Formeln (IV) bzw. (V), in welcher m bzw. n für die Zahl 2 stehen im wesentlichen nach folgenden zwei Synthesemethoden:

($b^1$) durch Umsetzung der entsprechenden Sulfonsäuren $R^5–SO_3H$ bzw. $R^6–SO_3H$ bzw. von deren Alkalimetall- oder Erdalkalimetall-Salzen mit Halogenierungsmitteln, wie z.B. Phosphor(V)chlorid (Phosphorpentachlorid), Phosphorylchlorid (Phosphoroxychlorid), Thionylchlorid, Phosgen oder Benzotrichlorid, gegebenenfalls in Gegenwart von Katalysatoren, wie z.B. Pyridin oder Dimethylformamid, und gegebenenfalls unter Verwendung von inerten Verdünnungsmitteln, wie z.B. Methylenchlorid, Chloroform, Acetonitril, Chlorbenzol und/oder Sulfolan bei Temperaturen zwischen −20 °C und +150 °C, vorzugsweise zwischen 0 °C und +100 °C; nach Verdünnen mit Wasser können die Sulfonsäurechloride – soweit sie kristallin anfallen – durch Absaugen isoliert werden oder durch Extrahieren mit einem mit Wasser nicht mischbaren Lösungsmittel, wie z.B. Methylenchlorid, Diethylether oder Hexan, Waschen und Trocknen der Extrakte, Einengen und Umkristallisieren bzw. Destillieren gereinigt werden; oder auch

($b^2$) für den Fall, daß $X^1$ und $X^2$ für Chlor stehen und $R^5$ bzw. $R^6$ für einen aromatischen Rest stehen, auf an sich bekannte Weise (vgl. J. Org. Chem. 25 (1960), 1824; DE-OS 2 308 262 und EP-PA 59 241) durch Umsetzung entsprechender Aminoverbindungen $R^5–NH_2$ bzw. $R^6–NH_2$ mit Natriumnitrit und Salzsäure, gegebenenfalls in Gegenwart von Essigsäure, bei Temperaturen zwischen −10 °C und +20 °C, vorzugsweise zwischen −5 °C und +10 °C, und anschließend (in situ) mit Schwefeldioxid oder einem Salz der schwefeligen Säure, wie z.B. Natriumsulfit oder Natriumhydrogensulfit in Gegenwart einer Kupferverbindung, wie z.B. Kupferchlorid oder Kupfersulfat, als Katalysator bei Temperaturen zwischen 0 °C und 80 °C, vorzugsweise zwischen 10 °C und 60 °C.

Die Aufarbeitung kann auf übliche Weise erfolgen: Bei Verdünnen mit Wasser fallen die Sulfonsäurechloride im allgemeinen kristallin an und können durch Absaugen isoliert werden. Sie können aber auch aus der wäßrigen Dispersion mit einem mit Wasser praktisch nicht mischbaren Lösungsmittel, wie z.B. Methylenchlorid oder Diethylether, extrahiert, getrocknet und durch Vakuumdestillation gereinigt werden.

Die bei Verfahren (c) als Ausgangsstoffe zu verwendenden Isothioharnstoffe sind durch die Formel (VI) definiert. In dieser Formel steht $R^{15}$ vorzugsweise für $C_1–C_4$-Alkyl oder Benzyl, insbesondere für Methyl, und $R^1$, $R^2$ und M haben vorzugsweise die gleichen Bedeutungen, wie sie oben im Rahmen der Substituentendefinition der Formel (I) vorzugsweise angegeben sind.

Als Beispiele für die Ausgangsstoffe der Formel (VI) seien genannt:

N′-(4,6-Dimethoxy-s-triazin-2-yl)-,
N′-(4,6-Dimethyl-pyrimidin-2-yl)-,
N′-(4-Methoxy-6-methyl-pyrimidin-2-yl)-,
N′-(4-Ethoxy-6-methyl-pyrimidin-2-yl)-,
N′-(4-Propoxy-6-methyl-pyrimidin-2-yl)-,
N′-(4-Isopropoxy-6-methyl-pyrimidin-2-yl)-,
N′-(4-Butoxy-6-methyl-pyrimidin-2-yl)-,
N′-(4-Isobutoxy-6-methyl-pyrimidin-2-yl)-,
N′-(4,6-Dimethoxy-pyrimidin-2-yl)-,
N′-(4,6-Dimethyl-s-triazin-2-yl)-,
N′-(4-Methoxy-6-methyl-s-triazin-2-yl)-,
N′-(4-Ethoxy-6-methyl-s-triazin-2-yl)-,
N′-(4,5,6-Trimethyl-pyrimidin-2-yl)- und
N′-(5-Chlor-4,6-dimethyl-pyrimidin-2-yl)-,
-N″-(2-fluor-benzolsulfonyl)-,
-N″-(2-chlor-benzolsulfonyl)-,
-N″-(2-brom-benzolsulfonyl)-,
-N″-(2-nitro-benzolsulfonyl)-,
-N″-(2-methyl-benzolsulfonyl)-,
-N″-(2-methoxycarbonyl-benzolsulfonyl)-,
-N″-(2-ethoxycarbonyl-benzolsulfonyl)-,
-N″-(2-methoxy-benzolsulfonyl)-,
-N″-(2-ethoxy-benzolsulfonyl)-,
-N″-(2-phenyl-benzolsulfonyl)-,
-N″-(2-trifluormethyl-benzolsulfonyl)-,
-N″-(2-difluormethoxy-benzolsulfonyl)-,
-N″-(2-trifluormethoxy-benzolsulfonyl)-,
-N″-(2-methyl-5-chlor-benzolsulfonyl)-,
-N″-(2,5-dichlor-benzolsulfonyl)- und
-N″-(2-chlor-5-trifluormethyl-benzolsulfonyl)-S-methyl-isothioharnstoff.

Die Isothioharnstoffe der Formel (VI) sind zum Teil bekannt (vgl. EP-PA 5 986). Man erhält diese Verbindungen auf an sich bekannte Weise durch Umsetzung von entsprechenden Isodithiocarbamidsäure-Derivaten der Formel (VIII)

$$R^1–N=C\begin{matrix}\diagup S–R^{15}\\ \diagdown S–R^{15}\end{matrix} \qquad (VIII)$$

in welcher

$R^1$ und $R^{15}$ die oben angegebenen Bedeutungen haben, mit Amino-hetarenen der Formel (IX)

$$M–NH–R^2 \qquad (IX)$$

in welcher

M und $R^2$ die oben angegebenen Bedeutungen haben, gegebenenfalls in Gegenwart einer starken, aber schwach nucleophilen Base, wie z.B. Natriumhydrid, und gegebenenfalls in Gegenwart

eines Verdünnungsmittels, wie z.B. Tetrahydrofuran, Dioxan, 1,2-Dimethoxyethan, Dimethylformamid oder Dimethylsulfoxid, bei Temperaturen zwischen −20 °C und 100 °C, vorzugsweise zwischen 0 °C und +80 °C. Die Aufarbeitung kann nach üblichen Methoden durchgeführt werden, beispielsweise durch Verdünnen mit Wasser und Ansäuern, z.B. mit Salzsäure, wonach die kristallin anfallenden Produkte der Formel (VI) durch Absaugen isoliert werden können.

Die als Zwischenprodukte benötigten Isodithiocarbonsäure-Derivate sind durch die Formel (VIII) definiert. In dieser Formel haben $R^1$ und $R^{15}$ vorzugsweise die gleichen Bedeutungen, wie sie oben im Rahmen der Substituentendefinitionen für Formel (I) bzw. (VI) vorzugsweise angegeben sind.

Als Beispiele für die Verbindungen der Formel (VIII) seien genannt:
N-(2-Fluor-benzolsulfonyl)-,
N-(2-Chlor-benzolsulfonyl)-,
N-(2-Brom-benzolsulfonyl)-,
N-(2-Nitro-benzolsulfonyl)-,
N-(2-Methyl-benzolsulfonyl)-,
N-(2-Methoxycarbonyl-benzolsulfonyl)-,
N-(2-Ethoxycarbonyl-benzolsulfonyl)-,
N-(2-Methoxy-benzolsulfonyl)-,
N-(2-Ethoxy-benzolsulfonyl)-,
N-(2-Phenyl-benzolsulfonyl)-,
N-(2-Difluormethoxy-benzolsulfonyl)-,
N-(2-Trifluormethoxy-benzolsulfonyl)-,
N-(2-Methyl-5-chlor-benzolsulfonyl)-,
N-(2,5-Dichlor-benzolsulfonyl)- und
N-(2-Chlor-5-trifluormethyl-benzolsulfonyl)-S′,S″-dimethyl-isodithiocarbamidsäureester.

Die Isodithiocarbamidsäure-Derivate der Formel (VIII) sind weitgehend noch nicht in der Literatur beschrieben. Man erhält diese Verbindungen auf an sich bekannte Weise (vgl. Chem. Ber. 99 (1966), 2885) durch Umsetzung von Aminoverbindungen der Formel (X)

$$R^1\text{–}NH_2 \qquad (X)$$

in welcher

$R^1$ die oben angegebene Bedeutung hat, mit Schwefelkohlenstoff in Gegenwart einer starken Base, wie z.B. Natriumhydroxid, und gegebenenfalls in Gegenwart von Verdünnungsmitteln, wie z.B. Wasser und Dimethylformamid, bei Temperaturen zwischen −20 °C und +150 °C, vorzugsweise zwischen 0 °C und 100 °C, und anschließende Umsetzung (in situ) mit einem Alkylierungsmittel der Formel (XI)

$$Hal^2\text{–}R^{15} \qquad (XI)$$

in welcher

$R^{15}$ die oben angegebene Bedeutung hat und

$Hal^2$ für Chlor, Brom oder Iod steht, bei Temperaturen zwischen −20 °C und +150 °C, vorzugsweise zwischen 0 °C und 100 °C.

Die nach Verdünnen mit Wasser kristallin anfallenden Produkte der Formel (VIII) können durch Absaugen isoliert werden.

In Formel (X) hat $R^1$ vorzugsweise die gleiche Bedeutung, wie sie oben im Rahmen der Substituentendefinition für Formel (I) vorzugsweise angegeben ist.

Als Beispiele für die Verbindungen der Formel (X) seien genannt: 2-Fluor-, 2-Chlor-, 2-Brom-, 2-Nitro-, 2-Methyl-, 2-Methoxycarbonyl-, 2-Ethoxycarbonyl-, 2-Methoxy-, 2-Ethoxy-, 2-Phenyl-, 2-Difluormethoxy-, 2-Trifluormethoxy-, 2-Methyl-5-chlor-, 2,5-Dichlor- und 2-Chlor-5-trifluormethylbenzolsulfonsäureamid.

Die Aminoverbindungen der Formel (X) sind zum Teil bekannt (vgl. EP-PA 23 422, 30 140, 35 893, 44 807, 44 808, 44 809, 51 466, 64 804, 70 041 und 70 802; US-PS 4 372 778).

Man erhält diese Verbindungen auf an sich bekannte Weise durch Umsetzung entsprechender Chlorverbindungen $R^1$–Cl mit Ammoniak, gegebenenfalls unter Verwendung inerter Verdünnungsmittel, wie z.B. Diethylether oder Tetrahydrofuran, bei Temperaturen zwischen −20 °C und +100 °C, vorzugsweise zwischen 0 °C und 50 °C. Die hierbei kristallin anfallenden Produkte der Formel (X) können durch Absaugen isoliert werden.

Beispiele für geeignete Vorprodukte der Formel $R^1$–Cl und Herstellungsmethoden hierfür sind oben bei der Beschreibung der Ausgangsstoffe für Verfahren (b) aufgeführt.

In Formel (XI) steht $R^{15}$ vorzugsweise für $C_1$–$C_4$-Alkyl oder Benzyl, insbesondere für Methyl, und $Hal^2$ für Chlor, Brom oder Iod.

Als Beispiele für die Verbindungen der Formel (XI) seien genannt: Methylchlorid, Methylbromid, Methyliodid, Ethylchlorid, Ethylbromid und Ethyliodid sowie Benzylchlorid und Benzylbromid.

Die Verbindungen der Formel (XI) sind bekannt.

Die weiter als Zwischenprodukte zu verwendenden Aminohetarene sind durch die Formel (IX) definiert. In dieser Formel steht vorzugsweise $R^2$ für die gleichen Reste, wie sie im Rahmen der Substituentendefinition für Formel (I) vorzugsweise angegeben sind und M steht vorzugsweise für Wasserstoff, Natrium, Kalium, ein Magnesium- oder Calciumäquivalent, insbesondere Wasserstoff.

Als Beispiele für die Verbindungen der Formel (IX) seien genannt:
4,6-Dimethyl-, 4,5,6-Trimethyl-,
5-Chlor-4,6-dimethyl-, 4-Methoxy-6-methyl-,
4-Ethoxy-6-methyl-, 4-Propoxy-6-methyl-,
4-Isopropoxy-6-methyl-, 4-Butoxy-6-methyl-,
4-Isobutoxy-6-methyl- und
4,6-Dimethoxy-2-amino-pyrimidin
sowie 4,6-Dimethyl-, 4-Methoxy-6-methyl-, 4-Ethoxy-6-methyl- und 4,6-Dimethoxy-2-amino-s-triazin.

Die Verbindungen der Formel (IX) sind bekannt und/oder können nach an sich bekannten Verfahren hergestellt werden (vgl. Chem. Pharm. Bull. 11 (1963), 1382–1388; US-PS 4 299 960).

Die beim erfindungsgemäßen Verfahren (c) weiter als Ausgangsstoffe zu verwendenden Aminoverbindungen sind durch die Formel (III) definiert. Die bevorzugten Bedeutungen der Formel (III)

sind oben im Rahmen der Beschreibung der Ausgangsstoffe für Verfahren (a) aufgeführt.

Die bei Verfahren (d) als Ausgangsstoffe zu verwendenden Guanidin-Derivate sind durch die Formel (I) und die oben unter (d) genannten Bedingungen allgemein definiert.

In Formel (I) – soweit sie die als Ausgangsstoffe für Verfahren (d) zu verwendenden Guanidin-Derivate betrifft – stehen $R^1$ bzw. $R^3$ vorzugsweise für die Reste $-S(O)_m-R^5$ bzw. $-S(O)_n-R^6$, worin m und n sowie $R^5$ und $R^6$ vorzugsweise die gleichen Bedeutungen haben, wie sie oben im Rahmen der Substituentendefinition für Formel (I) vorzugsweise angegeben sind; ferner haben $R^2$, $R^4$ und M vorzugsweise die gleichen Bedeutungen, wie sie oben im Rahmen der Substituentendefinition für Formel (I) vorzugsweise angegeben sind.

Als Beispiele für die als Ausgangsstoffe in Verfahren (d) zu verwendenden Verbindungen der Formel (I) seien genannt:
N'-(4,6-Dimethyl-pyrimidin-2-yl)-N''-methoxy-N'',N'''-bis-(2-chlor-benzolsulfonyl)-,
-N'',N'''-bis-(2-brom-benzolsulfonyl)-,
-N'',N'''-bis-(2-fluor-benzolsulfonyl)-,
-N'',N'''-bis-(2-methoxy-benzolsulfonyl)-,
-N'',N'''-bis-(2-methyl-benzolsulfonyl)- und
-N'',N'''-bis-(2-methoxycarbonyl-benzolsulfonyl)-guanidin.

Die als Ausgangsstoffe für Verfahren (d) zu verwendenden Guanidin-Derivate der Formel (I) sind noch nicht in der Literatur beschrieben. Sie können nach dem oben unter (b) beschriebenen Herstellungsverfahren erhalten werden.

Die beim erfindungsgemäßen Verfahren (c) weiter als Ausgangsstoffe zu verwendenden Aminoverbindungen sind durch die Formel (III) definiert. Die bevorzugten Bedeutungen von $R^3$ und $R^4$ sowie Beispiele für Verbindungen der Formel (III) sind oben im Rahmen der Beschreibung der Ausgangsstoffe für Verfahren (a) aufgeführt.

Die bei Verfahren (e) als Ausgangsstoffe zu verwendenden Guanidin-Derivate sind durch die Formel (I) und die oben unter (c) genannten Bedingungen definiert. In Formel (I) – soweit sie die als Ausgangsstoffe für Verfahren (e) zu verwendenden Guanidin-Derivate betrifft – steht M für Wasserstoff und die Reste $R^1$, $R^2$, $R^3$ und $R^4$ haben vorzugsweise die gleichen Bedeutungen, wie sie oben im Rahmen der Substituentendefinition für Formel (I) vorzugsweise angegeben sind.

Als Beispiele für die als Ausgangsstoffe in Verfahren (e) zu verwendenden Verbindungen der Formel (I) seien genannt:
N'-(4,6-Dimethyl-pyrimidin-2-yl)-N''-methoxy-N'',N'''-bis-(2-fluor-benzolsulfonyl)-,
-N'',N'''-bis-(2-chlor-benzolsulfonyl)-,
-N'',N'''-bis-(2-brom-benzolsulfonyl)-,
-N'',N'''-bis-(2-methoxy-benzolsulfonyl)-,
-N'',N'''-bis-(2-methyl-benzolsulfonyl)- und
-N'',N'''-bis-(2-methoxycarbonyl-benzolsulfonyl)-guanidin sowie
N'-(4,6-Dimethyl-pyrimidin-2-yl)-N''-methoxy-N'''-(2-fluor-benzolsulfonyl)-
-N'''-(2-chlor-benzolsulfonyl)-,
-N'''-(2-brom-benzolsulfonyl)-,
-N'''-(2-methyl-benzolsulfonyl)-,
-N'''-(2-methoxy-benzolsulfonyl)- und
-N'''-(2-methoxycarbonyl-benzolsulfonyl)-guanidin.

Die als Ausgangsstoffe für Verfahren (e) zu verwendenden Guanidin-Derivate der Formel (I) sind noch nicht in der Literatur beschrieben. Sie können nach den oben unter (a), (b), (c) und (d) beschriebenen Herstellungsverfahren erhalten werden.

Als Beispiele für die bei Verfahren (e) zu verwendenden Metallhydroxide, -hydride, -alkanolate bzw. metallorganischen Verbindungen seien genannt: Natrium-, Kalium-, Magnesium- und Calcium-hydroxid, Natrium- und Calcium-hydrid, Natrium-methanolat und -ethanolat, Kalium-methanolat, -ethanolat und Kalium-tert.-butanolat sowie Isopropylmagnesiumchlorid.

Als Beispiele für die gegebenenfalls bei Verfahren (e) zu verwendenden Amine seien genannt: Isopropylamin, Diisopropylamin, Isobutylamin, sec.-Butylamin, tert.-Butylamin, Diisobutylamin, Trimethylamin, Triethylamin, Dibenzylamin und Ethyl-diisopropylamin.

Die bei Verfahren (f) als Ausgangsstoffe zu verwendenden Guanidin-Derivate sind durch die Formel (I) definiert. In Formel (I) haben $R^1$, $R^2$, $R^3$, $R^4$ und M vorzugsweise die gleichen Bedeutungen, wie sie oben im Rahmen der Substituentendefinition für Formel (I) vorzugsweise angegeben sind. Beispiele für Verbindungen der Formel (I), welche auch bei Verfahren (f) als Ausgangsstoffe verwendet werden können, sind oben im Rahmen der Beschreibung der Ausgangsstoffe für Verfahren (e) aufgeführt.

Bei Verfahren (f) werden starke Säuren als Ausgangsstoffe eingesetzt. Vorzugsweise sind dies Halogenwasserstoffsäuren, wie Hydrogenfluorid, Hydrogenchlorid, Hydrogenbromid und Hydrogeniodid, weiter Schwefelsäure und Phosphorsäure oder gegebenenfalls durch Fluor oder Chlor substituierte Alkansulfonsäuren mit bis zu 4 Kohlenstoffatomen, wie z.B. Methansulfonsäure, Ethansulfonsäure, Chlormethansulfonsäure, 2-Chlorethansulfonsäure und Trifluormethansulfonsäure, ferner Benzolsulfonsäure, p-Toluolsulfonsäure, Naphthalin-1-sulfonsäure, Naphthalin-2-sulfonsäure, Naphthalin-1,4-, -1,5-, -1,6-, -2,6- und -2,7-disulfonsäure. Insbesondere bevorzugt sind Salzsäure (Hydrogenchlorid), Schwefelsäure, Benzolsulfonsäure und p-Toluolsulfonsäure.

Verfahren (a) wird vorzugsweise unter Verwendung von Verdünnungsmitteln durchgeführt. Als solche kommen praktisch alle inerten organischen Solventien in Betracht. Besonders geeignet sind Alkohole, wie z.B. Methanol, Ethanol, n- und iso-Propanol, n-, iso-, sec.- und tert.-Butanol. Ethanol wird als Lösungsmittel besonders bevorzugt.

Als Säureakzeptoren können praktisch alle üblicherweise verwendbaren Säurebindemittel eingesetzt werden. Hierzu gehören insbesondere Alkalimetall- und Erdalkalimetall-hydroxide, Alkalimetall- und Erdalkalimetall-carbonate, (gegebenenfalls (wäßriges) Ammoniak, ferner aliphatische, aromatische oder heterocyclische Amine, wie Triethylamin, N,N-Dimethylanilin, N,N-Dimethylbenzylamin, Pyridin, Diazabicyclooctan und Diazabicycloundecen (DBU).

Die Reaktionstemperatur kann bei Verfahren (a) innerhalb eines größeren Bereiches variiert werden. Im allgemeinen arbeitet man zwischen 0 °C und 150 °C, vorzugsweise zwischen 20 °C und 120 °C. Verfahren (a) wird im allgemeinen bei Normaldruck durchgeführt.

Zur Durchführung des erfindungsgemäßen Verfahrens (a) setzt man je Mol Cyanoverbindung der Formel (II) im allgemeinen zwischen 0,5 und 5 Mol, vorzugsweise zwischen 1 und 3 Mol Aminoverbindung der Formel (III) oder deren Hydrochlorid ein.

Die Ausgangsstoffe der Formeln (II) und (III) und gegebenenfalls die Verdünnungsmittel werden im allgemeinen bei Raumtemperatur oder unter leichter Außenkühlung zusammengegeben und das Reaktionsgemisch wird gegebenenfalls bei erhöhter Temperatur bis zum Reaktionsende gerührt.

Die Aufarbeitung und Isolierung der neuen Verbindungen der Formel (I) erfolgt nach üblichen Methoden: Man verdünnt – gegebenenfalls nach Abkühlen und gegebenenfalls nach Filtration – die Lösung mit Wasser oder man engt im Vakuum ein und löst den Rückstand in Wasser und stellt gegebenenfalls – gegebenenfalls nach Filtration – durch Zugabe eines der oben genannten Säureakzeptoren auf einen schwach alkalischen pH-Wert ein. Die Produkte der Formel (I) fallen hierbei kristallin an und können durch Absaugen isoliert werden.

Das erfindungsgemäße Verfahren (b) zur Herstellung der neuen Verbindungen der Formel (I) wird vorzugsweise unter Verwendung von Verdünnungsmitteln durchgeführt.

Als solche kommen praktisch alle inerten organischen, vorzugsweise jedoch aprotisch polare Solventien in Betracht. Hierzu gehören gegebenenfalls halogenierte Kohlenwasserstoffe, wie z.B. Methylenchlorid, Chloroform, Toluol und Chlorbenzol, Nitrile, wie z.B. Acetonitril und Propionsäurenitril, Dimethylformamid, Dimethylacetamid, Dimethylsulfoxid, Sulfolan, Hexamethylphosphorsäuretriamid, 1,2-Dimethoxyethan, Pyridin und 2-Methyl-5-ethyl-pyridin.

Als Säureakzeptoren können bei Verfahren (b) praktisch alle üblicherweise verwendeten Säurebindemittel eingesetzt werden. Hierzu gehören insbesondere Alkalimetall- und Erdalkalimetallhydroxide, Alkalimetall- und Erdalkalimetallhydride, metallorganische Verbindungen, wie Butyllithium, ferner aliphatische, aromatische oder heterocyclische Amine, wie Trimethylamin, Triethylamin, N,N-Dimethylanilin, N,N-Dimethylbenzylamin, Diazabicyclooctan (DABCO), Diazabicycloundecen (DBU), Pyridin und 2-Methyl-5-ethyl-pyridin.

Die Reaktionstemperaturen können bei Verfahren (b) innerhalb eines größeren Bereiches variiert werden. Im allgemeinen arbeitet man zwischen −80 °C und +100 °C, vorzugsweise zwischen −30 °C und +50 °C. Das erfindungsgemäße Verfahren (b) wird im allgemeinen bei Normaldruck durchgeführt.

Zur Durchführung des erfindungsgemäßen Verfahrens (b) setzt man je Mol Guanidin-Zwischenprodukt der Formel (I) im allgemeinen zwischen 0,5 und 5 Mol, vorzugsweise zwischen 1 und 3 Mol Halogen-Schwefel-Verbindung der Formel (IV) bzw. (V) ein.

Die Reaktionskomponenten werden gewöhnlich bei Raumtemperatur oder unter Außenkühlung zusammengegeben und das Reaktionsgemisch wird bis zum Reaktionsende gerührt.

Die Aufarbeitung und Isolierung der neuen Verbindungen erfolgt nach üblichen Methoden: Gegebenenfalls nach Abdestillieren flüchtiger Komponenten wird mit Wasser und einem mit Wasser nicht mischbaren Lösungsmittel, wie z.B. Methylenchlorid, Chloroform oder Toluol, geschüttelt, die organische Phase mit Wasser gewaschen, getrocknet, filtriert und eingeengt. Die im Rückstand verbleibenden Produkte der Formel (I) werden durch Digerieren mit organischen Lösungsmitteln, wie z.B. Diethylether, Essigester, Ethanol oder Isopropanol zur Kristallisation gebracht und gegebenenfalls durch Umkristallisieren gereinigt.

Das erfindungsgemäße Verfahren (c) zur Herstellung von Verbindungen der Formel (I) wird vorzugsweise unter Verwendung von Verdünnungsmitteln durchgeführt. Als solche kommen praktisch alle inerten organischen Lösungsmittel in Betracht. Hierzu gehören insbesondere gegebenenfalls chlorierte Kohlenwasserstoffe, wie z.B. Chloroform, Tetrachlorkohlenstoff, Toluol, Xylol, Chlorbenzol und 1,2-Dichlorbenzol, Ether, wie z.B. Diisopropyl- und Dibutylether, Tetrahydrofuran, Dioxan, 1,2-Dimethoxyethan und Diglycoldimethylether (Diglyme), Nitrile, wie z.B. Acetonitril und Propionsäurenitril, sowie Dimethylformamid, Dimethylacetamid, Dimethylsulfoxid und Sulfolan.

Als Säureakzeptoren können bei Verfahren (c) Säurebindemittel eingesetzt werden, welche in ihren nucleophilen Eigenschaften nicht nennenswert mit den Aminoverbindungen der Formel (III) konkurrieren. Als solche seien Alkalimetall- und Erdalkalimetall-carbonate, wie z.B. Kaliumcarbonat und Caliumcarbonat, tertiäre Amine, wie z.B. Triethylamin, N,N-Dimethylanilin und N,N-Dimethylbenzylamin, sowie Stickstoffheterocyclen, wie z.B. Pyridin, Diazabicyclooctan (DABCO) und Diazabicycloundecen (DBU) genannt.

Die Reaktionstemperatur kann bei Verfahren (c) innerhalb eines größeren Bereiches variiert werden. Im allgemeinen arbeitet man zwischen 0 °C und 200 °C, vorzugsweise zwischen 20 °C und 120 °C. Verfahren (c) wird im allgemeinen bei Normaldruck durchgeführt.

Zur Durchführung des erfindungsgemäßen Verfahrens (c) setzt man je Mol Isothioharnstoff der Formel (VI) im allgemeinen zwischen 1 und 5 Mol, vorzugsweise zwischen 1 und 3 Mol Aminoverbindung der Formel (III) oder deren Hydrochlorid ein.

Im allgemeinen werden die Isothioharnstoffe der Formel (VI) und das Verdünnungsmittel bei Raumtemperatur vorgelegt und die Aminoverbindungen der Formel (III) bzw. deren Hydrochloride und geeignete Säureakzeptoren zudosiert. Das Reaktionsgemisch wird dann im allgemeinen bei erhöhter Temperatur bis zum Reaktionsende gerührt. Die Produkte der Formel (I) fallen gewöhnlich beim Abkühlen in kristalliner Form an und können durch Absaugen isoliert werden. Soweit die Produkte der Formel (I) als Ammoniumsalze anfallen, können daraus nach Auflösen in Wasser durch Ansäuern, z.B. mit Salzsäure oder Schwefelsäure die korrespondierenden Säuren (M=H) hergestellt werden.

Das erfindungsgemäße Verfahren (d) wird vorzugsweise unter Verwendung von Verdünnungsmitteln durchgeführt. Als solche kommen praktisch alle inerten organischen Lösungsmittel und gegebenenfalls zusätzlich auch Wasser in Betracht. Hierzu gehören insbesondere Alkohole, wie Methanol, Ethanol, n- und iso-Propanol, Ether, wie Tetrahydrofuran, Dioxan und 1,2-Dimethoxyethan, Ester, wie Essigsäuremethylester und -ethylester, Nitrile, wie z.B. Acetonitril oder Propionsäurenitril, sowie Dimethylformamid und Wasser.

Als Säureakzeptoren können bei Verfahren (d) Säurebindemittel eingesetzt werden, welche in ihren nucleophilen Eigenschaften nicht nennenswert mit den Aminoverbindungen der Formel (III) konkurrieren.

Als solche seien Alkalimetall- und Erdalkalimetallcarbonate, wie z.B. Kaliumcarbonat und Calciumcarbonat, tertiäre Amine, wie z.B. Triethylamin, N,N-Dimethylanilin und N,N-Dimethylbenzylamin, sowie Stickstoffheterocyclen, wie z.B. Pyridin, Diazabicyclooctan (DABCO) und Diazabicycloundecen (DBU) genannt.

Die Reaktionstemperatur kann bei Verfahren (d) innerhalb eines größeren Bereiches variiert werden. Im allgemeinen arbeitet man zwischen 0 °C und 150 °C, vorzugsweise zwischen 10 °C und 100 °C. Verfahren (d) wird im allgemeinen bei Normaldruck durchgeführt.

Zur Durchführung des erfindungsgemäßen Verfahrens (d) setzt man je Mol Guanidin-Zwischenprodukt der Formel (I) im allgemeinen zwischen 1 und 10 Mol, vorzugsweise zwischen 2 und 5 Mol Aminoverbindung der Formel (III) oder deren Hydrochlorid ein.

Im allgemeinen werden die Guanidin-Derivate der Formel (I) und das Verdünnungsmittel bei Raumtemperatur oder unter leichtem Kühlen vorgelegt und die Aminoverbindung der Formel (III) bzw. deren Hydrochlorid und geeignete Säureakzeptoren zudosiert. Das Reaktionsgemisch wird dann im allgemeinen bei Raumtemperatur oder erhöhter Temperatur bis zum Reaktionsende gerührt.

Die Aufarbeitung kann nach üblichen Methoden durchgeführt werden. Soweit die Produkte der Formel (I) aus dem Reaktionsgemisch kristallin anfallen, können sie durch Absaugen isoliert werden. Anderenfalls wird – gegebenenfalls nach Einengen – mit Wasser verdünnt und mit einem mit Wasser praktisch nicht mischbaren Lösungsmittel, wie z.B. Methylenchlorid, extrahiert. Durch Waschen der Extraktionslösung mit Wasser, Trocknen, Filtrieren, Einengen des Filtrats und Umkristallisieren des Rückstandes können die Produkte der Formel (I) in reiner Form erhalten werden.

Das erfindungsgemäße Verfahren (e) wird vorzugsweise unter Verwendung von Verdünnungsmitteln durchgeführt. Als solche kommen praktisch alle inerten organischen Lösungsmittel in Betracht. Hierzu gehören insbesondere Alkohole, wie z.B. Ethanol, n- und iso-Propanol, Ether, wie z.B. Tetrahydrofuran, Dioxan und 1,2-Dimethoxyethan, Ester, wie z.B. Essigsäuremethylester und -ethylester sowie Nitrile, wie z.B. Acetonitril.

Die Reaktionstemperatur kann bei Verfahren (e) innerhalb eines größeren Bereiches variiert werden. Im allgemeinen arbeitet man zwischen −20 °C und +50 °C, vorzugsweise zwischen 0 °C und 30 °C. Verfahren (e) wird im allgemeinen bei Normaldruck durchgeführt.

Zur Durchführung des erfindungsgemäßen Verfahrens (e) setzt man je Mol Guanidin-Derivat der Formel (I) im allgemeinen zwischen 0,9 und 1,2 Mol, vorzugsweise zwischen 0,95 und 1,1 Mol Metallverbindung bzw. Amin ein.

Im allgemeinen werden die Guanidin-Derivate der Formel (I) und das Verdünnungsmittel vorgelegt und – gegebenenfalls unter leichter Außenkühlung – die Metallverbindung bzw. das Amin – gegebenenfalls im Verdünnungsmittel gelöst – zudosiert. Das Reaktionsgemisch wird bis zum Reaktionsende gerührt. Die salzartigen Produkte der Formel (I) fallen im allgemeinen kristallin an und können durch Absaugen isoliert werden.

Das erfindungsgemäße Verfahren (f) wird vorzugsweise unter Verwendung von Verdünnungsmitteln durchgeführt. Als solche kommen praktisch alle inerten organischen Lösungsmittel in Betracht. Hierzu gehören insbesondere Alkohole, wie Methanol, Ethanol, n- und iso-Propanol, Ether, wie Tetrahydrofuran, Dioxan und 1,2-Dimethoxyethan, sowie Ester, wie Essigsäuremethylester und -ethylester.

Soweit die als Ausgangsstoffe verwendeten Säuren in wäßriger Lösung eingesetzt werden, kann vorteilhaft auch Essigsäureanhydrid als Verdünnungsmittel verwendet werden.

Die Reaktionstemperatur kann bei Verfahren (f) innerhalb eines größeren Bereiches variiert werden. Im allgemeinen arbeitet man zwischen −20 °C und +50 °C, vorzugsweise zwischen 0 °C und 30 °C. Verfahren (f) wird im allgemeinen bei Normaldruck durchgeführt.

Zur Durchführung des erfindungsgemäßen Verfahrens (f) setzt man je Mol Guanidin-Derivat der Formel (I) im allgemeinen zwischen 1 und 10 Mol, vorzugsweise zwischen 1,5 und 5 Mol einer starken Säure ein.

Im allgemeinen werden die Guanidin-Derivate der Formel (I) und das Verdünnungsmittel vorgelegt und – gegebenenfalls unter leichter Außenkühlung – die starke Säure zudosiert. Das Reaktionsgemisch wird bis zum Reaktionsende gerührt. Die 1 : 1-Addukte fallen im allgemeinen kristallin an und können durch Absaugen isoliert werden.

Die erfindungsgemäßen Wirkstoffe beeinflussen das Pflanzenwachstum und können deshalb als Defoliants, Desiccants, Krautabtötungsmittel und insbesondere als Unkrautvernichtungsmittel, aber auch als Pflanzenwachstumsregulatoren verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide oder als Pflanzenwachstumsregulatoren wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe greifen auch in den Metabolismus der Pflanzen ein und können deshalb wie bereits angegeben, unter bestimmten Voraussetzungen als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- oder Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens («Lagerns») der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So

ist es beispielsweise möglich, den Gehalt an Zukker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z. B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z. B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle, ist aber auch in anderen Kulturen wie z. B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden («Ausdünnung»), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z. B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid. Als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Für die Mischungen kommen bekannte Herbizide wie z.B.
1-Amino-6-ethylthio-3-(2,2-dimethylpropyl)-1,3,5-triazin-2,4(1H,3H)-dion oder
N-(2-Benzthiazolyl)-N,N'-dimethyl-harnstoff zur Unkrautbekämpfung in Getreide;
4-Amino-3-methyl-6-phenyl-1,2,4-triazin-5(4H)-on zur Unkrautbekämpfung in Zuckerrüben und
4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effektes ab. Im allgemeinen liegen die Aufwandmengen zwischen 0,001 und 10 kg Wirkstoff pro ha, vorzugsweise zwischen 0,01 und 5 kg/ha.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Die nachfolgenden Beispiele dienen zur weiteren Erläuterung der Erfindung.

Herstellungsbeispiele:
Beispiel 1:

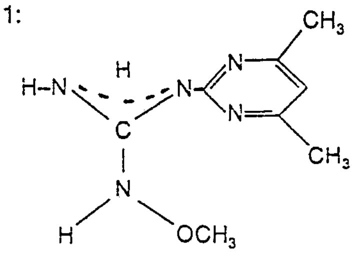

(Verfahren a)

Eine Mischung aus 109 g (0,67 Mol) O-Methylhydroxylamin-Hydrochlorid, 99 g (0,67 Mol) 2-Cyanamino-4,6-dimethyl-pyrimidin und 600 ml Ethanol wird 7 Stunden unter Rückfluß zum Sieden erhitzt. Anschließend wird der Alkohol im Wasserstrahlvakuum abdestilliert, der Rückstand in heißem Wasser gelöst und diese Lösung zu 100 ml konzentriertem Ammoniak gegeben. Das auskristallisierte Produkt wird abgesaugt und aus Ethanol umkristallisiert.

Man erhält 71,8 g (55% der Theorie) N'-(4,6-Dimethyl-pyrimidin-2-yl)-N''-methoxy-guanidin vom Schmelzpunkt 134 °C bis 136 °C.

Beispiel 2:

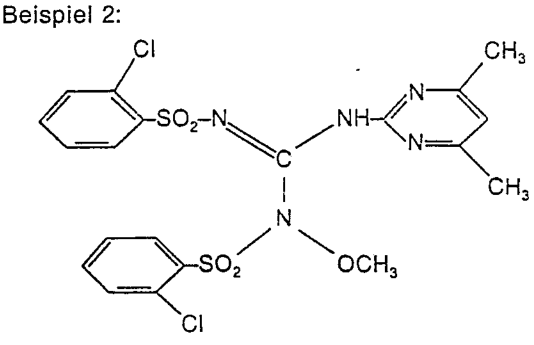

(Verfahren b)

Eine Mischung aus 29,4 g (0,15 Mol) N'-(4,6-Dimethyl-pyrimidin-2-yl)-N''-methoxy-guanidin, 63,6 g (0,3 Mol) 2-Chlor-benzolsulfonsäurechlorid und 150 ml Pyridin wird 2 Tage bei 20 °C gerührt. Nach weitgehendem Abdestillieren des Pyridins im Wasserstrahlvakuum wird der Rückstand mit 200 ml Wasser versetzt und mit 200 ml Methylenchlorid extrahiert. Die organische Phase wird abgetrennt, getrocknet und eingeengt.

Der Rückstand wird durch Digerieren mit Ethanol zur Kristallisation gebracht.

Man erhält 41,2 g (51% der Theorie) N'-(4,6-Dimethyl-pyrimidin-2-yl)-N''-methoxy-N'',N'''-bis-(2-chlor-benzol-sulfonyl)-guanidin vom Schmelzpunkt 164 °C bis 166 °C.

Die obige Strukturformel gilt für den kristallinen Zustand und wird durch Röntgen-Struktur-Analyse bewiesen. Weitere spektroskopische Daten (IR, $^{1}$H- und $^{13}$C-NMR) sowie die Elementaranalyse sind in Einklang mit dieser Strukturzuordnung.

Beispiel 3:

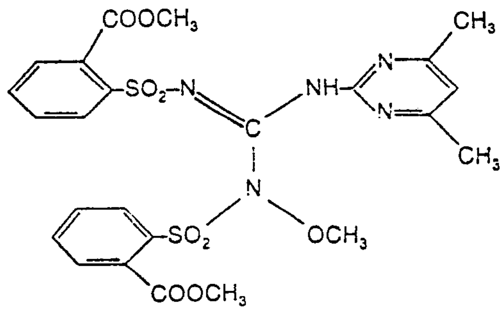

(Verfahren b)

113 g (0,48 Mol) 2-Methoxycarbonyl-benzolsulfonsäure-chlorid werden zu einer auf −10 °C gekühlten Mischung aus 35,1 g (0,18 Mol) N'-(4,6-Dimethyl-pyrimidin-2-yl)-N''-methoxy-guanidin und 160 ml Pyridin gegeben und das Reaktionsgemisch bei 20 °C zwei Tage gerührt. Nach weitgehendem Abdestillieren des Pyridins im Wasserstrahlvakuum wird der Rückstand mit 200 ml Wasser versetzt und mit 200 ml Methylenchlorid extrahiert. Die organische Phase wird abgetrennt, mit Natriumsulfat getrocknet, filtriert und eingeengt. Der Rückstand wird durch Digerieren mit Isopropanol zur Kristallisation gebracht.

Man erhält 59 g (55% der Theorie) N'-(4,6-Dimethyl-pyrimidin-2-yl)-N''-methoxy-N'',N'''-bis-(2-methoxycarbonyl-benzolsulfonyl)-guanidin vom Schmelzpunkt 165 °C.

Die obige Strukturformel gilt für den kristallinen Zustand und wurde durch Röntgen-Struktur-Analyse bewiesen. Weitere spektroskopische Daten (IR, $^{1}$H- und $^{13}$C-NMR) sowie die Elementaranalyse sind im Einklang mit dieser Strukturzuordnung.

Beispiel 4:

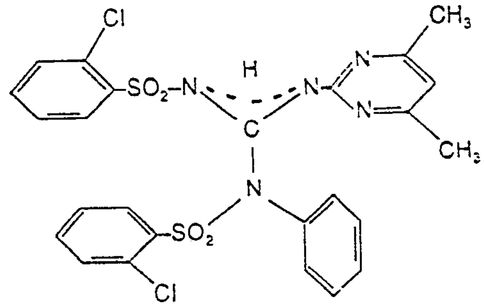

(Verfahren b)

21,2 g (0,1 Mol) 2-Chlor-benzolsulfonsäurechlorid werden bei 20 °C zu einer Mischung aus 12,1 g (0,05 Mol) N'-(4,6-Dimethyl-pyrimidin-2-yl)-N''-phenyl-guanidin, 10,5 g (0,1 Mol) Triethylamin und 100 ml Chloroform tropfenweise gegeben und das Reaktionsgemisch wird 15 Stunden bei 20 °C gerührt. Anschließend wird das Reaktionsgemisch mit Wasser geschüttelt, die organische Phase abgetrennt und eingeengt. Der Rückstand wird in Ethanol suspendiert, die Suspension filtriert, das Filtrat eingeengt und der Rückstand durch Digerieren mit Essigsäureethylester zur Kristallisation gebracht.

Man erhält 6,6 g (22% der Theorie) N'-(4,6-Dimethyl-pyrimidin-2-yl)-N''-phenyl-N'',N'''-bis-(2-chlor-benzolsulfonyl)-guanidin vom Schmelzpunkt 120 °C.

Beispiel 5:

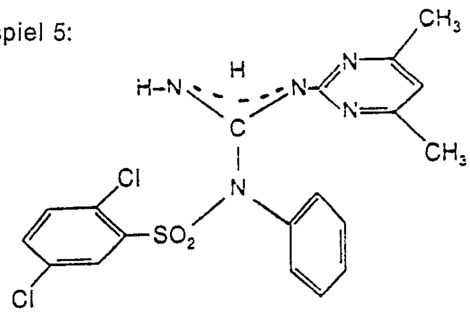

(Verfahren b)

12,2 g (0,05 Mol) 2,5-Dichlor-benzolsulfonsäurechlorid werden bei 0 °C bis 10 °C zu einer Mischung aus 12,1 g (0,05 Mol) N'-(4,6-Dimethyl-pyrimidin-2-yl)-N''-phenyl-guanidin, 5,1 g (0,05 Mol) Triethylamin und 150 ml Chloroform gegeben, und das Reaktionsgemisch wird 15 Stunden bei 20 °C gerührt. Dann wird das Reaktionsgemisch mit 100 ml 5%iger Salzsäure gewaschen, eingeengt, der Rückstand mit Ethanol digeriert und abgesaugt.

Man erhält 3,5 g (16% der Theorie) N'-(4,6-Dimethyl-pyrimidin-2-yl)-N''-phenyl-N'''-(2,5-dichlor-benzolsulfonyl)-guanidin vom Schmelzpunkt 188 °C.

Das aus der Mutterlauge allmählich auskristallisierende Produkt wurde nach einigen Tagen durch Absaugen isoliert. Man erhielt 1,8 g (8% der Theorie) N'-(4,6-Dimethyl-pyrimidin-2-yl)-N''-phenyl-N''-(2,5-dichlor-benzolsulfonyl)-guanidin (5) vom Schmelzpunkt 153 °C.

Beispiel 6:

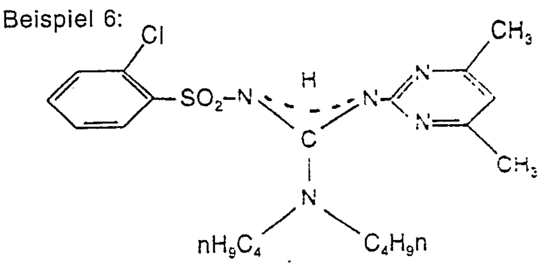

(Verfahren b)

5,3 g (0,025 Mol) 2-Chlor-benzolsulfonsäurechlorid werden bei 20 °C zu einer Mischung aus 7,3 g (0,025 Mol) N'-(4,6-Dimethyl-pyrimidin-2-yl)-N'',N'''-dibutyl-guanidin und 5,3 g (0,05 Mol) Triethylamin getropft. Das Reaktionsgemisch wird 15 Stunden bei 20 °C gerührt, dann mit 100 ml 5%iger Salzsäure gewaschen und eingeengt. Der Rückstand wird durch Digerieren mit Ethanol zur Kristallisation gebracht.

Man erhält 1,8 g (16% der Theorie) N'-(4,6-Dimethyl-pyrimidin-2-yl)-N'',N'''-dibutyl-N'''-(2-chlor-benzolsulfonyl)-guanidin vom Schmelzpunkt 150 °C.

Beispiel 7a:

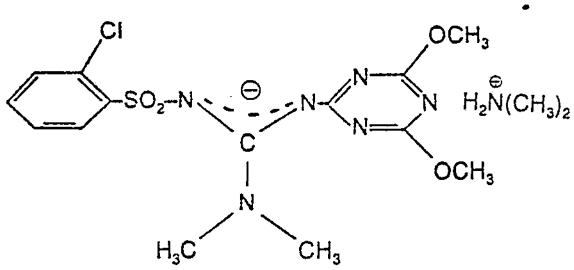

und

Beispiel 7b:

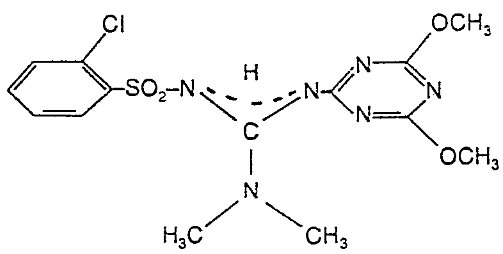

(Verfahren c)

4,5 g (0,1 Mol) Dimethylamin werden bei 25 °C bis 35 °C in ein Gemisch aus 15 g (0,037 Mol) N'-(4,6-Dimethoxy-s-triazin-2-yl)-N''-(2-chlor-benzolsulfonyl)-S-methyl-isothioharnstoff und 100 ml Dioxan eingeleitet. Das Reaktionsgemisch wird dann eine Stunde bei 80 °C gerührt. Nach dem Abkühlen wurde das kristallin angefallene Produkt durch Absaugen isoliert.

Man erhält 12,1 g (73% der Theorie) N'-(4,6-Dimethoxy-s-triazin-2-yl)-N'',N'''-dimethyl-N'''-(2-chlor-benzolsulfonyl)-guanidin-dimethyl-ammoniumsalz (7a) vom Schmelzpunkt 162 °C.

Das Ammoniumsalz (7a) wird in 20 ml Wasser gelöst und mit konzentrierter Salzsäure angesäuert. Das hierbei kristallin angefallene Produkt wird durch Absaugen isoliert.

Man erhält 4 g (28% der Theorie) N'-(4,6-Dimethoxy-s-triazin-2-yl)-N'',N'''-dimethyl-N'''-(2-chlor-benzolsulfonyl)-guanidin (7b) vom Schmelzpunkt 185 °C.

Beispiel 8:

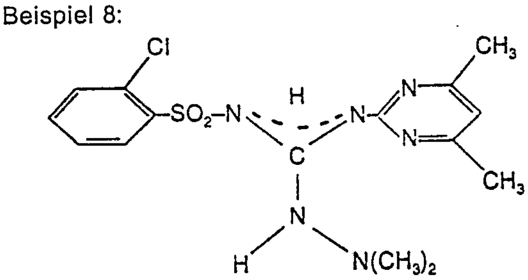

(Verfahren d)

Eine Mischung aus 5,5 g (0,01 Mol) N'-(4,6-Dimethyl-pyrimidin-2-yl)-N''-methoxy-N'',N'''-bis-(2-chlor-benzolsulfonyl)-guanidin (2), 1,5 g (0,025 Mol) N,N-Dimethylhydrazin, 20 ml Ethanol und 10 ml Wasser werden 15 Minuten unter Rückfluß zum Sieden erhitzt. Nach dem Erkalten wird das kristallin angefallene Produkt durch Absaugen isoliert.

Man erhält 2,5 g (65% der Theorie) N'-(4,6-Dimethyl-pyrimidin-2-yl)-N''-dimethylamino-N'''-(2-chlor-benzolsulfonyl)-guanidin vom Schmelzpunkt 176 °C.

Beispiel 9:

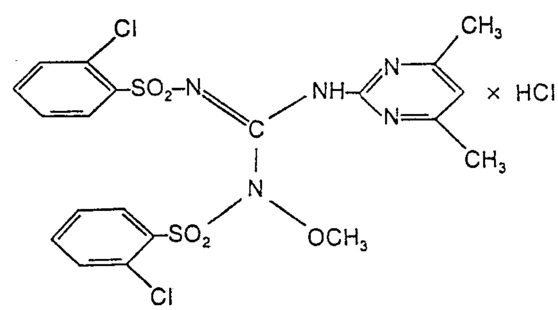

(Verfahren f)

5 ml konzentrierte Salzsäure (0,05 Mol) werden bei 20 °C zu einer Mischung aus 5,5 g (0,01 Mol) N'-(4,6-Dimethyl-pyrimidin-2-yl)-N''-methoxy-N'',N'''-bis-(2-chlor-benzolsulfonyl)-guanidin und 25 ml Acetanhydrid gegeben (exotherme Reaktion!) und das Reaktionsgemisch wird 2 Stunden gerührt. Das kristallin angefallene Produkt wird durch Absaugen isoliert.

Man erhält 4,3 g (74% der Theorie) N'-(4,6-Dimethyl-pyrimidin-2-yl)-N''-methoxy-N'',N'''-bis-(2-chlor-benzolsulfonyl)-guanidin-Hydrochlorid vom Schmelzpunkt 142 °C.

Nach den in den vorausgehenden Beispielen exemplarisch beschriebenen Verfahren konnten die in der nachstehenden Tabelle aufgeführten Verbindungen der Formel (I) hergestellt werden.

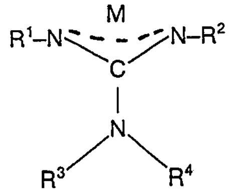

(I)

Tabelle 1:

| Beisp. Nr. | $R^1$ | $R^2$ | $R^3$ | $R^4$ | M | Schmelzpunkt (°C) |
|---|---|---|---|---|---|---|
| 10 | H | 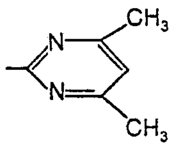 | H | $-OC_2H_5$ | H | 66 |
| 11 | H | 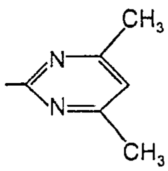 | H | $-OCH_2-$ 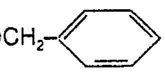 | H | 77 |
| 12 | H | 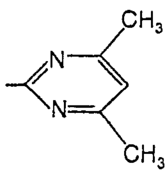 | $-C_4H_9n$ | $-C_4H_9n$ | H | 88 |
| 13 | H | 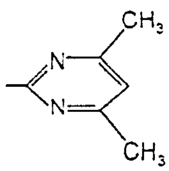 | H | $-N(CH_3)_2$ | H | 149 |
| 14 | H | 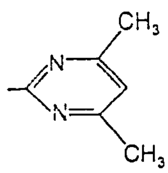 | $CH_3$ | 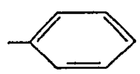 | H | 197 |
| 15 | H | 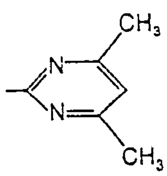 | 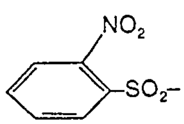 | 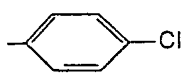 | H | 103 |
| 16 | H | 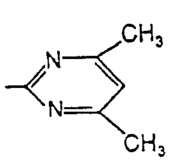 | 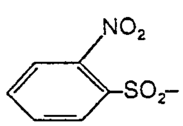 | $-CH_2-$ 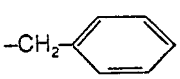 | H | 168 |
| 17 | 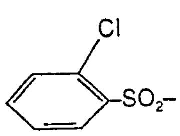 | 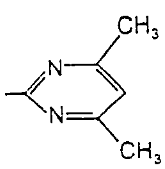 | $-C_4H_9n$ | $-C_4H_9n$ | H | 150 |
| 18 | 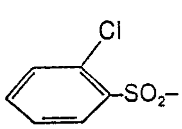 | 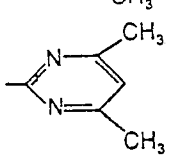 | $-CH_3$ | 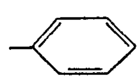 | H | 200 |

Tabelle 1 – Fortsetzung:

| Beisp. Nr. | $R^1$ | $R^2$ | $R^3$ | $R^4$ | M | Schmelzpunkt (°C) |
|---|---|---|---|---|---|---|
| 19 | 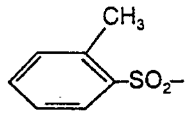 | 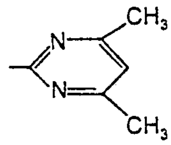 | $-CH_3$ | 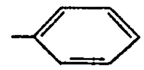 | H | 171 |
| 20 | H | 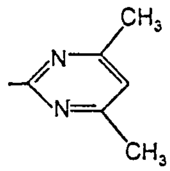 | 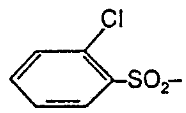 | 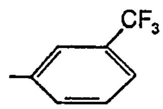 | H | 221 |
| 21 | H | 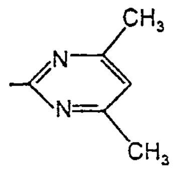 | 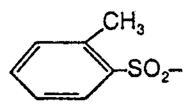 | $-OCH_3$ | H | 91 |
| 22 | 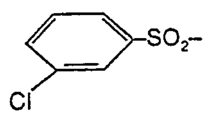 | 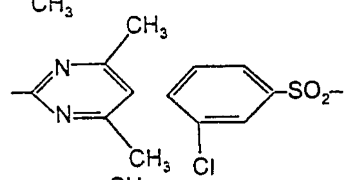 | | $-OCH_3$ | H | 154 |
| 23 | 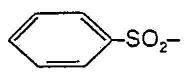 | 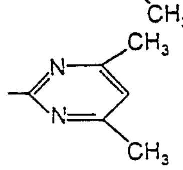 | 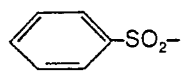 | $-OCH_3$ | H | 149 |
| 24 | 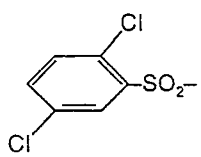 | 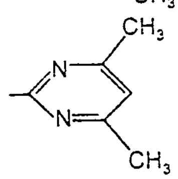 | 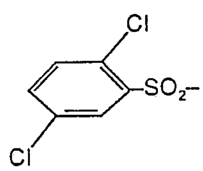 | $-OCH_3$ | H | 142 |
| 25 | 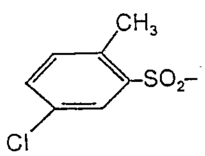 | 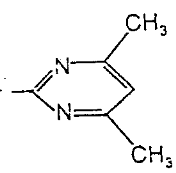 | 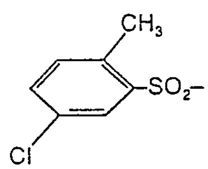 | $-OCH_3$ | H | 135 |
| 26 | 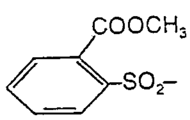 | 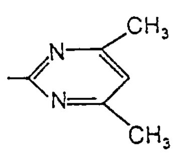 | 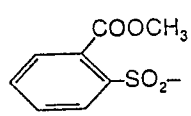 | $-OC_2H_5$ | H | 129 |
| 27 | 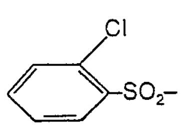 | 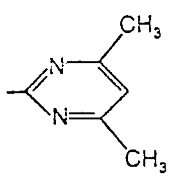 | 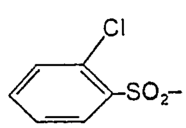 | $-OC_2H_5$ | H | 170 |
| 28 | 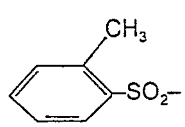 | 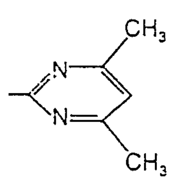 | 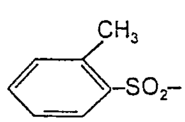 | $-OCH_3$ | H | 129 |

Tabelle 1 – Fortsetzung:

| Beisp. Nr. | $R^1$ | $R^2$ | $R^3$ | $R^4$ | M | Schmelzpunkt (°C) |
|---|---|---|---|---|---|---|
| 29 | 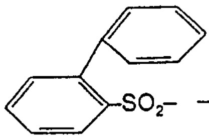 | 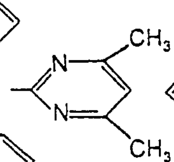 | 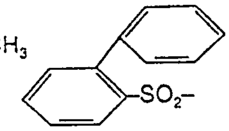 | $-OCH_3$ | H | 171 |
| 30 | 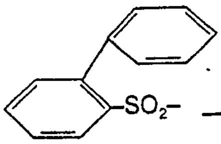 | 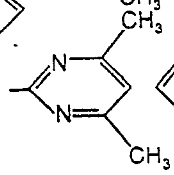 | 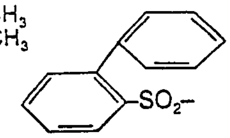 | $-OC_2H_5$ | H | 174 |
| 31 | 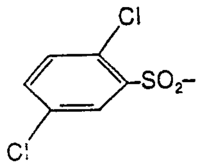 | 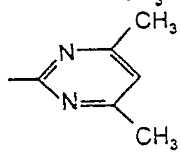 | 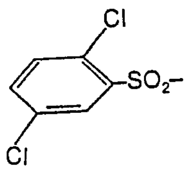 | $-OC_2H_5$ | H | 158 |
| 32 | 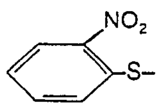 | 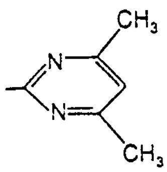 | 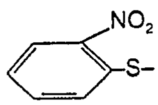 | $-OCH_3$ | H | 150 |
| 33 | 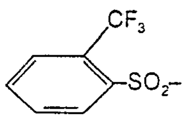 | 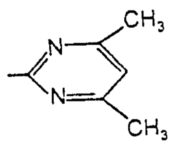 | 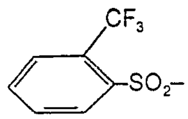 | $-OCH_3$ | H | 121 |
| 34 | 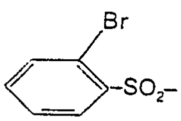 | 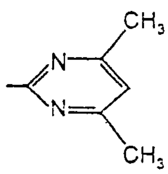 | 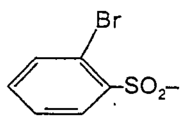 | $-OCH_3$ | H | 147 |
| 35 | 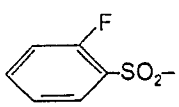 | 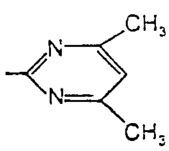 | 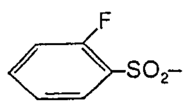 | $-OCH_3$ | H | 166 |
| 36 | 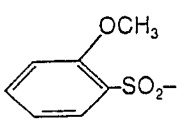 | 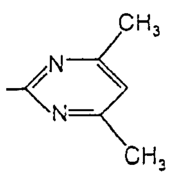 | 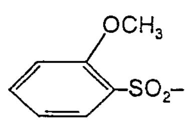 | $-OCH_3$ | H | 196 |
| 37 | 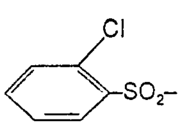 | 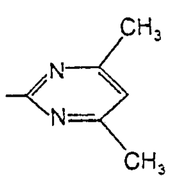 | 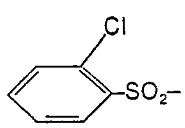 | 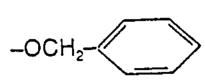 | H | 175 |
| 38 | 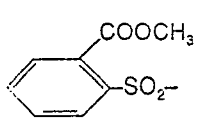 | 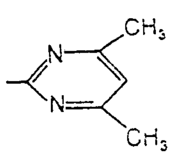 | 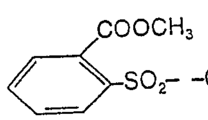 | 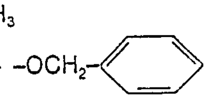 | H | 85 |

Tabelle 1 – Fortsetzung:

| Beisp. Nr. | $R^1$ | $R^2$ | $R^3$ | $R^4$ | M | Schmelzpunkt (°C) |
|---|---|---|---|---|---|---|
| 39 | 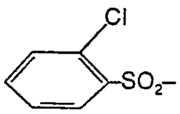 | 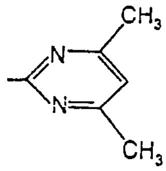 | $-CH_3$ | $-CH_3$ | H | 160 |
| 40 | 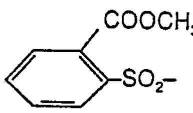 | 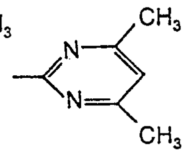 | $-CH(CH_3)_2$ | $-CH(CH_3)_2$ | H | 189 |
| 41 | 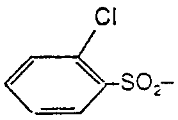 | 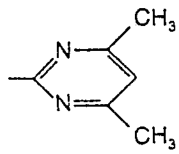 | $-CH(CH_3)_2$ | $-CH(CH_3)_2$ | H | 88 |
| 42 | 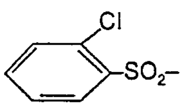 | 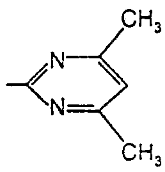 | 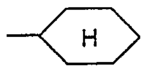 | 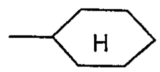 | H | 122 |
| 43 | 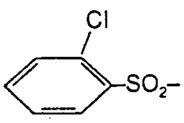 | 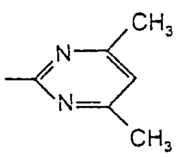 | H | $-OCH_3$ | H | 142 |
| 44 | 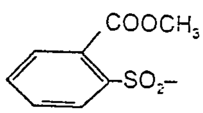 | 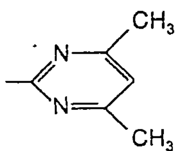 | H | $-N(CH_3)_2$ | H | 150 |
| 45 | 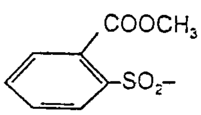 | 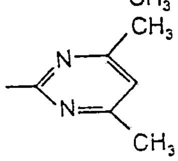 | H | $-OCH_3$ | H | 132 |
| 46 | 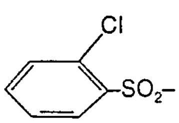 | 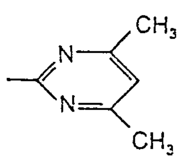 | $-C_2H_2$ | $-C_2H_5$ | H | 134 |
| 47 | 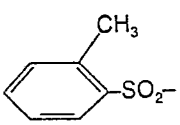 | 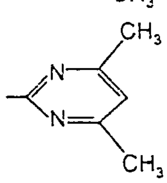 | $-C_2H_5$ | $-C_2H_5$ | H | 145 |
| 48 | 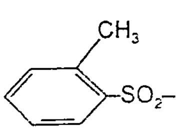 | 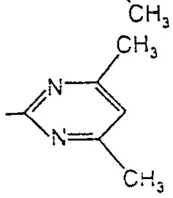 | H | $-N(CH_3)_2$ | H | 128 |

Tabelle 1 – Fortsetzung:

| Beisp. Nr. | $R^1$ | $R^2$ | $R^3$ | $R^4$ | M | Schmelzpunkt (°C) |
|---|---|---|---|---|---|---|
| 49 | 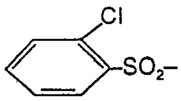 | 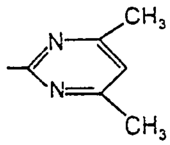 | H | $-NHCOCH_3$ | H | 185 |
| 50 | 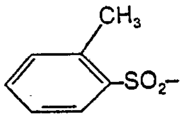 | 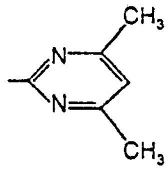 | H | $-NHCOCH_3$ | H | 143 |
| 51 | 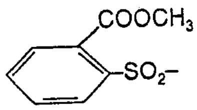 | 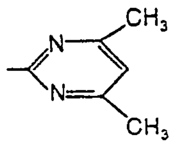 | H | $-NHCOCH_3$ | H | 150 |
| 52 | 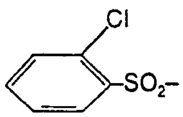 | 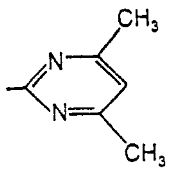 | $-CH_2CH_2-O-CH_2CH_2-$ | | H | 192 |
| 53 | 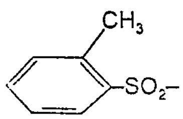 | 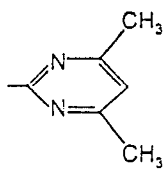 | H | $-OCH_3$ | H | 114 |
| 54 | 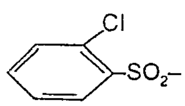 | 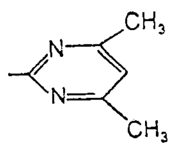 | H | $-NHCOOCH_3$ | H | 187 |
| 55 | 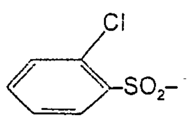 | 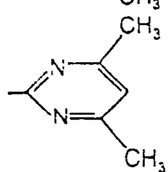 | H | 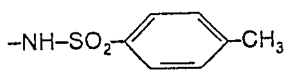 | H | 203 |
| 56 | 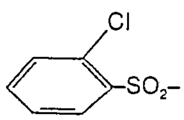 | 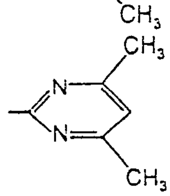 | H | 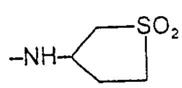 | H | 153 |
| 57 | 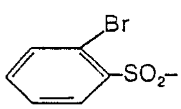 | 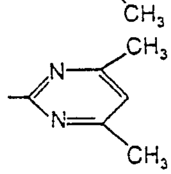 | H | $-OCH_3$ | H | 166 |
| 58 | 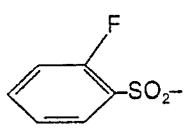 | 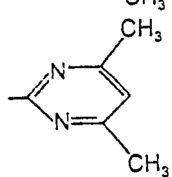 | H | $-OCH_3$ | H | 160 |
| 59 | 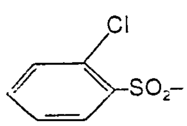 | 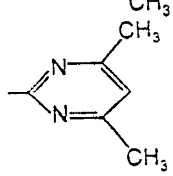 | H | 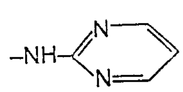 | H | 210 |

Tabelle 1 – Fortsetzung:

| Beisp. Nr. | $R^1$ | $R^2$ | $R^3$ | $R^4$ | M | Schmelzpunkt (°C) |
|---|---|---|---|---|---|---|
| 60 | 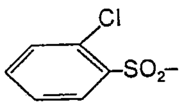 | 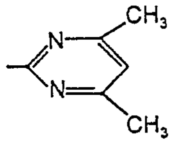 | H | 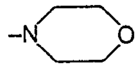 | H | 188 |
| 61 | 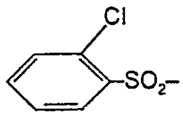 | 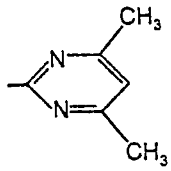 | 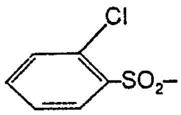 | $-OCH_3$ | Na | 182 |
| 62 | 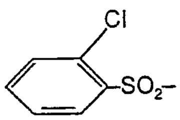 | 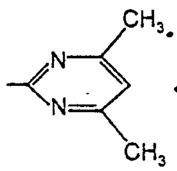 | 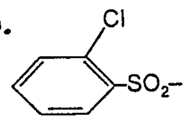 | $-OCH_3$ | K | 165 |
| 63 | 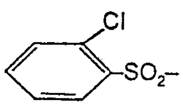 | 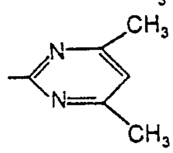 | 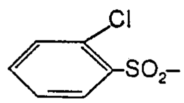 | $-OCH_3$ | 1/2 Ca | 165 |
| 64 | 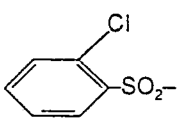 | 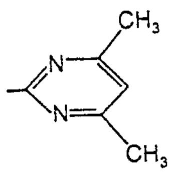 | $-CH_3$ | $-OH$ | H | 161 |
| 65 | H | 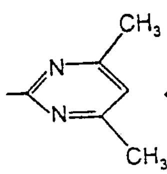 | 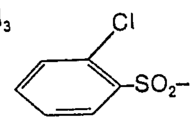 | $-OCH_3$ | H | 90 |
| 66 | 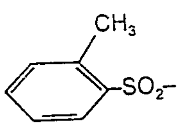 | 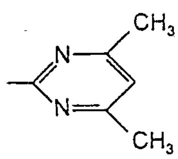 | H | $-N(CH_3)_2$ | H | 140 |
| 67 | 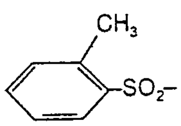 | 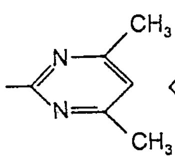 | 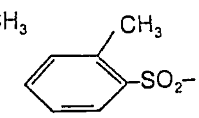 | $-OC_2H_5$ | H | 139 |
| 68 | 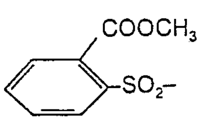 | 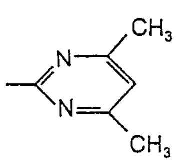 | H | 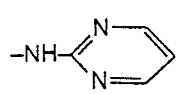 | H | 205 |
| 69 | 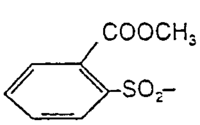 | 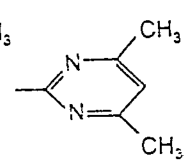 | H | $-NHCOOCH_3$ | H | 142 |

Tabelle 1 – Fortsetzung:

| Beisp. Nr. | $R^1$ | $R^2$ | $R^3$ | $R^4$ | M | Schmelzpunkt (°C) |
|---|---|---|---|---|---|---|
| 70 | 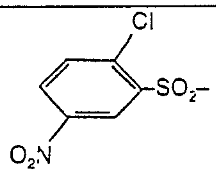 | 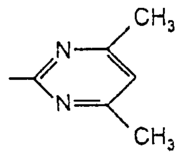 | H | $-OCH_3$ | H | 163 |
| 71 | 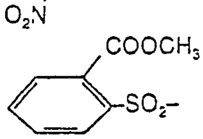 | 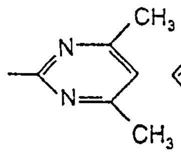 | 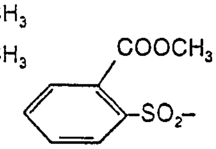 | $-OCH_3$ | K | 170 |
| 72 | 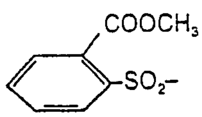 | 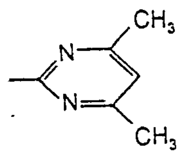 | $CH_3$ | $CH_3$ | H | 136 |
| 73 | 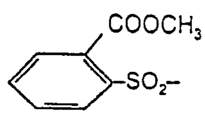 | 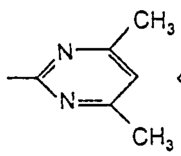 | 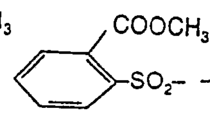 | $-OCH_2CH_2CH_3$ | H | 110 |
| 74 | 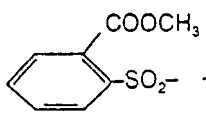 | 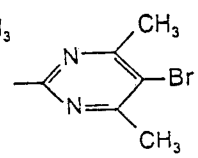 | 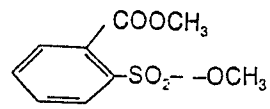 | $-OCH_3$ | H | 112 |
| 75 | 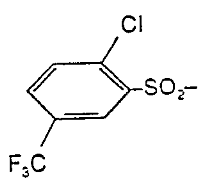 | 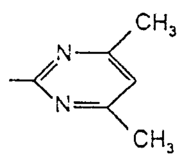 | H | $-OCH_3$ | H | 135 |
| 76 | 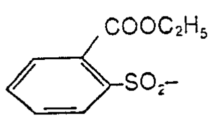 | 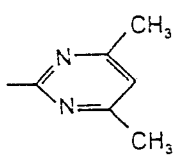 | H | $-OCH_3$ | H | 126 |
| 77 | 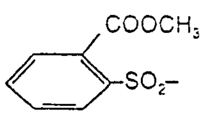 | 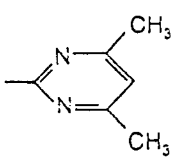 | 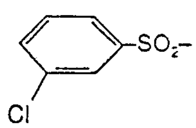 | $-OCH_3$ | H | 159 |
| 78 | 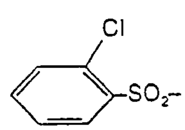 | 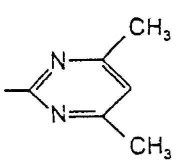 | H | 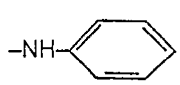 | H | 118 |
| 79 | 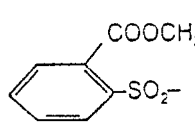 | 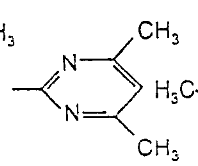 | 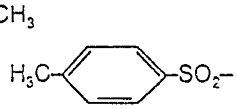 | $-OCH_3$ | H | 150 |

Tabelle 1 – Fortsetzung:

| Beisp. Nr. | $R^1$ | $R^2$ | $R^3$ | $R^4$ | M | Schmelzpunkt (°C) |
|---|---|---|---|---|---|---|
| 80 | 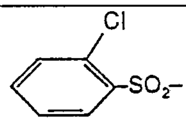 | 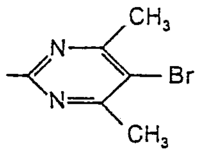 | 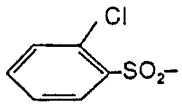 | $-OCH_3$ | H | 157 |
| 81 | 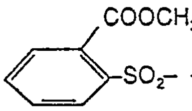 | 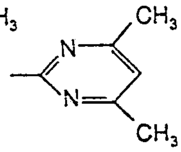 | 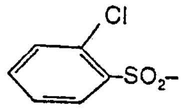 | $-OCH_3$ | H | 150 |
| 82 | 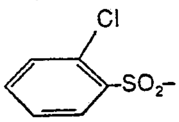 | 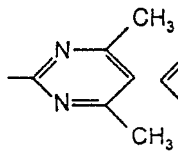 | 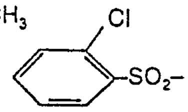 | 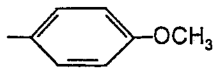 | H | 95 |
| 83 | 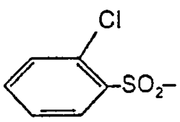 | 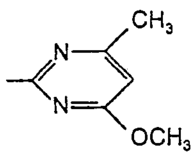 | H | $-OCH_3$ | H | 138 |
| 84 | 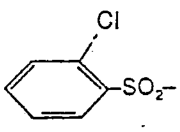 | 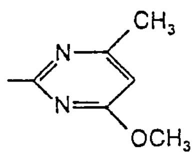 | H | $-N(CH_3)_2$ | H | 169 |
| 85 | 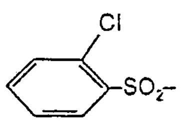 | 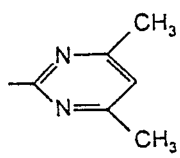 | $CH_3$ | $CH_3$ | H | 170 |
| 86 | 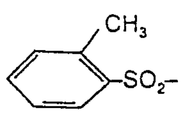 | 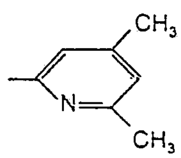 | $CH_3$ | $CH_3$ | H | 88 |
| 87 | 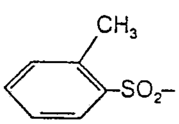 | 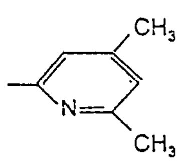 | H | $-OCH_3$ | H | 91 |
| 88 | 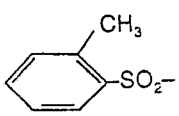 | 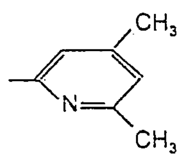 | H | $-N(CH_3)_2$ | H | 134 |

Nachstehend wird die Herstellung einiger weiterer Verbindungen nach dem erfindungsgemäßen Verfahren (b) exemplarisch beschrieben:

Herstellung der vorausgehend als Beispiel (65) aufgeführten Verbindung

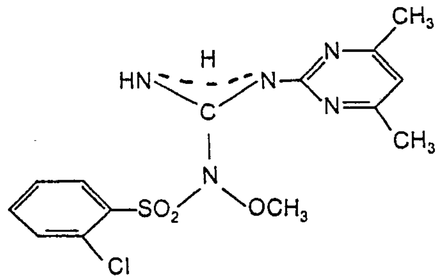

(65)

10,6 g (0,05 Mol) 2-Chlor-benzolsulfonsäurechlorid werden zu einer auf −7 °C gekühlten Mischung aus 4,9 g (0,025 Mol) N′-(4,6-Dimethyl-pyrimidin-2-yl)-N″-methoxy-guanidin und 50 ml Pyridin tropfenweise gegeben.

Nach 10 Minuten Rühren bei −7 °C wird das Reaktionsgemisch mit 400 ml Wasser versetzt. Das kristallin angefallene Produkt wird durch Absaugen isoliert.

Man erhält 5,2 g (56% der Theorie) N′-(4,6-Dimethyl-pyrimidin-2-yl)-N″-(2-chlor-benzolsulfonyl)-N″-methoxy-guanidin vom Schmelzpunkt 90 °C.

Herstellung der vorausgehend als Beispiel (4) aufgeführten Verbindung

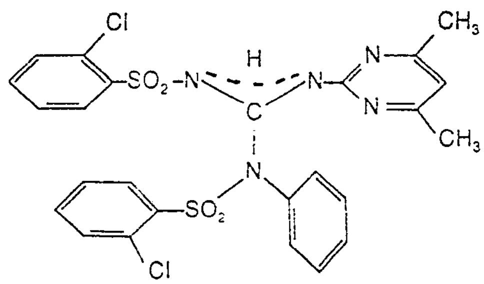

(4)

10,6 g (0,05 Mol) 2-Chlor-benzolsulfonsäurechlorid werden bei 30 °C zu einer Mischung aus 9,3 g (0,05 Mol) Tributylamin, 6,1 g (0,025 Mol) N′-(4,6-Dimethyl-pyrimidin-2-yl)-N″-phenyl-guanidin und 60 ml Cyclohexan tropfenweise gegeben. Nach 15 Stunden Rühren bei 25 °C wird eingeengt und der Rückstand mit 50 ml Ethanol verrieben. Das kristallin angefallene Produkt wird durch Absaugen isoliert.

Man erhält 1,4 g (13% der Theorie) N′-(4,6-Dimethyl-pyrimidin-2-yl)-N″-phenyl-N‴-(2-chlor-benzolsulfonyl)-guanidin vom Schmelzpunkt 196 °C.

Die Mutterlauge wird mit 400 ml Wasser und 10 ml konzentrierter Salzsäure versetzt. Das hierbei gebildete kristalline Produkt wird durch Absaugen isoliert.

Man erhält 5,0 g (34% der Theorie) N′-(4,6-Dimethyl-pyrimidin-2-yl)-N″-phenyl-N″,N‴-bis-(2-chlor-benzolsulfonyl)-guanidin (4) vom Schmelzpunkt 120 °C.

Weiter wurden analog Beispiel 9 folgende Säureaddukte von Verbindungen der Formel (I) erhalten:

(3a) 1 : 1 – Addukt der Verbindung von Beispiel (3) mit Schwefelsäure;

(2a) 1 : 1 – Addukt der Verbindung von Beispiel (2) mit p-Toluolsulfonsäure;

(43a) 1 : 1 – Addukt der Verbindung von Beispiel (43) mit Schwefelsäure.

Beispiele zur Herstellung der Ausgangsstoffe der Formel (II)

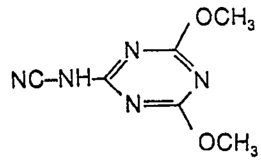

(II–1)

(Verfahren ($a^1$))

52,7 g (0,3 Mol) 2-Chlor-4,6-dimethoxy-s-triazin werden zu einer Lösung von 30 g (0,3 Mol) Cyanamid-Dinatriumsalz in 600 ml Aceton gegeben, und das Reaktionsgemisch wird 6 Studen unter Rückfluß zum Sieden erhitzt. Nach Abdestillieren des Lösungsmittels wird der kristalline Rückstand in 250 ml Wasser gelöst und die Lösung mit konzentrierter Salzsäure angesäuert. Das kristallin angefallene Produkt wird durch Absaugen isoliert.

Man erhält 33 g (61% der Theorie) 2-Cyanamino-4,6-dimethyl-s-triazin mit einem Schmelzpunkt über 300 °C.

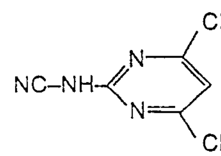

(II–2)

(Verfahren ($a^2$))

Ein Gemisch aus 42 g (0,5 Mol) Cyanoguanidin (‹Dicyandiamid›) und 50 g (0,5 Mol) 2,4-Pentandion (‹Acetylaceton›) wird 15 Stunden auf 120 °C erhitzt. Dann wird nach Abkühlen das Reaktionsgemisch mit 500 ml Wasser versetzt und die Lösung bei 0 °C bis 10 °C mit Salzsäure angesäuert. Das hierbei kristallin angefallene Produkt wird durch Absaugen isoliert. Man erhält 51,8 g (70% der Theorie) 2-Cyanamino-4,6-dimethyl-pyrimidin vom Schmelzpunkt 205 °C.

Beispiele zur Herstellung von Ausgangsstoffen der Formeln (IV) und (V):

(IV–1)

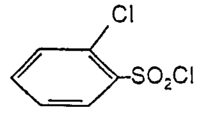

(Verfahren ($b^1$))

295 ml Phosphorylchlorid (‹Phosphoroxychlorid›) werden bei 20 °C bis 30 °C tropfenweise zu einer Mischung aus 172 g (0,8 Mol) 2-Chlor-benzolsulfonsäure-Natriumsalz, 300 ml Acetonitril und 300 ml Sulfolan gegeben. Das Reaktionsgemisch wird 4 Stunden bei 70 °C gerührt, anschließend auf 5 °C abgekühlt und mit Eiswasser verdünnt. Nach Extrahieren mit Petrolether, Waschen der Extraktionslösung mit Wasser, Trocknen, Filtrieren und

Einengen wird das im Rückstand verbliebene Produkt durch Vakuumdestillation gereinigt.

Man erhält 117 g (70% der Theorie) 2-Chlor-benzolsulfonsäurechlorid vom Siedepunkt 110 °C/0,8 Torr.

Auf die gleiche Weise konnten die nachstehend aufgeführten Verbindungen der Formel (IV) hergestellt werden:

(IV–2) 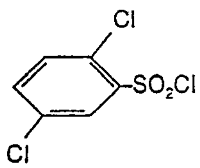 Öl

(IV–3) 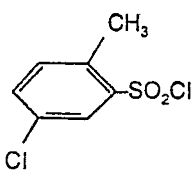 Öl

(IV–4) 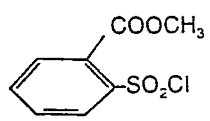

(Verfahren ($b^2$))

75,5 g (0,5 Mol) 2-Aminobenzoesäuremethylester werden in 176 ml konzentrierte Salzsäure und 100 ml Essigsäure gelöst. Hierzu wird bei 0 °C eine Lösung von 34,4 g Natriumnitrit in 70 ml Wasser getropft. Nach 15 minütigem Nachrühren wird das Reaktionsgemisch langsam zu einer auf 0 °C gekühlten gesättigten Lösung von Schwefeldioxid in 450 ml Essigsäure gegeben. Nach Entfernung des Kühlbades wird bis zum Ende der Gasentwicklung gerührt, wobei 10 g Kupfer(II)-chlorid portionsweise eingetragen werden. Nach Verdünnen mit Eiswasser, Extrahieren mit Methylenchlorid, Waschen der Extraktionslösung mit Wasser, Trocknen, Filtrieren und Einengen wird das im Rückstand verbliebene Produkt durch Vakuumdestillation gereinigt.

Man erhält 45 g (38% der Theorie) 2-Methoxy-carbonyl-benzolsulfonsäurechlorid vom Siedepunkt 150 °C/1 Torr.

Auf die gleiche Weise konnten die nachstehend aufgeführten Verbindungen der Formel (IV) hergestellt werden:

(IV–9) 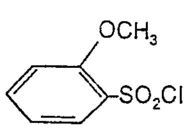 (Öl, Zersetzung bei Destillation)

(IV–10) 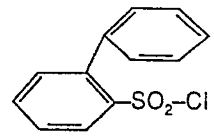 Fp. 100 °C

(IV–11) 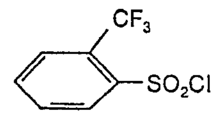 Öl

(IV–12) 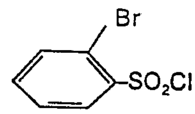 Kp. 142 °C/3 Torr

(IV–13) 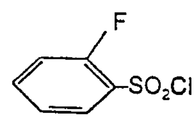 Kp. 106 °C/3 Torr

Beispiele zur Herstellung von Ausgangsstoffen der Formel (VI)

(VI–1) 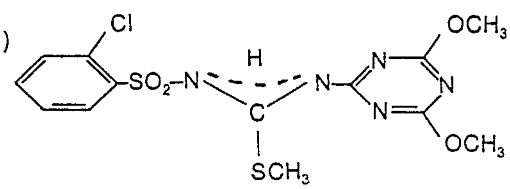

11 g (0,4 Mol) Natriumhydrid (80%ig) werden bei 20 °C portionsweise zu einer Suspension von 31,2 g (0,2 Mol) 2-Amino-4,6-dimethyl-s-triazin in 200 ml Tetrahydrofuran gegeben. Nach zwölfstündigem Rühren werden 60 g (0,2 Mol) N-(2-Chlor-benzolsulfonyl-S′,S″-dimethyl-isodithiocarbamidsäureester dazugegeben, wobei die Reaktionstemperatur auf 60 °C ansteigt. Das Reaktionsgemisch wird 5 Stunden bei 20 °C gerührt, mit 800 ml Wasser verdünnt und filtriert. Nach Ansäuern mit konzentrierter Salzsäure kristallisiert das Produkt und wird durch Absaugen isoliert.

Man erhält 42 g (48% der Theorie) N′-(4,6-Dimethoxy-s-triazin-2-yl)-N″-(2-chlor-benzolsulfonyl)-S-methyl-isothioharnstoff vom Schmelzpunkt 176 °C.

Auf die gleiche Weise konnten die nachstehend aufgeführten Verbindungen der Formel (VI) hergestellt werden:

(VI–2) 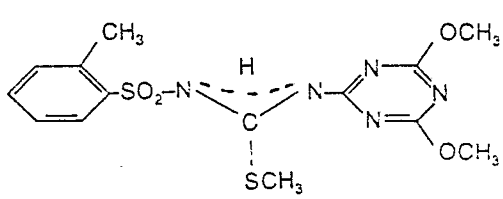 Fp. 159 °C

(VI-3)

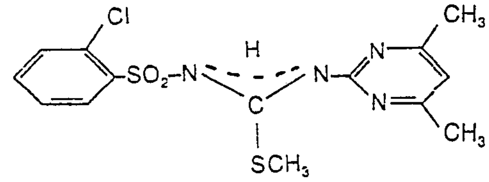

Fp. 157 °C

(VI-4)

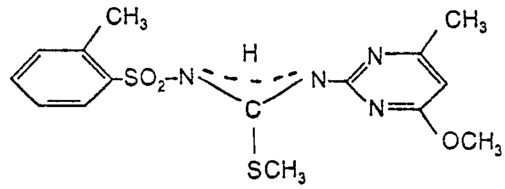

Fp. 163 °C

(VI-5)

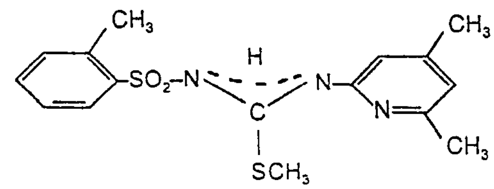

Fp. 118 °C

(VI-6)

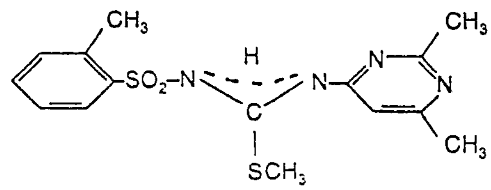

Fp. 175 °C

Beispiele zur Herstellung von Ausgangsstoffen der Formel (VIII):

(VIII-1)

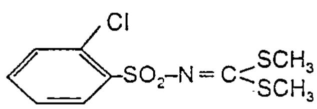

Zu einer Lösung von 20 g (0,1 Mol) 2-Chlor-benzolsulfonsäureamid in 80 ml Dimethylformamid werden bei 20 °C gleichzeitig (aus verschiedenen Tropftrichtern) 8 g (0,2 Mol) Natriumhydroxid – gelöst in 15 ml Wasser – und 6 ml (0,11 Mol) Schwefelkohlenstoff tropfenweise gegeben. Nach einstündigem Rühren werden 13 ml (0,22 Mol) Methyliodid zugetropft, und das Reaktionsgemisch wird eine weitere Stunde bei 20 °C gerührt. Durch Zugabe von 500 ml Wasser wird das Produkt ausgefällt und durch Absaugen isoliert.

Man erhält 22,1 g (75% der Theorie) N-(2-Chlor-benzolsulfonyl)-S′,S″-dimethyl-isodithiocarbamidsäureester vom Schmelzpunkt 112 °C.

Auf die gleiche Weise konnte die folgende Verbindung der Formel (VIII) hergestellt werden:

(VIII-2)

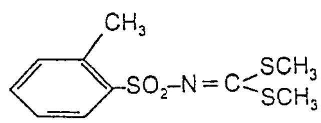

Fp. 103 °C

Verwendungsbeispiele

Beispiel A

Pre-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)

100% = totale Vernichtung

Bei diesem Test zeigt z. B. die folgende Verbindung der Herstellungsbeispiele eine ausgezeichnete Wirksamkeit: (2).

Beispiel B

Wuchshemmung bei Sojabohnen

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

In diesem Test zeigt z. B. die folgende Verbindung gemäß den Herstellungsbeispielen eine ausgezeichnete Wirksamkeit: (2).

Beispiel C

Wuchshemmung bei Gerste

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstepflanzen werden im Gewächshaus bis zum 2-Blattstadium angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

In diesem Test zeigen z. B. die folgenden Verbindungen gemäß den Herstellungsbeispielen eine ausgezeichnete Wirksamkeit: (2), (10), (22), (23), (24), (25), (27), (28), (29) und (30).

Beispiel D

Wuchshemmung bei Baumwolle

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

In diesem Test zeigt z. B die folgende Verbindung gemäß den Herstellungsbeispielen eine ausgezeichnete Wirksamkeit: (2).

**Patentansprüche**

1. Guanidin-Derivate der allgemeinen Formel (I),

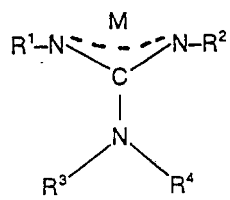

(I)

in welcher

$R^1$ für Wasserstoff oder für den Rest $-S(O)_m-R^5$ steht, worin

m für die Zahlen Null, 1 oder 2 steht und

$R^5$ für den Rest

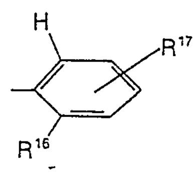

steht, worin

$R^{16}$ und $R^{17}$ gleich oder verschieden sind und für Wasserstoff, Halogen, Cyano, Nitro, $C_1$–$C_6$-Alkyl [welches gegebenenfalls durch Fluor, Chlor, Cyano, $C_1$–$C_4$-Alkoxy-carbonyl, $C_1$–$C_4$-Alkoxy, $C_1$–$C_4$-Alkylthio, $C_1$–$C_4$-Alkylsulfinyl, $C_1$–$C_4$-Alkylsulfonyl oder Phenyl substituiert ist], für $C_2$–$C_6$-Alkenyl [welches gegebenenfalls durch Fluor, Chlor, Cyano oder $C_1$–$C_4$-Alkoxy-carbonyl substituiert ist], für $C_1$–$C_4$-Alkoxy [welches gegebenenfalls durch Fluor, Chlor, Cyano, $C_1$–$C_4$-Alkoxy-carbonyl, $C_1$–$C_4$-Alkylthio, $C_1$–$C_4$-Alkylsulfinyl oder $C_1$–$C_4$-Alkylsulfonyl substituiert ist], für den Rest $-S(O)_p-R^{18}$, wobei p für die Zahlen Null, 1 oder 2 steht und $R^{18}$ für $C_1$–$C_4$-Alkyl [welches gegebenenfalls durch Fluor oder Chlor substituiert ist], für Phenyl oder für den Rest $-CO-R^{19}$ stehen, wobei $R^{19}$ für $C_1$–$C_6$-Alkyl, $C_1$–$C_6$-Alkoxy, $C_3$–$C_6$-Alkenoxy, $C_1$–$C_4$-Alkylthio, $C_1$–$C_4$-Alkylamino oder Di-($C_1$–$C_4$-alkyl)-amino steht [welche gegebenenfalls durch Fluor und/oder Chlor substituiert sind]; worin weiter

$R^5$ für den Rest

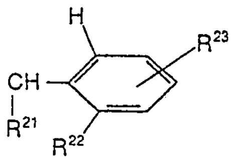

steht, worin

$R^{21}$ für Wasserstoff oder $C_1$–$C_3$-Alkyl steht und

$R^{22}$ und $R^{23}$ gleich oder verschieden sind und für Wasserstoff, Fluor, Chlor, Nitro, Cyano, $C_1$–$C_4$-Alkoxy [welches gegebenenfalls durch Fluor und/oder Chlor substituiert ist], $C_1$–$C_4$-Alkoxy-carbonyl, $C_1$–$C_4$-Alkylsulfonyl oder Di-($C_1$–$C_4$-alkyl)-aminosulfonyl stehen; worin weiter

$R^5$ für den Rest

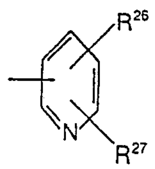

steht, worin

$R^{26}$ und $R^{27}$ gleich oder verschieden sind und für Wasserstoff, Fluor, Chlor, Nitro, Cyano, $C_1$–$C_4$-Alkyl [welches gegebenenfalls durch Fluor und/oder Chlor substituiert ist], $C_1$–$C_4$-Alkoxy [welches gegebenenfalls durch Fluor und/oder Chlor substituiert ist], für $C_1$–$C_4$-Alkylthio, $C_1$–$C_4$-Alkylsulfinyl oder $C_1$–$C_4$-Alkylsulfonyl [welche gegebenenfalls durch Fluor und/oder Chlor substituiert sind], sowie für Di-($C_1$–$C_4$-alkyl)-aminosulfonyl oder $C_1$–$C_4$-Alkoxy-carbonyl stehen, worin weiter

$R^5$ für den Rest

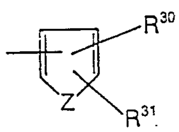

steht, worin

$R^{30}$ und $R^{31}$ gleich oder verschieden sind und für Wasserstoff, Fluor, Chlor, Brom, Cyano, Nitro, $C_1$–$C_4$-Alkyl [welches gegebenenfalls durch Fluor und/oder Chlor substituiert ist], $C_1$–$C_4$-Alkoxy [welches gegebenenfalls durch Fluor und/oder Chlor substituiert ist], $C_1$–$C_4$-Alkylthio, $C_1$–$C_4$-Alkylsulfinyl oder $C_1$–$C_4$-Alkylsulfonyl [welches gegebenenfalls durch Fluor und/oder Chlor substituiert ist], Di-($C_1$–$C_4$-alkyl)-aminosulfonyl oder $C_1$–$C_4$-Alkoxy-carbonyl stehen, und

Z für Schwefel steht, in welcher weiter

$R^2$ für den Rest

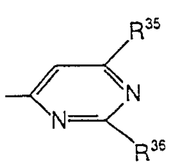

steht, worin

$R^{35}$ und $R^{36}$ gleich oder verschieden sind und für Wasserstoff, Fluor, Chlor, Brom, $C_1$–$C_4$-Alkyl [welches gegebenenfalls durch Fluor und/oder Chlor substituiert ist], $C_1$–$C_4$-Alkoxy [welches gegebenenfalls durch Fluor und/oder Chlor substituiert ist], $C_1$–$C_4$-Alkylamino oder Di-($C_1$–$C_4$-alkyl)-amino stehen mit der Maßgabe, daß wenigstens einer der Reste $R^{35}$ und $R^{36}$ von Wasserstoff verschieden ist; in welcher weiter

$R^2$ für den Rest

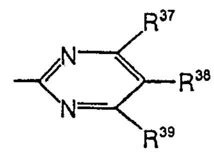

steht, worin

$R^{37}$ für Wasserstoff, Fluor, Chlor, Brom, $C_1$–$C_4$-Alkyl [welches gegebenenfalls durch Fluor und/oder Chlor substituiert ist] oder $C_1$–$C_4$-Alkoxy [welches gegebenenfalls durch Fluor und/oder Chlor substituiert ist], steht,

$R^{38}$ für Wasserstoff, Fluor, Chlor, Brom, $C_1$–$C_4$-Alkyl [welches gegebenenfalls durch Fluor und/oder Chlor substituiert ist], Cyano, Formyl, $C_1$–$C_4$-Alkyl-carbonyl oder $C_1$–$C_4$-Alkoxy-carbonyl steht und

$R^{39}$ für $C_1$–$C_4$-Alkyl [welches gegebenenfalls durch Fluor und/oder Chlor substituiert ist], $C_1$–$C_4$-Alkoxy [welches gegebenenfalls durch Fluor und/oder Chlor substituiert ist], Amino, $C_1$–$C_4$-Alkylamino oder Di-($C_1$–$C_4$-alkyl)-amino steht, oder

$R^{38}$ und $R^{39}$ gemeinsam für $C_3$–$C_4$-Alkandiyl stehen, in welcher weiter

$R^2$ für den Rest

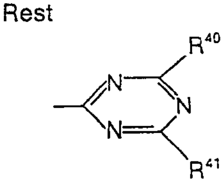

steht, worin

$R^{40}$ und $R^{41}$ gleich oder verschieden sind und für $C_1$–$C_4$-Alkyl [welches gegebenenfalls durch Fluor und/oder Chlor substituiert ist] oder $C_1$–$C_4$-Alkoxy [welches gegebenenfalls durch Fluor und/oder Chlor substituiert ist] stehen; in welcher weiter

$R^3$ für Wasserstoff, $C_1$–$C_4$-Alkyl [welches gegebenenfalls durch Fluor, Chlor, Brom, Cyano, Hydroxy oder $C_1$–$C_2$-Alkoxy substituiert ist], $C_3$–$C_6$-Cycloalkyl, $C_3$–$C_6$-Alkenyl, $C_3$–$C_6$-Alkinyl, Benzyl [welches gegebenenfalls durch Fluor, Chlor oder Methyl substituiert ist] oder für den Rest $-S(O)_n-R^6$ steht, worin

n für die Zahlen Null, 1 oder 2 steht und

$R^6$ die oben für $R^5$ angegebene Bedeutung hat, jedoch nicht in jedem Einzelfall mit $R^5$ identisch ist; in welcher weiter

$R^3$ und $R^4$ gemeinsam für $C_4$–$C_6$-Alkandiyl stehen, welches gegebenenfalls durch eine Sauerstoff-Brücke unterbrochen ist, in welcher weiter

$R^4$ für den Rest $-X-R^8$ steht, worin

X für Sauerstoff steht und

$R^8$ für $C_1$–$C_6$-Alkyl [welches gegebenenfalls durch Fluor, Chlor, $C_1$–$C_4$-Alkoxy, $C_1$–$C_4$-Alkylthio, $C_1$–$C_4$-Alkylsulfinyl oder $C_1$–$C_4$-Alkylsulfonyl substituiert ist], $C_3$–$C_6$-Alkenyl, $C_3$–$C_6$-Alkinyl, $C_3$–$C_6$-

Cycloalkyl, Benzyl [welches gegebenenfalls durch Fluor, Chlor oder Methyl substituiert ist] oder Phenyl [welches gegebenenfalls durch Fluor, Chlor, Brom, Nitro, Cyano, $C_1$–$C_4$-Alkyl, Trifluormethyl, $C_1$–$C_4$-Alkoxy, Trifluormethoxy, $C_1$–$C_4$-Alkylthio oder Trifluormethylthio substituiert ist] steht; in welcher weiter

$R^4$ für den Rest

$$-N<\begin{matrix}R^9\\R^{10}\end{matrix}$$

steht, worin

$R^9$ für Wasserstoff oder $C_1$–$C_4$-Alkyl steht und

$R^{10}$ für $C_1$–$C_4$-Alkyl, $C_3$–$C_6$-Alkenyl, $C_3$–$C_6$-Alkinyl, $C_3$–$C_6$-Cycloalkyl [welches gegebenenfalls durch eine $-SO_2$-Brücke unterbrochen ist], Benzyl oder Phenylethyl, Phenyl [welches gegebenenfalls durch Fluor, Chlor, Brom, Nitro, Cyano, $C_1$–$C_4$-Alkyl, Trifluormethyl, $C_1$–$C_4$-Alkoxy, Trifluormethoxy, $C_1$–$C_4$-Alkylthio oder Trifluormethylthio substituiert ist], Pyrimidyl, $C_1$–$C_4$-Alkyl-carbonyl, Benzoyl, $C_1$–$C_4$-Alkoxy-carbonyl, $C_1$–$C_4$-Alkylsulfonyl oder Phenylsulfonyl [welches gegebenenfalls durch Fluor, Chlor, Brom oder Methyl substituiert ist], steht, oder

$R^9$ und $R^{10}$ gemeinsam für $C_4$–$C_6$-Alkandiyl [welches gegebenenfalls durch eine Sauerstoffbrücke unterbrochen ist], stehen; in welcher weiter

$R^4$ für den Rest

$$-N=C<\begin{matrix}R^{11}\\R^{12}\end{matrix}$$

steht, worin

$R^{11}$ für Wasserstoff oder $C_1$–$C_4$-Alkyl steht und

$R^{12}$ für $C_1$–$C_4$-Alkyl, Benzyl oder Phenylethyl oder Phenyl [welches gegebenenfalls durch Fluor, Chlor, Brom, $C_1$–$C_4$-Alkyl, Trifluormethyl, Cyano, Nitro, $C_1$–$C_4$-Alkoxy oder Trifluormethoxy substituiert ist], steht, oder

$R^{11}$ und $R^{12}$ gemeinsam für $C_4$–$C_6$-Alkandiyl stehen; in welcher weiter

$R^4$ – für den Fall, daß $R^3$ von Wasserstoff verschieden ist – für Wasserstoff oder Hydroxy oder für $C_1$–$C_6$-Alkyl [welches gegebenenfalls durch Fluor, Chlor, Cyano, $C_1$–$C_4$-Alkoxy-carbonyl, Hydroxy oder $C_1$–$C_4$-Alkoxy substituiert ist], $C_3$–$C_6$-Cycloalkyl [welches gegebenenfalls durch eine $-SO_2$-Brücke unterbrochen ist], $C_3$–$C_6$-Alkenyl, $C_3$–$C_6$-Alkinyl, Benzyl [welches gegebenenfalls durch Fluor, Chlor und/oder Methyl substituiert ist] oder Pheny: [welches gegebenenfalls durch Fluor, Chlor, Cyano, Nitro, Amino, $C_1$–$C_4$-Alkyl, Trifluormethyl, $C_1$–$C_4$-Alkoxy, Trifluormethoxy, $C_1$–$C_4$-Alkylthio, Trifluormethylthio, Aminosulfonyl oder $C_1$–$C_4$-Alkoxy-carbonyl substituiert ist] steht, in welcher ferner

M für Wasserstoff, ein Natrium-, Kalium-, Magnesium-, Calcium- oder Eisen-äquivalent oder einen gegebenenfalls durch $C_1$–$C_6$-Alkyl, $C_3$–$C_6$-Alkenyl und/oder Benzyl substituierten Ammoniumrest steht, sowie 1 : 1-Addukte von Verbindungen der Formel (I) – wie vorausgehend definiert – mit Halogenwasserstoffsäuren, mit Schwefelsäure, Phosphorsäure, mit gegebenenfalls durch Fluor oder Chlor substituierten Alkansulfonsäuren mit bis zu 4 Kohlenstoffatomen oder Benzol- oder Naphthalinsulfonsäuren, welche gegebenenfalls durch Fluor, Chlor oder Methyl substituiert sind.

2. Guanidin-Derivate der allgemeinen Formel (I) gemäß Anspruch 1, dadurch gekennzeichnet, daß darin

$R^1$ für den Rest $-S(O)_m-R^5$ steht, worin

m für die Zahl 2 steht und

$R^5$ für den Rest

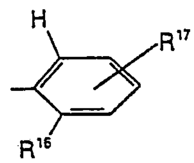

steht, worin

$R^{16}$ für Fluor, Chlor, Brom, Methyl, Trifluormethyl, $C_1$–$C_2$-Alkoxy, Difluormethoxy, Trifluormethoxy, Phenyl oder $C_1$–$C_2$-Alkoxy-carbonyl steht und

$R^{17}$ für Wasserstoff steht; in welcher weiter

$R^2$ für den Rest

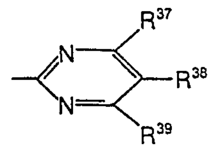

steht, worin

$R^{37}$ für Wasserstoff, Methyl oder Methoxy steht,

$R^{38}$ für Wasserstoff, Chlor, Methyl, Acetyl oder Methoxycarbonyl steht und

$R^{39}$ für $C_1$–$C_4$-Alkyl oder $C_1$–$C_4$-Alkoxy oder zusammen mit $R^{38}$ für $C_3$–$C_4$-Alkandiyl steht; in welcher weiter

$R^3$ für Wasserstoff, Methyl oder den Rest $-S(O)_n-R^6$ steht, worin n für die Zahl 2 steht und $R^6$ die oben für $R^5$ angegebene Bedeutung hat; in welcher weiter

$R^4$ für $C_1$–$C_4$-Alkoxy, $C_3$–$C_4$-Alkenoxy, $C_3$–$C_4$-Alkinoxy, Benzyloxy oder für den Rest

$$-N<\begin{matrix}R^9\\R^{10}\end{matrix}$$

steht, worin

$R^9$ für Wasserstoff oder Methyl steht und

$R^{10}$ für $C_1$–$C_3$-Alkyl, Phenyl, Acetyl, Methoxycarbonyl, Phenylsulfonyl oder p-Toluolsulfonyl steht; in welcher weiter

M für Wasserstoff, Natrium, Kalium, ein Magnesium- oder Calcium-äquivalent steht; sowie – für den Fall, daß M für Wasserstoff steht – die 1 : 1-Addukte der vorausgehend definierten Verbindungen mit Salzsäure, Schwefelsäure, Benzolsulfonsäure und p-Toluolsulfonsäure.

3. Guanidin-Derivate der allgemeinen Formel (I) gemäß Anspruch 1, dadurch gekennzeichnet, daß darin

$R^1$ für Wasserstoff oder den Rest $-S(O)_m-R^5$ steht, worin

m für die Zahlen Null, 1 oder 2 steht und

$R^5$ für den Rest

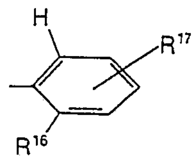

steht, worin

$R^{16}$ und $R^{17}$ für Wasserstoff stehen, oder

$R^{16}$ für Chlor, Nitro, Methyl, Trifluormethyl oder für Methoxy und

$R^{17}$ für Fluor, Chlor, Brom, Methyl, Trifluormethyl, Cyano, Nitro oder Methoxy steht; in welcher weiter

$R^2$ für den Rest

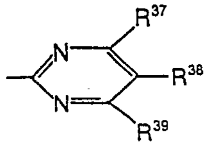

steht, worin

$R^{37}$ für Wasserstoff, Methyl oder Methoxy steht,

$R^{38}$ für Wasserstoff, Chlor, Methyl, Acetyl oder Methoxycarbonyl steht und

$R^{39}$ für $C_1$–$C_4$-Alkyl oder $C_1$–$C_4$-Alkoxy oder

$R^{38}$ und $R^{39}$ zusammen für $C_3$–$C_4$-Alkandiyl stehen, in welcher weiter

$R^3$ für Wasserstoff, Methyl oder den Rest $-S(O)_n-R^6$ steht, worin

n für die Zahlen Null, 1 oder 2 steht und

$R^6$ die oben für $R^5$ angegebene Bedeutung hat; in welcher weiter

$R^4$ für $C_1$–$C_4$-Alkoxy, $C_3$–$C_4$-Alkenoxy, $C_3$–$C_4$-Alkinoxy, Benzoyloxy oder für den Rest

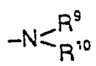

steht, worin

$R^9$ für Wasserstoff oder Methyl steht und

$R^{10}$ für $C_1$–$C_3$-Alkyl, Phenyl, Acetyl, Methoxycarbonyl, Phenylsulfonyl oder p-Toluolsulfonyl steht; in welcher weiter

$R^4$ für Hydroxy steht mit der Maßgabe, daß dann $R^3$ von Wasserstoff verschieden ist; in welcher weiter

M für Wasserstoff, Natrium, Kalium, ein Magnesium- oder Calcium-äquivalent steht, sowie – für den Fall, daß M für Wasserstoff steht – die 1 : 1-Addukte der vorausgehend definierten Verbindungen mit Salzsäure, Schwefelsäure, Benzolsulfonsäure und p-Toluolsulfonsäure.

4. Guanidin-Derivate der allgemeinen Formel (I) gemäß Anspruch 1, dadurch gekennzeichnet, daß darin

$R^1$ für den Rest $-S(O)_m-R^5$ steht, worin

m für die Zahl 2 steht und

$R^5$ für den Rest

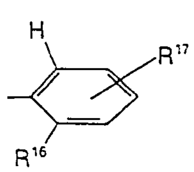

steht, worin

$R^{16}$ für Fluor, Chlor, Brom, Methyl, Trifluormethyl, $C_1$–$C_2$-Alkoxy, Difluormethoxy, Trifluormethoxy oder $C_1$–$C_2$-Alkoxy-carbonyl steht und

$R^{17}$ für Wasserstoff steht; in welcher weiter

$R^2$ für den Rest

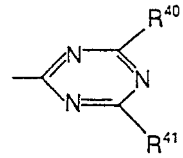

steht, worin

$R^{40}$ für Methyl, Methoxy oder Ethoxy steht und

$R^{41}$ für Methyl, Methoxy oder Ethoxy steht; in welcher weiter

$R^3$ für Wasserstoff, Methyl oder den Rest $-S(O)_n-R^6$ steht, worin

n für die Zahl 2 steht und

$R^6$ die oben für $R^5$ angegebene Bedeutung hat; in welcher weiter

$R^4$ für $C_1$–$C_4$-Alkoxy, $C_3$–$C_4$-Alkenoxy, $C_3$–$C_4$-Alkinoxy, Benzyloxy oder für den Rest

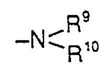

steht, worin

$R^9$ für Wasserstoff oder Methyl steht und

$R^{10}$ für $C_1$–$C_3$-Alkyl, Phenyl, Acetyl, Methoxycarbonyl, Phenylsulfonyl oder p-Toluolsulfonyl steht; in welcher weiter

M für Wasserstoff, Natrium, Kalium, ein Magnesium- oder Calcium-äquivalent steht; sowie – für den Fall, daß M für Wasserstoff steht – die 1 : 1-Addukte der vorausgehend definierten Verbindungen mit Salzsäure, Schwefelsäure, Benzolsulfonsäure und p-Toluolsulfonsäure.

5. Guanidin-Derivate der allgemeinen Formel (I) gemäß Anspruch 1, dadurch gekennzeichnet, daß darin

$R^1$ für Wasserstoff oder den Rest $-S(O)_m-R^5$ steht, worin

m für die Zahlen Null, 1 oder 2 steht und

$R^5$ für den Rest

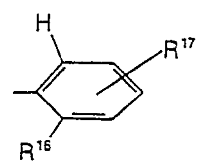

steht, worin

$R^{16}$ und $R^{17}$ beide für Wasserstoff stehen, oder

$R^{16}$ für Chlor, Nitro, Methyl, Trifluormethyl oder Methoxy und

$R^{17}$ für Fluor, Chlor, Brom, Methyl, Trifluormethyl, Cyano, Nitro oder Methoxy steht; in welcher weiter

$R^2$ für den Rest

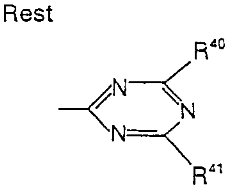

steht, worin

$R^{40}$ für Methyl, Methoxy oder Ethoxy steht und

$R^{41}$ für Methyl, Methoxy oder Ethoxy steht; in

welcher weiter

$R^3$ für Wasserstoff, Methyl oder den Rest $-S(O)_n-R^6$ steht, worin

n für die Zahlen Null, 1 oder 2 steht und

$R^6$ die oben für $R^5$ angegebene Bedeutung hat; in welcher weiter

$R^4$ für $C_1$–$C_4$-Alkoxy, $C_3$–$C_4$-Alkenoxy, $C_3$–$C_4$-Alkinoxy, Benzyloxy oder für den Rest

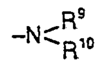

steht, worin

$R^9$ für Wasserstoff oder Methyl steht und

$R^{10}$ für $C_1$–$C_3$-Alkyl, Phenyl, Acetyl, Methoxycarbonyl, Phenylsulfonyl oder p-Toluolsulfonyl steht; in welcher weiter

$R^4$ für Hydroxy steht mit der Maßgabe, daß dann $R^3$ von Wasserstoff verschieden ist; in welcher weiter

M für Wasserstoff, Natrium, Kalium, ein Magnesium- oder Calcium-äquivalent steht, sowie – für den Fall, daß M für Wasserstoff steht – die 1 : 1-Addukte der vorausgehend definierten Verbindungen mit Salzsäure, Schwefelsäure, Benzolsulfonsäure und p-Toluolsulfonsäure.

6. Verfahren zur Herstellung von Guanidin-Derivaten der Formel (I) gemäß Anspruch 1, dadurch gekennzeichnet, daß man

(a) für den Fall, daß $R^1$ für Wasserstoff steht, $R^3$ für die in Anspruch 1 genannten Reste – ausgenommen den Rest $-S(O)_n-R^6$ – steht, M für Wasserstoff steht und die Reste $R^2$ und $R^4$ die in Anspruch 1 angegebenen Bedeutungen haben, Cyanoverbindungen der Formel (II)

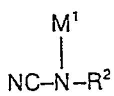 (II)

in welcher

$M^1$ für Wasserstoff steht und

$R^2$ die in Anspruch 1 angegebene Bedeutung hat, mit Aminoverbindungen der Formel (III)

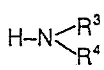 (III)

in welcher

$R^3$ für die in Anspruch 1 genannten Reste – ausgenommen den Rest $-S(O)_n-R^6$ – steht und

$R^4$ die in Anspruch 1 angegebene Bedeutung hat, bzw. mit Hydrochloriden von Aminoverbindungen der Formel (III), gegebenenfalls in Gegenwart von Verdünnungsmitteln umsetzt und gegebenenfalls die Umsetzungsprodukte mit Säureakzeptoren behandelt; oder daß man

(b) für den Fall, daß $R^1$ für den Rest $-S(O)_m-R^5$ steht, worin

m und $R^5$ die in Anspruch 1 angegebenen Bedeutungen haben, daß

M die bei (a) angegebene Bedeutung hat und

$R^2$, $R^3$ und $R^4$ die in Anspruch 1 angegebene Bedeutung haben, oder für den Fall, daß $R^3$ für den Rest $-S(O)_n-R^6$ steht, worin

n und $R^6$ die in Anspruch 1 angegebenen Bedeutungen haben, daß

M die bei (a) angegebene Bedeutung hat und

$R^1$, $R^2$ und $R^4$ die in Anspruch 1 angegebene Bedeutung haben, die nach dem oben unter (a) beschriebenen Herstellungsverfahren erhältlichen Guanidin-Derivate der Formel (I), in welcher $R^1$, $R^2$, $R^3$, $R^4$ und M die bei (a) genannten Bedeutungen haben, mit Halogen-Schwefel-Verbindungen der Formel (IV)

$$R^5-S(O)_m-X^1 \quad (IV)$$

in welcher

$X^1$ für Fluor, Chlor oder Brom steht und

m und $R^5$ die in Anspruch 1 angegebenen Bedeutungen haben, und/oder mit Halogen-Schwefel-Verbindungen der Formel (V)

$$R^6-S(O)_n-X^2 \quad (V)$$

in welcher

$X^2$ für Fluor, Chlor oder Brom steht und

n und $R^6$ die in Anspruch 1 angegebenen Bedeutungen haben, gegebenenfalls in Gegenwart von Säureakzeptoren und gegebenenfalls in Gegenwart von Verdünnungsmitteln umsetzt; oder daß man

(c) für den Fall, daß $R^3$ für die in Anspruch 1 genannten Reste – ausgenommen den Rest $-S(O)_n-R^6$ – steht und M, $R^1$, $R^2$ und $R^4$ die in Anspruch 1 angegebenen Bedeutungen haben, Isothioharnstoffe der Formel (VI)

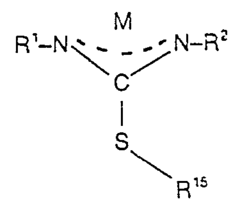 (VI)

in welcher

$R^{15}$ für $C_1$–$C_4$-Alkyl oder Benzyl steht und

M und die Reste $R^1$ und $R^2$ die in Anspruch 1 angegebenen Bedeutungen haben, mit Aminoverbindungen der Formel (III)

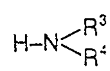 (III)

in welcher

$R^3$ für die in Anspruch 1 genannten Reste – ausgenommen den Rest $-S(O)_n-R^6$ – steht und

$R^4$ die in Anspruch 1 angegebene Bedeutung hat, bzw. mit Hydrochloriden von Aminoverbindungen der Formel (III), gegebenenfalls in Gegenwart von Säureakzeptoren und gegebenenfalls in Gegenwart von Verdünnungsmitteln umsetzt und gegebenenfalls die Umsetzungsprodukte mit Säuren behandelt; oder daß man

(d) für den Fall, daß $R^3$ für die in Anspruch 1 genannten Reste – ausgenommen den Rest $-S(O)_n-R^6$ – steht und

M, $R^1$, $R^2$ und $R^4$ die in Anspruch 1 angegebenen Bedeutungen haben, Guanidin-Derivate der Formel (I), in welcher

$R^3$ für den Rest $-S(O)_n-R^6$ steht, worin n und $R^6$ die in Anspruch 1 angegebenen Bedeutungen haben sowie

M und die Reste $R^1$, $R^2$ und $R^4$ die in Anspruch 1 angegebenen Bedeutungen haben, mit Aminoverbindungen der Formel (III)

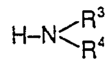 (III)

in welcher

$R^3$ für die in Anspruch 1 genannten Reste – ausgenommen den Rest $-S(O)_n-R^6$ – steht und

$R^4$ die in Anspruch 1 angegebene Bedeutung hat, bzw. mit Hydrochloriden von Aminoverbindungen der Formel (III), gegebenenfalls in Gegenwart von Säureakzeptoren und gegebenenfalls in Gegenwart von Verdünnungsmitteln umsetzt; oder daß man

(e) für den Fall, daß M für ein Metalläquivalent oder für einen gegebenenfalls substituierten Ammoniumrest – jeweils wie in Anspruch 1 definiert – steht und die Reste $R^1$, $R^2$, $R^3$ und $R^4$ die in Anspruch 1 angegebenen Bedeutungen haben, Guanidin-Derivate der Formel (I), in welcher

M für Wasserstoff steht und die Reste $R^1$, $R^2$, $R^3$ und $R^4$ die in Anspruch 1 angegebenen Bedeutungen haben, mit Metall-hydroxiden, -hydriden oder -alkanolaten oder mit metallorganischen Verbindungen bzw. mit Ammoniak oder entsprechenden Aminen gegebenenfalls in Gegenwart von Verdünnungsmitteln umsetzt; oder daß man

(f) für den Fall, daß 1 : 1-Addukte von Guanidin-Derivaten der Formel (I) mit starken Säuren herzustellen sind, Guanidin-Derivate der Formel (I), in welcher

M und die Reste $R^1$, $R^2$, $R^3$ und $R^4$ die in Anspruch 1 angegebenen Bedeutungen haben, mit starken Säuren gegebenenfalls unter Verwendung von inerten Verdünnungsmitteln umsetzt.

7. Cyanoverbindungen der Formel (II)

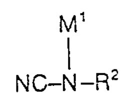 (II)

in welcher

$M^1$ für Wasserstoff steht und

$R^2$ die in Anspruch 1 angegebene Bedeutung hat, ausgenommen die Verbindung 2-Cyanamino-4,6-dimethyl-pyrimidin.

8. Herbizide Mittel, gekennzeichnet durch einen Gehalt an mindestens einem Guanidin-Derivat der Formel (I) gemäß Anspruch 1.

9. Verwendung von Guanidin-Derivaten der allgemeinen Formel (I) gemäß Anspruch 1 zur Bekämpfung von Unkraut.

10. Pflanzenwuchsregulierende Mittel, gekennzeichnet durch einen Gehalt an mindestens einem Guanidin-Derivat der allgemeinen Formel (I) gemäß Anspruch 1.

11. Verwendung von Guanidin-Derivaten der allgemeinen Formel (I) gemäß Anspruch 1, als Pflanzenwuchsregulatoren.

12. Verfahren zur Herstellung von herbiziden bzw. pflanzenwuchsregulierenden Mitteln, dadurch gekennzeichnet, daß man Guanidin-Derivate der allgemeinen Formel (I) gemäß Anspruch 1 mit Streckmitteln und/oder oberflächenaktiven Mitteln vermischt.

## Claims

1. Guanidine derivatives of the general formula (I)

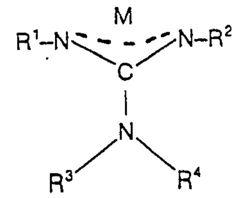 (I)

in which

$R^1$ represents hydrogen or represents the radical $-S(O)_m-R^5$ where

m represents the numbers zero, 1 or 2 and

$R^5$ represents the radical

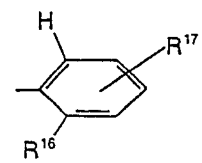

where

$R^{16}$ and $R^{17}$ are identical or different and represent hydrogen, halogen, cyano, nitro, $C_1$–$C_6$-alkyl [which is optionally substituted by fluorine, chlorine, cyano, $C_1$–$C_4$-alkoxy-carbonyl, $C_1$–$C_4$-alkoxy, $C_1$–$C_4$-alkylthio, $C_1$–$C_4$-alkylsulphinyl, $C_1$–$C_4$-alkylsulphonyl or phenyl], or represent $C_2$–$C_6$-alkenyl [which is optionally substituted by fluorine, chlorine, cyano or $C_1$–$C_4$-alkoxy-carbonyl], or represent $C_1$–$C_4$-alkoxy [which is optionally substituted by fluorine, chlorine, cyano, $C_1$–$C_4$-alkoxy-carbonyl, $C_1$–$C_4$-alkylthio, $C_1$–$C_4$-alkylsulphinyl or $C_1$–$C_4$-alkylsulphonyl], or represent the radical $-S(O)_p-R^{18}$, where p represents the numbers zero, 1 or 2 and $R^{18}$ represents $C_1$–$C_4$-alkyl [which is optionally substituted by fluorine or chlorine], or represent phenyl or represent the radical $-CO-R^{19}$, where $R^{19}$ represents $C_1$–$C_6$-alkyl, $C_1$–$C_6$-alkoxy, $C_3$–$C_6$-alkenoxy, $C_1$–$C_4$-alkylthio, $C_1$–$C_4$-alkylamino or di-($C_1$–$C_4$-alkyl)-amino [which are optionally substituted by fluorine and/or chlorine]; where furthermore

$R^5$ represents the radical

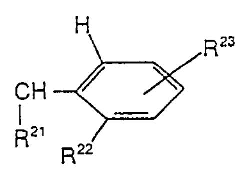

where

$R^{21}$ represents hydrogen or $C_1$–$C_3$-alkyl and

$R^{22}$ and $R^{23}$ are identical or different and represent hydrogen, fluorine, chlorine, nitro, cyano, $C_1$–$C_4$-alkoxy [which is optionally substituted by fluorine and/or chlorine], $C_1$–$C_4$-alkoxy-carbonyl, $C_1$–$C_4$-alkylsulphonyl or di-($C_1$–$C_4$-alkyl)-amino-sulphonyl; where furthermore

$R^5$ represents the radical

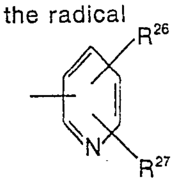

where

$R^{26}$ and $R^{27}$ are identical or different and represent hydrogen, fluorine, chlorine, nitro, cyano, $C_1$–$C_4$-alkyl [which is optionally substituted by fluorine and/or chlorine], $C_1$–$C_4$-alkoxy [which is optionally substituted by fluorine and/or chlorine], or represent $C_1$–$C_4$-alkylthio, $C_1$–$C_4$-alkylsulphinyl or $C_1$–$C_4$-alkylsulphonyl [which are optionally substituted by fluorine and/or chlorine], as well as di-($C_1$–$C_4$-alkyl)-aminosulphonyl or $C_1$–$C_4$-alkyl-carbonyl, where furthermore

$R^5$ represents the radical

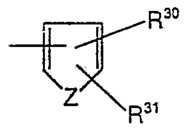

where

$R^{30}$ and $R^{31}$ are identical or different and represent hydrogen, fluorine, chlorine, bromine, cyano, nitro, $C_1$–$C_4$-alkyl [which is optionally substituted by fluorine and/or chlorine], $C_1$–$C_4$-alkoxy [which is optionally substituted by fluorine and/or chlorine], $C_1$–$C_4$-alkylthio, $C_1$–$C_4$-alkylsulphinyl or $C_1$–$C_4$-alkylsulphonyl [which is optionally substituted by fluorine and/or chlorine], di-($C_1$–$C_4$-alkyl)-aminosulphonyl or $C_1$–$C_4$-alkoxycarbonyl, and

Z represents sulphur, in which furthermore

$R^2$ represents the radical

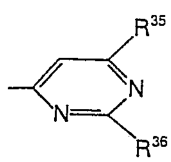

where

$R^{35}$ and $R^{36}$ are identical or different and represent hydrogen, fluorine, chlorine, bromine, $C_1$–$C_4$-alkyl [which is optionally substituted by fluorine and/or chlorine], $C_1$–$C_4$-alkoxy [which is optionally substituted by fluorine and/or chlorine], $C_1$–$C_4$-alkylamino or di-($C_1$–$C_4$-alkyl)-amino, with the proviso that at least one of the radicals $R^{35}$ and $R^{36}$ is other than hydrogen;

in which furthermore

$R^2$ represents the radical

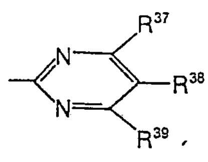

where

$R^{37}$ represents hydrogen, fluorine, chlorine, bromine, $C_1$–$C_4$-alkyl [which is optionally substituted by fluorine and/or chlorine] or $C_1$–$C_4$-alkoxy [which is optionally substituted by fluorine and/or chlorine],

$R^{38}$ represents hydrogen, fluorine, chlorine, bromine, $C_1$–$C_4$-alkyl [which is optionally substituted by fluorine and/or chlorine], cyano, formyl, $C_1$–$C_4$-alkyl-carbonyl or $C_1$–$C_4$-alkoxy-carbonyl and

$R^{39}$ represents $C_1$–$C_4$-alkyl [which is optionally substituted by fluorine and/or chlorine], $C_1$–$C_4$-alkoxy [which is optionally substituted by fluorine and/or chlorine], amino, $C_1$–$C_4$-alkylamino or di-($C_1$–$C_4$-alkyl)-amino, or

$R^{38}$ and $R^{39}$ together represent $C_3$–$C_4$-alkanediyl, in which furthermore

$R^2$ represents the radical

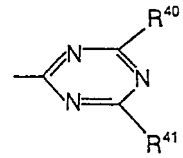

where

$R^{40}$ and $R^{41}$ are identical or different and represent $C_1$–$C_4$-alkyl [which is optionally substituted by fluorine and/or chlorine] or $C_1$–$C_4$-alkoxy [which is optionally substituted by fluorine and/or chlorine];

in which furthermore

$R^3$ represents hydrogen, $C_1$–$C_4$-alkyl [which is optionally substituted by fluorine, chlorine, bromine, cyano, hydroxyl or $C_1$–$C_2$-alkoxy], $C_3$–$C_6$-cycloalkyl, $C_3$–$C_6$-alkenyl, $C_3$–$C_6$-alkinyl or benzyl [which is optionally substituted by fluorine, chlorine or methyl], or represents the radical $-S(O)_n-R^6$, where

n represents the numbers zero, 1 or 2 and

$R^6$ has the meaning indicated above for $R^5$, but is not identical with $R^5$ in each individual case;

in which furthermore

$R^3$ and $R^4$ together represent $C_4$–$C_6$-alkanediyl which is optionally interrupted by an oxygen bridge,

in which furthermore

$R^4$ represents the radical $-X-R^8$, where

X represents oxygen and

$R^8$ represents $C_1$–$C_6$-alkyl [which is optionally substituted by fluorine, chlorine, $C_1$–$C_4$-alkoxy, $C_1$–$C_4$-alkylthio, $C_1$–$C_4$-alkylsulphinyl or $C_1$–$C_4$-alkylsulphonyl], $C_3$–$C_6$-alkenyl, $C_3$–$C_6$-alkinyl, $C_3$–$C_6$-cycloalkyl, benzyl [which is optionally substituted by fluorine, chlorine or methyl] or phenyl [which is optionally substituted by fluorine, chlorine, bromine, nitro, cyano, $C_1$–$C_4$-alkyl, trifluoromethyl, $C_1$–$C_4$-alkoxy, trifluoromethoxy, $C_1$–$C_4$-alkylthio or trifluoromethylthio];

in which furthermore

$R^4$ represents the radical

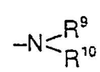

where

$R^9$ represents hydrogen or $C_1$–$C_4$-alkyl and

$R^{10}$ represents $C_1$–$C_4$-alkyl, $C_3$–$C_6$-alkenyl, $C_3$–$C_6$-alkinyl, $C_3$–$C_6$-cycloalkyl [which is optionally interrupted by an $-SO_2$-bridge], benzyl or phenylethyl, phenyl [which is optionally substituted by fluorine, chlorine, bromine, nitro, cyano, $C_1$–$C_4$-alkyl, trifluoromethyl, $C_1$–$C_4$-alkoxy, trifluoromethoxy, $C_1$–$C_4$-alkylthio or trifluoromethylthio], pyrimidyl, $C_1$–$C_4$-alkyl-carbonyl, benzoyl, $C_1$–$C_4$-alkoxy-carbonyl, $C_1$–$C_4$-alkylsulphonyl or phenylsulphonyl [which is optionally substituted by fluorine, chlorine, bromine or methyl], or

$R^9$ and $R^{10}$ together represent $C_4$–$C_6$-alkanediyl [which is optionally interrupted by an oxygen

bridge];
in which furthermore
$R^4$ represents the radical

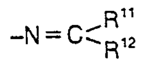

where
$R^{11}$ represents hydrogen or $C_1$–$C_4$-alkyl and
$R^{12}$ represents $C_1$–$C_4$-alkyl, benzyl or phenylethyl or phenyl [which is optionally substituted by fluorine, chlorine, bromine, $C_1$–$C_4$-alkyl, trifluoromethyl, cyano, nitro, $C_1$–$C_4$-alkoxy or trifluoromethoxy], or
$R^{11}$ and $R^{12}$ together represent $C_4$–$C_6$-alkanediyl;
in which furthermore
$R^4$ – in the event that $R^3$ is other than hydrogen – represents hydrogen or hydroxyl or represents $C_1$–$C_6$-alkyl [which is optionally substituted by fluorine, chlorine, cyano, $C_1$–$C_4$-alkoxy-carbonyl, hydroxyl or $C_1$–$C_4$-alkoxy], $C_3$–$C_6$-cycloalkyl [which is optionally interrupted by an $-SO_2$-bridge], $C_3$–$C_6$-alkenyl, $C_3$–$C_6$-alkinyl, benzyl [which is optionally substituted by fluorine, chlorine and/or methyl] or phenyl [which is optionally substituted by fluorine, chlorine, cyano, nitro, amino, $C_1$–$C_4$-alkyl, trifluoromethyl, $C_1$–$C_4$-alkoxy, trifluoromethoxy, $C_1$–$C_4$-alkylthio, trifluoromethylthio, aminosulphonyl or $C_1$–$C_4$-alkoxy-carbonyl],
in which furthermore
M represents hydrogen, a sodium, potassium, magnesium, calcium or iron equivalent or an ammonium radical which is optionally substituted by $C_1$–$C_6$-alkyl, $C_3$–$C_6$-alkenyl and/or benzyl, as well as 1 : 1 adducts of compounds of the formula (I) – as defined at the beginning – with hydrohalic acids, with sulphuric acid, phosphoric acid, with alkanesulphonic acids which have up to 4 carbon atoms and are optionally substituted by fluorine or chlorine, or with benzene- or naphthalenesulphonic acids which are optionally substituted by fluorine, chlorine or methyl.

2. Guanidine derivatives of the general formula (I) according to Claim 1, characterized in that, in this formula
$R^1$ represents the radical $-S(O)_m-R^5$, where
m represents the number 2 and
$R^5$ represents the radical

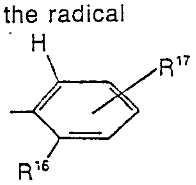

where
$R^{16}$ represents fluorine, chlorine, bromine, methyl, trifluoromethyl, $C_1$–$C_2$-alkoxy, difluoromethoxy, trifluoromethoxy, phenyl or $C_1$–$C_2$-alkoxy-carbonyl and
$R^{17}$ represents hydrogen; in which furthermore
$R^2$ represents the radical

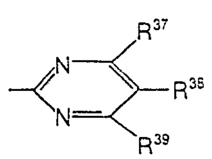

where
$R^{37}$ represents hydrogen, methyl or methoxy,
$R^{38}$ represents hydrogen, chlorine, methyl, acetyl or methoxycarbonyl and
$R^{39}$ represents $C_1$–$C_4$-alkyl or $C_1$–$C_4$-alkoxy, or together with $R^{38}$ represents $C_3$–$C_4$-alkanediyl;
in which furthermore
$R^3$ represents hydrogen, methyl or the radical $-S(O)_n-R^6$, where n represents the number 2 and $R^6$ has the meaning indicated above for $R^5$;
in which furthermore
$R^4$ represents $C_1$–$C_4$-alkoxy, $C_3$–$C_4$-alkenoxy, $C_3$–$C_4$-alkinoxy or benzyloxy, or represents the radical

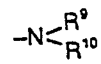

where
$R^9$ represents hydrogen or methyl and
$R^{10}$ represents $C_1$–$C_3$-alkyl, phenyl, acetyl, methoxy-carbonyl, phenylsulphonyl or p-toluenesulphonyl;
in which furthermore
M represents hydrogen, sodium, potassium, a magnesium or calcium equivalent; as well as – in the event that M represents hydrogen – the 1 : 1 adducts of the compounds with hydrochloric acid, sulphuric acid, benzenesulphonic acid and p-toluenesulphonic acid, which have been defined at the beginning.

3. Guanidine derivatives of the general formula (I), according to Claim 1, characterized in that, in this formula,
$R^1$ represents hydrogen or the radical $-S(O)_m-R^5$, where
m represents the numbers zero, 1 or 2 and
$R^5$ represents the radical

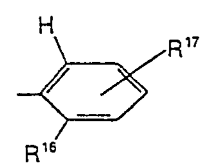

where
$R^{16}$ and $R^{17}$ represent hydrogen, or
$R^{16}$ represents chlorine, nitro, methyl or trifluoromethyl, or represents methoxy, and
$R^{17}$ represents fluorine, chlorine, bromine, methyl, trifluoromethyl, cyano, nitro or methoxy;
in which furthermore
$R^2$ represents the radical

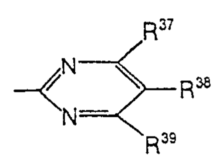

where
$R^{37}$ represents hydrogen, methyl or methoxy,
$R^{38}$ represents hydrogen, chlorine, methyl, acetyl or methoxycarbonyl and
$R^{39}$ represents $C_1$–$C_4$-alkyl or $C_1$–$C_4$-alkoxy, or
$R^{38}$ and $R^{39}$ together represent $C_3$–$C_4$-alkanediyl,
in which furthermore
$R^3$ represents hydrogen or methyl, or represents the radical $-S(O)_n-R^6$, where

n represents the numbers zero, 1 or 2 and

$R^6$ has the meaning indicated above for $R^5$; in which furthermore

$R^4$ represents $C_1$–$C_4$-alkoxy, $C_3$–$C_4$-alkenoxy, $C_3$–$C_4$-alkinoxy or benzoyloxy, or represents the radical

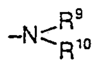

where

$R^9$ represents hydrogen or methyl and

$R^{10}$ represents $C_1$–$C_3$-alkyl, phenyl, acetyl. methoxycarbonyl, phenylsulphonyl or p-toluenesulphonyl;

in which furthermore

$R^4$ represents hydroxyl, with the proviso that in this event $R^3$ is other than hydrogen;

in which furthermore

M represents hydrogen, sodium, potassium, a magnesium or calcium equivalent, as well as – in the event that M represents hydrogen – the 1 : 1 adducts of the compounds with hydrochloric acid, sulphuric acid, benzenesulphonic acid and p-toluenesulphonic acid which have been defined at the beginning.

4. Guanidine derivatives of the general formula (I) according to Claim 1, characterized in that, in this formula,

$R^1$ represents the radical $-S(O)_m-R^5$, where

m represents the number 2 and

$R^5$ represents the radical

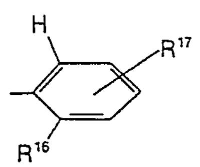

where

$R^{16}$ represents fluorine, chlorine, bromine, methyl, trifluoromethyl, $C_1$–$C_2$-alkoxy, difluoromethoxy, trifluoromethoxy or $C_1$–$C_2$-alkoxy-carbonyl and

$R^{17}$ represents hydrogen;

in which furthermore

$R^2$ represents the radical

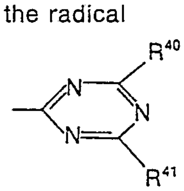

where

$R^{40}$ represents methyl, methoxy or ethoxy and

$R^{41}$ represents methyl, methoxy or ethoxy;

in which furthermore

$R^3$ represents hydrogen or methyl, or represents the radical $-S(O)_n-R^6$, where

n represents the number 2 and

$R^6$ has the meaning indicated above for $R^5$;

in which furthermore

$R^4$ represents $C_1$–$C_4$-alkoxy, $C_3$–$C_4$-alkenoxy, $C_3$–$C_4$-alkinoxy or benzyloxy, or represents the radical

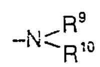

where

$R^9$ represents hydrogen or methyl and

$R^{10}$ represents $C_1$–$C_3$-alkyl, phenyl, acetyl, methoxycarbonyl, phenylsulphonyl or p-toluenesulphonyl;

in which furthermore

M represents hydrogen, sodium, potassium, a magnesium or calcium equivalent; as well as – in the event that M represents hydrogen – the 1 : 1 adducts of the compounds with hydrochloric acid, sulphuric acid, benzenesulphonic acid and p-toluenesulphonic acid which have been defined at the beginning.

5. Guanidine derivatives of the general formula (I) according to Claim 1, characterized in that, in this formula,

$R^1$ represents hydrogen or the radical $-S(O)_m-R^5$, where

m represents the numbers zero, 1 or 2, and

$R^5$ represents the radical

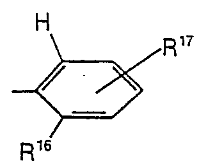

where

$R^{16}$ and $R^{17}$ both represent hydrogen, or

$R^{16}$ represents chlorine, nitro, methyl, trifluoromethyl or methoxy and

$R^{17}$ represents fluorine, chlorine, bromine, methyl, trifluoromethyl, cyano, nitro or methoxy;

in which furthermore

$R^2$ represents the radical

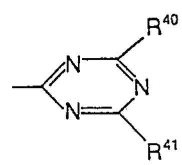

where

$R^{40}$ represents methyl, methoxy or ethoxy and

$R^{41}$ represents methyl, methoxy or ethoxy;

in which furthermore

$R^3$ represents hydrogen, methyl or the radical $-S(O)_n-R^6$, where

n represents the numbers zero, 1 or 2 and

$R^6$ has the meaning indicated above for $R^5$;

in which furthermore

$R^4$ represents $C_1$–$C_4$-alkoxy, $C_3$–$C_4$-alkenoxy, $C_3$–$C_4$-alkinoxy or benzyloxy, or represents the radical

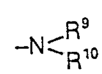

where

$R^9$ represents hydrogen or methyl and

$R^{10}$ represents $C_1$–$C_3$-alkyl, phenyl, acetyl, methoxycarbonyl, phenylsulphonyl or p-toluenesulphonyl;

in which furthermore

$R^4$ represents hydroxyl with the proviso that in this event $R^3$ is other than hydrogen;

in which furthermore

M represents hydrogen, sodium, potassium, a magnesium or calcium equivalent, as well as – in the event that M represents hydrogen – the 1 : 1 adducts of the compounds with hydrochloric acid, sulphuric acid, benzenesulphonic acid and p-toluenesulphonic acid which have been defined at the beginning.

6. Process for the preparation of guanidine derivatives of the formula (I) according to Claim 1, characterized in that

(a) in the event that $R^1$ represents hydrogen, $R^3$ represents the radicals mentioned in Claim 1 – with the exception of the radical $-S(O)_n-R^6$, – M represents hydrogen and the radicals $R^2$ and $R^4$ have the meanings indicated in Claim 1, cyano compounds of the formula (II)

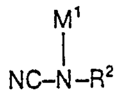 (II)

in which

$M^1$ represents hydrogen and

$R^2$ has the meaning indicated in Claim 1 are reacted with amino compounds of the formula (III)

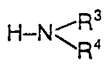 (III)

in which

$R^3$ represents the radicals mentioned in Claim 1 – with the exception of the radical $-S(O)_n-R^6$ – and

$R^4$ has the meaning indicated in Claim 1, or with hydrochlorides of amino compounds of the formula (III),

if appropriate in the presence of diluents and, if appropriate, the reaction products are treated with acid acceptors; or in that

(b) in the event that $R^1$ represents the radical $-S(O)_m-R^5$, where

m and $R^5$ have the meanings indicated in Claim 1, that

M has the meaning indicated in the case of (a) and

$R^2$, $R^3$ and $R^4$ have the meaning indicated in Claim 1, or in event that $R^3$ represents the radical $-S(O)_n-R^6$, where

n and $R^6$ have the meanings indicated in Claim 1, that

M has the meaning indicated in the case of (a) and

$R^1$, $R^2$ and $R^4$ have the meaning indicated in Claim 1, the guanidine derivatives of the formula (I), in which $R^1$, $R^2$, $R^3$, $R^4$ and M have the meanings indicated in the case of (a), which can be obtained by the preparation process described above under (a) are reacted with halogen-sulphur compounds of the formula (IV)

$$R^5-S(O)_m-X^1 \qquad (IV)$$

in which

$X^1$ represents fluorine, chlorine or bromine and

m and $R^5$ have the meanings indicated in Claim 1, and/or with halogen-sulphur compounds of the formula (V)

$$R^6-S(O)_n-X^2 \qquad (V)$$

in which

$X^2$ represents fluorine, chlorine or bromine and

n and $R^6$ have the meanings indicated in Claim 1, if appropriate in the presence of acid acceptors and if appropriate in the presence of diluents; or in that

(c) in the event that $R^3$ represents the radicals given in Claim 1 – with the exception of the radical $-S(O)_n-R^6$ – and M, $R^1$, $R^2$ and $R^4$ have the meanings indicated in Claim 1, isothioureas of the formula (VI)

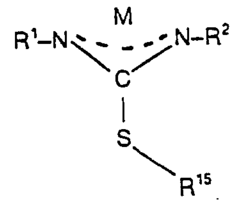 (VI)

in which

$R^{15}$ represents $C_1$–$C_4$-alkyl or benzyl and

M and the radicals $R^1$ and $R^2$ have the meanings indicated in Claim 1, are reacted with amino compounds of the formula (III)

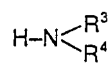 (III)

in which

$R^3$ represents the radicals given in Claim 1 – with the exception of the radical $-S(O)_n-R^6$ – and

$R^4$ has the meaning indicated in Claim 1, or with hydrochlorides of amino compounds of the formula (III), if appropriate in the presence of acid acceptors and if appropriate in the presence of diluents, and, if appropriate, the reaction products are treated with acids; or in that

(d) in the event that $R^3$ represents the radical given in Claim 1 – with the exception of the radical $-S(O)_n-R^6$ – and M, $R^1$, $R^2$ and $R^4$ have the meanings indicated in Claim 1, guanidine derivatives of the formula (I) in which

$R^3$ represents the radical $-S(O)_n-R^6$, where n and $R^6$ have the meanings indicated in Claim 1 and

M and the radicals $R^1$, $R^2$ and $R^4$ have the meanings indicated in Claim 1,

are reacted with amino compounds of the formula (III)

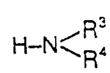 (III)

in which

$R^3$ represents the radicals given in Claim 1 – with the exception of the radical $-S(O)_n-R^6$ – and

$R^4$ has the meaning indicated in Claim 1, or with hydrochlorides of amino compounds of the formula (III), if appropriate in the presence of acid acceptors and if appropriate in the presence of diluents; or in that

(e) in the event that M represents a metal equivalent or represents an optionally substituted ammonium radical – in each case as defined in Claim 1 – and the radicals $R^1$, $R^2$, $R^3$ and $R^4$ have the

meanings indicated in Claim 1, guanidine derivatives of the formula (I) in which M represents hydrogen and the radicals $R^1$, $R^2$, $R^3$ and $R^4$ have the meanings indicated in Claim 1,
are reacted with metal hydroxides, metal hydrides or metal alkanolates or with organometal compounds or with ammonia or corresponding amines, if appropriate in the presence of diluents; or in that

(f) in the event that 1 : 1 adducts of guanidine derivatives of the formula (I) with strong acids are to be prepared, guanidine derivatives of the formula (I) in which M and the radicals $R^1$, $R^2$, $R^3$ and $R^4$ have the meanings indicated in Claim 1, are reacted with strong acids, if appropriate with the use of inert diluents.

7. Cyano compounds of the formula (II)

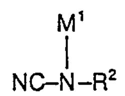

(II)

in which

$M^1$ represents hydrogen, and

$R^2$ has the meaning indicated in Claim 1, with the exception of the compound
2-cyanoamino-4,6-dimethyl-pyrimidine.

8. Herbicidal agents, characterized in that they contain at least one guanidine derivatives of the formula (I), according to Claim 1.

9. Use of guanidine derivatives of the general formula (I) according to Claim 1 for combating weeds.

10. Plant-growth regulating agents, characterized in that they contain at least one guanidine derivative of the general formula (I) according to Claim 1.

11. Use of guanidine derivatives of the general formula (I) according to Claim 1, as plant-growth regulators.

12. Process for the preparation of herbicidal or plant-growth regulating agents, characterized in that guanidine derivatives of the general formula (I) according to Claim 1 are mixed with extenders and/or surface-active agents.

**Revendications**

1. Dérivés de guanidine de formule générale (I)

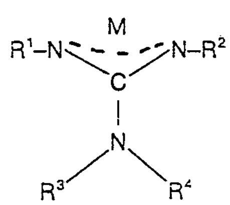

(I)

dans laquelle

$R^1$ représente l'hydrogène ou le reste $-S(O)_m-R^5$, dans lequel

m représente les nombres zéro, 1 ou 2 et

$R^5$ représente le reste de formule

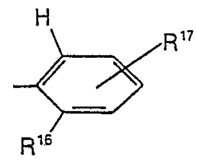

dans lequel

$R^{16}$ et $R^{17}$ sont identiques ou différents et représentent l'hydrogène, un halogène, un groupe cyano, nitro, alkyle en $C_1$ à $C_6$ [qui est éventuellement substitué par du fluor, du chlore, un radical cyano, (alkoxy en $C_1$ à $C_4$)-carbonyle, alkoxy en $C_1$ à $C_4$, alkylthio en $C_1$ à $C_4$, alkylsulfinyle en $C_1$ à $C_4$, alkylsulfonyle en $C_1$ à $C_4$ ou phényle], un groupe alcényle en $C_2$ à $C_6$ [qui est substitué, le cas échéant, par du fluor, du chlore, un radical cyano ou (alkoxy en $C_1$ à $C_4$)-carbonyle], un groupe alkoxy en $C_1$ à $C_4$ [qui est substitué, le cas échéant, par du fluor, du chlore, un radical cyano, (alkoxy en $C_1$ à $C_4$)-carbonyle, alkylthio en $C_1$ à $C_4$, alkylsulfinyle en $C_1$ à $C_4$ ou alkylsulfonyle en $C_1$ à $C_4$], le reste $-S(O)_p-R^{18}$ dans lequel p représente les nombres zéro, 1 ou 2 et $R^{18}$ est un groupe alkyle en $C_1$ à $C_4$ [qui est substitué, le cas échéant, par du fluor ou du chlore], un groupe phényle ou le reste $-CO-R^{19}$ dans lequel $R^{19}$ est un radical alkyle en $C_1$ à $C_6$, alkoxy en $C_1$ à $C_6$, alcénoxy en $C_3$ à $C_6$, alkylthio en $C_1$ à $C_4$, alkylamino en $C_1$ à $C_4$ ou di-(alkyle en $C_1$ à $C_4$)-amino [qui sont substitués, le cas échéant, par du fluor et/ou du chlore]; en outre

$R^5$ représente le reste de formule

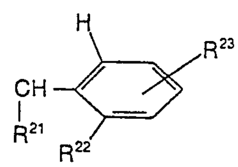

dans lequel

$R^{21}$ est l'hydrogène ou un groupe alkyle en $C_1$ à $C_3$ et

$R^{22}$ et $R^{23}$ sont identiques ou différents et représentent l'hydrogène, le fluor, le chlore, un radical nitro, cyano, alkoxy en $C_1$ à $C_4$ [qui est substitué, le cas échéant, par du fluor et/ou du chlore], un groupe (alkoxy en $C_1$ à $C_4$)-carbonyle, alkylsulfonyle en $C_1$ à $C_4$ ou di-(alkyle en $C_1$ à $C_4$)-aminosulfonyle; en outre

$R^5$ représente le reste

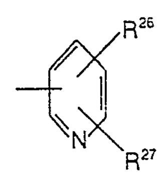

dans lequel

$R^{26}$ et $R^{27}$ sont identiques ou différents et représentent l'hydrogène, le fluor, le chlore, un groupe nitro, cyano, alkyle en $C_1$ à $C_4$ [qui est substitué, le cas échéant, par du fluor et/ou du chlore], un groupe alkoxy en $C_1$ à $C_4$ [qui est substitué, le cas échéant, par du fluor et/ou par du chlore], un groupe alkylthio en $C_1$ à $C_4$, alkylsulfinyle en $C_1$ à

$C_4$ ou alkylsulfonyle en $C_1$ à $C_4$ [qui sont substitués, le cas échéant, par du fluor et/ou du chlore], ainsi qu'un groupe di-(alkyle en $C_1$ à $C_4$)-aminosulfonyle ou (alkoxy en $C_1$ à $C_4$)-carbonyle, en outre

$R^5$ représente le reste

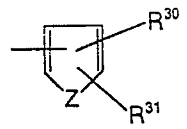

dans lequel

$R^{30}$ et $R^{31}$ sont identiques ou différents et représentent l'hydrogène, le fluor, le chlore, le brome, un groupe cyano, nitro, alkyle en $C_1$ à $C_4$ [qui est substitué, le cas échéant, par du fluor et/ou du chlore], un groupe alkoxy en $C_1$ à $C_4$ [qui est substitué, le cas échéant, par du fluor et/ou du chlore], un groupe alkylthio en $C_1$ à $C_4$, alkylsulfinyle en $C_1$ à $C_4$ ou alkylsulfonyle en $C_1$ à $C_4$ [qui est substitué, le cas échéant, par du fluor et/ou du chlore], un groupe di-(alkyle en $C_1$ à $C_4$)-aminosulfonyle ou (alkoxy en $C_1$ à $C_4$)-carbonyle et

Z représente le soufre, dans laquelle en outre

$R^2$ représente le reste

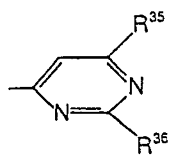

dans lequel

$R^{35}$ et $R^{36}$ sont identiques ou différents et représentent l'hydrogène, le fluor, le chlore, le brome, un groupe alkyle en $C_1$ à $C_4$ [qui est substitué, le cas échéant, par du fluor et/ou du chlore], un groupe alkoxy en $C_1$ à $C_4$ [qui est substitué, le cas échéant, par du fluor et/ou du chlore], un groupe alkylamino en $C_1$ à $C_4$ ou di-(alkyle en $C_1$ à $C_4$)-amino, sous réserve qu'au moins l'un des restes $R^{35}$ et $R^{36}$ soit différent de l'hydrogène;

dans laquelle en outre

$R^2$ représente le reste

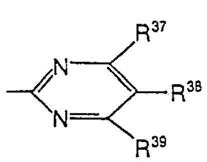

dans lequel

$R^{37}$ est l'hydrogène, le fluor, le chlore, le brome, un groupe alkyle de $C_1$ à $C_4$ [qui est substitué, le cas échéant, par du fluor et/ou du chlore] ou un groupe alkoxy en $C_1$ à $C_4$ [qui est substitué, le cas échéant, par du fluor et/ou du chlore],

$R^{38}$ représente l'hydrogène, le fluor, le chlore, le brome, un groupe alkyle en $C_1$ à $C_4$ [qui est substitué, le cas échéant, par du fluor et/ou du chlore], un groupe cyano, formyle, (alkyle en $C_1$ à $C_4$)-carbonyle ou (alkoxy en $C_1$ à $C_4$)-carbonyle et

$R^{39}$ est un groupe alkyle en $C_1$ à $C_4$ [qui est substitué, le cas échéant, par du fluor et/ou chlore], un groupe alkoxy en $C_1$ à $C_4$ [qui est substitué, le cas échéant, par du fluor et/ou du chlore], un groupe amino, alkylamino en $C_1$ à $C_4$ ou di-(alkyle en $C_1$ à $C_4$)-amino, ou bien

$R^{38}$ et $R^{39}$ forment conjointement un groupe alcanediyle en $C_3$ ou $C_4$,

dans laquelle en outre

$R^2$ représente le reste

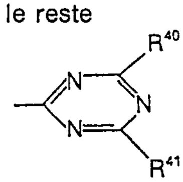

dans lequel

$R^{40}$ et $R^{41}$ sont identiques ou différents et représentent un groupe alkyle en $C_1$ à $C_4$ [qui est substitué, le cas échéant, par du fluor et/ou du chlore] ou un groupe alkoxy en $C_1$ à $C_4$ [qui est substitué, le cas échéant, par du fluor et/ou du chlore;

dans laquelle en outre

$R^3$ représente l'hydrogène, un groupe alkyle en $C_1$ à $C_4$ [qui est substitué, le cas échéant, par du fluor, du chlore, du brome, un radical cyano, hydroxy ou alkoxy en $C_1$ à $C_2$], un groupe cycloalkyle en $C_3$ à $C_6$, alcényle en $C_3$ à $C_6$, alcynyle en $C_3$ à $C_6$, benzyle [qui est substitué, le cas échéant, par du fluor, du chlore ou un radical méthyle] ou le reste de formule $-S(O)_n-R^6$ dans laquelle

n représente les nombres zéro, 1 ou 2 et

$R^6$ a la définition indiquée ci-dessus pour $R^5$, sans toutefois être identique à $R^5$ dans chaque cas individuel;

dans laquelle en outre

$R^3$ et $R^4$ forment conjointement un groupe alcanediyle en $C_4$ à $C_6$ qui est interrompu, le cas échéant par un pont oxygène,

dans laquelle en outre

$R^4$ représente le reste $-X-R^8$ dans lequel

X désigne l'oxygène et

$R^8$ est un groupe alkyle en $C_1$ à $C_6$ [qui est substitué, le cas échéant, par du fluor, du chlore, un radical alkoxy en $C_1$ à $C_4$, alkylthio en $C_1$ à $C_4$, alkylsulfinyle en $C_1$ à $C_4$ ou alkylsulfonyle en $C_1$ à $C_4$], un groupe alcényle en $C_3$ à $C_6$, alcynyle en $C_3$ à $C_6$, cycloalkyle en $C_3$ à $C_6$, benzyle [qui est substitué, le cas échéant, par du fluor, du chlore ou un radical méthyle] ou un groupe phényle [qui est substitué, le cas échéant, par du fluor, du chlore, du brome, un radical nitro, cyano, alkyle en $C_1$ à $C_4$, trifluorométhyle, alkoxy en $C_1$ à $C_4$, trifluoromethoxy, alkylthio en $C_1$ à $C_4$ ou trifluorométhylthio];

dans laquelle en outre

$R^4$ représente le reste

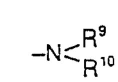

dans lequel

$R^9$ est l'hydrogène ou un reste alkyle en $C_1$ à $C_4$ et

$R^{10}$ est un groupe en $C_1$ à $C_4$, alcényle en $C_3$ à $C_6$, alcynyle en $C_3$ à $C_6$, cycloalkyle en $C_3$ à $C_6$ [qui est interrompu, le cas échéant, par un pont $-SO_2-$], benzyle ou phényléthyle, un groupe phényle [qui est substitué, le cas échéant, par du fluor, du chlore, du brome, un radical nitro, cyano, alkyle en $C_1$ à $C_4$, trifluorométhyle, alkoxy en $C_1$ à $C_4$, trifluorométhoxy, alkylthio en $C_1$ à $C_4$ ou trifluoro-

méthylthio], pyrimidyle, (alkyle en $C_1$ à $C_4$)-carbonyle, benzoyle, (alkoxy en $C_1$ à $C_4$)-carbonyle, alkylsulfonyle en $C_1$ à $C_4$ ou phénylsulfonyle [qui est substitué, la cas échéant, par du fluor, du chlore, du brome ou un radical méthyle], ou bien

$R^9$ et $R^{10}$ forment conjointement un groupe alcanediyle en $C_4$ à $C_6$ [qui est interrompu, le cas échéant, par un pont oxygène];

dans laquelle en outre

$R^4$ représente le reste

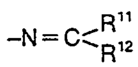

dans lequel

$R^{11}$ est l'hydrogène ou un groupe alkyle en $C_1$ à $C_4$ et

$R^{12}$ est un groupe alkyle en $C_1$ à $C_4$, benzyle ou phényléthyle ou un groupe phényle [qui est substitué, le cas échéant, par du fluor, du chlore, du brome, un radical alkyle en $C_1$ à $C_4$, trifluorométhyle, cyano, nitro, alkoxy en $C_1$ à $C_4$ ou trifluorométhoxy], ou bien

$R^{11}$ et $R^{12}$ forment conjointement un groupe alcanediyle en $C_4$ à $C_6$;

dans laquelle en outre

$R^4$ – au cas où $R^3$ est différent de l'hydrogène – représente l'hydrogène ou un groupe hydroxy ou représente un groupe alkyle en $C_1$ à $C_6$ [qui est substitué, le cas échéant, par du fluor, du chlore, un radical cyano, (alkoxy en $C_1$ à $C_4$)-carbonyle, hydroxy ou alkoxy en $C_1$ à $C_4$], un groupe cycloalkyle en $C_3$ à $C_6$ [qui est interrompu, le cas échéant, par un pont $-SO_2-$], un groupe alcényle en $C_3$ à $C_6$, alcynyle en $C_3$ à $C_6$, benzyle [qui est substitué, le cas échéant, par du fluor, du chlore et/ou un radical méthyle] ou un groupe phényle [qui est substitué, le cas échéant, par du fluor, du chlore, un radical cyano, nitro, amino, alkyle en $C_1$ à $C_4$, trifluorométhyle, alkoxy en $C_1$ à $C_4$, trifluorométhoxy, alkylthio en $C_1$ à $C_4$, trifluorométhylthio, aminosulfonyle ou (alkoxy en $C_1$ à $C_4$)-carbonyle];

dans laquelle en outre

M désigne l'hydrogène, un équivalent de sodium, potassium, magnésium, calcium ou fer ou un reste ammonium substitué, le cas échéant, par un radical alkyle en $C_1$ à $C_6$, alcényle en $C_3$ à $C_6$ et/ou benzyle,

ainsi que les produits d'addition dans le rapport 1 : 1 de composés de formule (I) – telle que définie ci-dessus – avec des hydracides halogénés, l'acide sulfurique, l'acide phosphorique, des acides alcanesulfoniques éventuellement substitués par du fluor ou du chlore et ayant jusqu'à 4 atomes de carbone ou des acides benzène- ou naphthalènesulfoniques qui sont substitués, le cas échéant, par du fluor, du chlore ou un radical méthyle.

2. Dérivés de guanidine de formule générale (I) suivant la revendication 1, caractérisés en ce que dans cette formule

$R^1$ représente le reste $-S(O)_m-R^5$, dans laquelle m représente le nombre 2 et

$R^5$ est le reste de formule

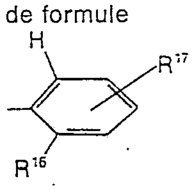

dans laquelle

$R^{16}$ est le fluor, le chlore, le brome, un radical méthyle, trifluorométhyle, alkoxy en $C_1$ ou $C_2$, difluorométhoxy, trifluorométhoxy, phényle ou (alkoxy en $C_1$ ou $C_2$)-carbonyle et

$R^{17}$ est l'hydrogène;

en outre

$R^2$ représente le reste

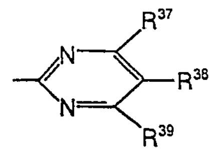

dans lequel

$R^{37}$ est l'hydrogène, un radical méthyle ou méthoxy,

$R^{38}$ est l'hydrogène, le chlore, un radical méthyle, acéthyle ou méthoxycarbonyle et

$R^{39}$ est un radical alkyle en $C_1$ à $C_4$ ou alkoxy en $C_1$ à $C_4$ ou forme conjointement avec $R^{38}$ un radical alcanediyle en $C_3$ ou $C_4$;

en outre

$R^3$ représente l'hydrogène, le radical méthyle ou le reste $-S(O)_n-R^6$, dans lequel n est égal à 2 et $R^6$ a la définition indiquée ci-dessus pour $R^5$;

en outre

$R^4$ est un reste alkoxy en $C_1$ à $C_4$, alcénoxy en $C_3$ ou $C_4$, alcynoxy en $C_3$ ou $C_4$, benzyloxy ou le reste

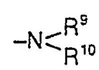

dans lequel

$R^9$ est l'hydrogène ou le radical méthyle et

$R^{10}$ est un groupe alkyle en $C_1$ à $C_3$, phényle, acétyle, méthoxycarbonyle, phénylsulfonyle ou p-toluènesulfonyle;

en outre

M est l'hydrogène, le sodium, le potassium, un équivalent de magnésium ou de calcium; ainsi que

– pour le cas où M représente l'hydrogène – les produits d'addition dans le rapport 1 : 1 des composés définis ci-dessus avec l'acide chlorhydrique, l'acide sulfurique, l'acide benzènesulfonique et l'acide p-toluènesulfonique.

3. Dérivés de guanidine de formule générale (I) suivant la revendication 1, caractérisés en ce que dans cette formule

$R^1$ représente l'hydrogène ou le reste $-S(O)_m-R^5$, où

m représente les nombres zéro, 1 ou 2 et

$R^5$ représente le reste

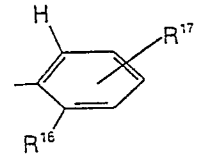

dans lequel

$R^{16}$ et $R^{17}$ représentent l'hydrogène, ou bien

$R^{16}$ est le chlore, un radical nitro, méthyle, trifluorométhyle ou méthoxy et

$R^{17}$ est le fluor, le chlore, le brome, un radical méthyle, trifluorométhyle, cyano, nitro ou méthoxy;

en outre

$R^2$ représente le reste

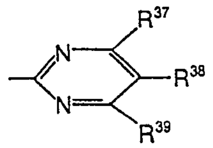

dans lequel

$R^{37}$ est l'hydrogène, le radical méthyle ou méthoxy,

$R^{38}$ est l'hydrogène, le chlore, le radical méthyle, acétyle ou méthoxycarbonyle et

$R^{39}$ est un radical alkyle en $C_1$ à $C_4$ ou alkoxy en $C_1$ à $C_4$, ou bien

$R^{38}$ et $R^{39}$ forment conjointement un groupe alcanediyle en $C_3$ ou $C_4$,

en outre

$R^3$ représente l'hydrogène, un radical méthyle ou le reste $-S(O)_n-R^6$, dans lequel

n représente les nombres zéro, 1 ou 2 et

$R^6$ a la définition indiquée ci-dessus pour $R^5$;

en outre

$R^4$ est un reste alkoxy en $C_1$ à $C_4$, alcénoxy en $C_3$ ou $C_4$, alcynoxy en $C_3$ ou $C_4$, benzoyloxy ou le reste

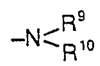

dans lequel

$R^9$ représente l'hydrogène ou le radical méthyle et

$R^{10}$ est un groupe alkyle en $C_1$ à $C_3$, phényle, acétyle, méthoxycarbonyle, phénylsulfonyle ou p-toluènesulfonyle;

en outre

$R^4$ est un groupe hydroxy, sous réserve que $R^3$ soit alors différent de l'hydrogène;

en outre

M désigne l'hydrogène, le sodium, le potassium, un équivalent de magnésium ou un équivalent de calcium, ainsi que

– au cas où M représente l'hydrogène – les produits d'addition dans le rapport 1 : 1 des composés définis ci-dessus avec l'acide chlorhydrique, l'acide sulfurique, l'acide benzènesulfonique et l'acide p-toluène-sulfonique.

4. Dérivés de guanidine de formule générale (I) suivant la revendication 1, caractérisés en ce que dans cette formule

$R^1$ représente le reste $-S(O)_n-R^5$, où

m est le nombre 2 et

$R^5$ représente le reste

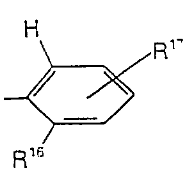

dans lequel

$R^{16}$ est le fluor, le chlore, le brome, un radical méthyle, trifluorométhyle, alkoxy en $C_1$ ou $C_2$, difluorométhoxy, trifluorométhoxy ou (alkoxy en $C_1$ ou $C_2$)-carbonyle et

$R^{17}$ représente l'hydrogène;

en outre

$R^2$ représente le reste

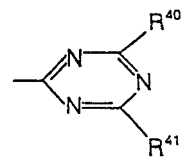

dans lequel

$R^{40}$ est un radical méthyle, méthoxy ou éthoxy et

$R^{41}$ est un radical méthyle, méthoxy ou éthoxy;

en outre

$R^3$ est l'hydrogène, un radical méthyle ou le reste $-S(O)_n-R^6$, dans lequel

n représente le nombre 2 et

$R^6$ a la définition indiquée ci-dessus pour $R^5$; en outre

$R^4$ est un reste alkoxy en $C_1$ à $C_4$, alcénoxy en $C_3$ ou $C_4$, alcynoxy en $C_3$ ou $C_4$, benzyloxy ou le reste

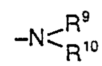

dans lequel

$R^9$ est l'hydrogène ou le radical méthyle et

$R^{10}$ est un reste alkyle en $C_1$ à $C_3$, phényle, acétyle, méthoxycarbonyle, phénylsulfonyle ou p-toluènesulfonyle;

en outre

M est l'hydrogène, le sodium, le potassium, un équivalent de magnésium ou de calcium; ainsi que

– au cas où M représente l'hydrogène – les produits d'addition dans le rapport 1 : 1 des composés définis ci-dessus avec l'acide chlorhydrique, l'acide sulfurique, l'acide benzènesulfonique ou l'acide p-toluènesulfonique.

5. Dérivés de guanidine de formule générale (I) suivant la revendication 1, caractérisés en ce que dans cette formule

$R^1$ est l'hydrogène ou le reste $-S(O)_m-R^5$, où

m représente les nombres zéro, 1 ou 2 et

$R^5$ représente le reste de formule

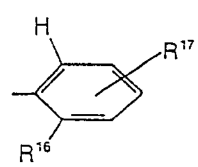

dans laquelle

$R^{16}$ et $R^{17}$ sont tous deux de l'hydrogène, ou bien

$R^{16}$ représente du chlore, un radical nitro, méthyle, trifluorométhyle ou méthoxy et

$R^{17}$ représente du fluor, du chlore, du brome, un radical méthyle, trifluorométhyle, cyano, nitro ou méthoxy;

en outre

$R^2$ représente le reste

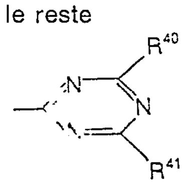

dans lequel

$R^{40}$ est un radical méthyle, méthoxy ou éthoxy et

$R^{41}$ est un radical méthyle, méthoxy ou éthoxy;

en outre

$R^3$ représente l'hydrogène, un radical méthyle ou le reste $-S(O)_n-R^6$, dans lequel

n représente les nombres zéro, 1 ou 2 et

$R^6$ a la définition indiquée ci-dessus pour $R^5$;

en outre

$R^4$ est un reste alkoxy en $C_1$ à $C_4$, alcénoxy en $C_3$ ou $C_4$, alcynoxy en $C_3$ ou $C_4$, benzyloxy ou le reste

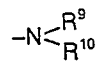

dans lequel

$R^9$ représente l'hydrogène ou le radical méthyle et

$R^{10}$ est un radical alkyle en $C_1$ à $C_3$, phényle, acétyle, méthoxycarbonyle, phénylsulfonyle ou p-toluènesulfonyle;

en outre

$R^4$ est un radical hydroxy, sous réserve que $R^3$ soit alors différent de l'hydrogène;

en outre

M représente l'hydrogène, le sodium, le potassium, un équivalent de magnésium ou un équivalent de calcium, ainsi que

– au cas où M représente l'hydrogène – les produits d'addition dans le rapport 1 : 1 des composés définis ci-dessus avec l'acide chlorhydrique, l'acide sulfurique, l'acide benzènesulfonique et l'acide p-toluènesulfonique.

6. Procédé de préparation de dérivés de guanidine de formule (I) suivant la revendication 1, caractérisé en ce que:

(a) au cas où $R^1$ représente l'hydrogène, $R^3$ représente les restes mentionnés dans la revendication 1 – à l'exception du reste $-S(O)_n-R^6$, – M représente l'hydrogène et les restes $R^2$ et $R^4$ ont les définitions indiquées dans la revendication 1, on fait réagir des composés à fonction cyano de formule (II)

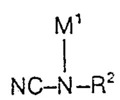

(II)

dans laquelle

$M^1$ représente l'hydrogène et

$R^2$ a la définition indiquée dans la revendication 1, avec des composés aminés de formule (III)

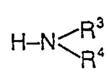

(III)

dans laquelle

$R^3$ représente les restes mentionnés dans la revendication 1 – excepté le reste $-S(O)_n-R^6$ – et

$R^4$ a la définition indiquée dans la revendication 1,

ou avec des chlorhydrates de composés aminés de formule (III),

le cas échéant en présence de diluants et on traite éventuellement les produits de réaction avec des accepteurs d'acides; ou

(b) au cas où $R^1$ représente le reste $-S(O)_m-R^5$, dans lequel

m et $R^5$ ont les définitions indiquées dans la revendication 1,

M a la définition indiquée en (a) et

$R^2$, $R^3$ et $R^4$ ont la définition indiquée dans la revendication 1,

ou au cas où $R^3$ représente le reste $-S(O)_n-R^6$, dans lequel

n et $R^6$ ont les définitions indiquées dans la revendication 1,

M a la définition indiquée en (a) et

$R^1$, $R^2$ et $R^4$ ont la définition indiquée dans la revendication 1,

on fait réagir les dérivés de guanidine obtenus par le procédé de préparation décrit en (a) ci-dessus, de formule (I) dans laquelle $R^1$, $R^2$, $R^3$, $R^4$ et M ont les définitions indiquées en (a),

avec des composés halogénosulfurés de formule (IV)

$$R^5-S(O)_m-X^1 \qquad (IV)$$

dans laquelle

$X^1$ représente le fluor, le chlore ou le brome et

m et $R^5$ ont les définitions indiquées dans la revendication 1

et/ou avec des composés halogénosulfurés de formule (V)

$$R^6-S(O)_n-X^2 \qquad (V)$$

dans laquelle

$X^2$ représente le fluor, le chlore ou le brome et

n et $R^6$ ont les définitions indiquées dans la revendication 1,

le cas échéant en présence d'accepteurs d'acides et en la présence éventuelle de diluants; ou bien

(c) au cas où $R^3$ représente les restes mentionnés dans la revendication 1

– à l'exception du reste $-S(O)_n-R^6$ – et M, $R^1$, $R^2$ et $R^4$ ont les définitions indiquées dans la revendication 1,

on fait réagir des isothiourées de formule (VI),

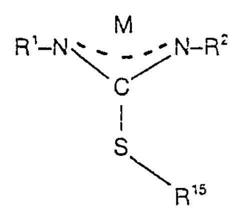

(VI)

dans laquelle

$R^{15}$ est un reste alkyle en $C_1$ à $C_4$ ou benzyle et

M et les restes $R^1$ et $R^2$ ont les définitions indiquées dans la revendication 1,

avec des composés aminés de formule (III)

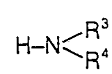

(III)

dans laquelle

$R^3$ représente les restes mentionnés dans la revendication 1 – à l'exception du reste $-S(O)_n-R^6$ – et

$R^4$ a la définition indiquée dans la revendication 1,

ou bien avec des chlorhydrates de composés aminés de formule (III), éventuellement en présence d'accepteurs d'acides et en la présence éventuelle de diluants et on traite, le cas échéant, les produits de réaction avec des acides; ou bien

(d) au cas où $R^3$ représente les restes mentionnées dans la revendication 1 – à l'exception du reste $-S(O)_n-R^6$ – et M, $R^1$, $R^2$ et $R^4$ ont les définitions indiquées dans la revendication 1, on fait réagir des dérivés de guanidine de formule (I), dans laquelle

$R^3$ représente le reste $-S(O)_n-R^6$ où n et $R^6$ ont les définitions indiquées dans la revendication 1 ainsi que

M et les restes $R^1$, $R^2$ et $R^4$ ont les définitions données dans la revendication 1, avec des composés aminés de formule (III)

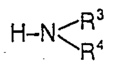 (III)

dans laquelle

$R^3$ représente les restes mentionnés dans la revendication 1 – à l'exception du reste $-S(O)_n-R^6$- et

$R^4$ a la définition indiquée dans la revendication 1, ou avec des chlorhydrates de composés aminés de formule (III), le cas échéant en présence d'accepteurs d'acides et en la présence éventuelle de diluants; ou bien

(e) au cas où M représente un équivalent de métal ou un reste ammonium éventuellement substitué – dans chaque cas comme défini dans la revendication 1 – et les restes $R^1$, $R^2$, $R^3$ et $R^4$ ont les définitions indiquées dans la revendication 1, on fait réagir des dérivés de guanidine de formule (I), dans laquelle M représente l'hydrogène et les restes $R^1$, $R^2$, $R^3$ et $R^4$ ont les définitions indiquées dans la revendication 1, avec des hydroxydes, hydrures ou alcanolates métalliques ou des dérivés organométalliques ou avec l'ammoniac ou des amines correspondantes, le cas échéant en présence de diluants; ou bien

(f) au cas où on désire préparer des produits d'addition dans le rapport 1 : 1 de dérivés de guanidine de formule (I) avec des acides forts, on fait réagir des dérivés de guanidine de formule (I), dans laquelle M et les restes $R^1$, $R^2$, $R^3$ et $R^4$ ont les définitions indiquées dans la revendication 1, avec des acides forts, en utilisant, le cas échéant, des diluants inertes.

7. Composé à fonction cyano de formule (II)

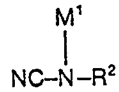 (II)

dans laquelle

$M^1$ représente l'hydrogène et

$R^2$ a la définition indiquée dans la revendication 1, excepté la 2-cyanamino-4,6-diméthylpyrimidine.

8. Compositions herbicides, caractérisées par une teneur en au moins un dérivé de guanidine de formule (I) suivant la revendication 1.

9. Utilisation de dérivés de guanidine de formule générale (I) suivant la revendication 1 pour combattre les mauvaises herbes.

10. Composition influençant la croissance des plantes, caractérisée par une teneur en au moins un dérivé de guanidine de formule générale (I) suivant la revendication 1.

11. Utilisation de dérivés de guanidine de formule générale (I) suivant la revendication 1 comme substances de croissance des plantes.

12. Procédé de préparation de compositions herbicides et influençant la croissance des plantes, caractérisé en ce qu'on mélange des dérivés de guanidine de formule générale (I) suivant la revendication 1 avec des diluants et/ou des agents tensio-actifs.